(19) Europäisches Patentamt
European Patent Office
Office européen des brevets

(11) Numéro de publication: **0 245 170 B1**

(12) # FASCICULE DE BREVET EUROPEEN

(45) Date de publication du fascicule du brevet: **29.11.89**

(21) Numéro de dépôt: **87401018.4**

(22) Date de dépôt: **04.05.87**

(51) Int. Cl.⁴: **C07J 1/00**, C07J 5/00, C07J 7/00, C07J 9/00, C07J 21/00, C07J 41/00, C07J 43/00, C07J 51/00, C07J 63/00, C07J 71/00, A61K 31/565

(54) **Nouveaux produits 19-nor ou 19-nor D-homo stéroides substitués en position 11 beta par un radical phényle portant un radical alkynile, leur procédé de préparation, leur application comme médicaments et les compositions les renfermant.**

(30) Priorité: **06.05.86 FR 8606517**

(43) Date de publication de la demande: **11.11.87 Bulletin 87/46**

(45) Mention de la délivrance du brevet: **29.11.89 Bulletin 89/48**

(84) Etats contractants désignés: **CH DE GB IT LI NL SE**

(56) Documents cités:
**EP-A- 0 057 115**
**EP-A- 0 104 387**
**WO-A-83/03099**
**FR-A- 2 377 418**

(73) Titulaire: **ROUSSEL-UCLAF, 35, boulevard des Invalides, F-75007 Paris(FR)**

(72) Inventeur: **Teutsch, Jean Georges, Résidence Lavoisier-Bât. 3 3, rue Lavoisier, F-93500 Pantin(FR)**
Inventeur: **Klich, Michel, 9, rue Robert Jumel, F-93250 Villemomble(FR)**
Inventeur: **Philibert, Daniel, 16, rue Chevalier, F-94210 La Varenne-Saint-Hilaire(FR)**

(74) Mandataire: **Bourgouin, André et al, Département des Brevets ROUSSEL UCLAF B.P. no 9, F-93230 Romainville(FR)**

**EP 0 245 170 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux produits 19-nor ou 19-nor D-homo stéroïdes substitués en position 11β par un radical phényl portant un radical alkynyle, leur procédé de préparation, leur application comme médicaments et les compositions pharmaceutiques les renfermant.

Dans les demandes de brevets européens EP 0 057 115 et 0 104 387 la demande internationale WO. 8 303 099 et la demande de brevet français BF 2 377 418 sont également décrits des stéroïdes comportant en 11β un radical phényle. Cependant, aucune de ces demandes de brevets ne décrit ni ne suggère la présence d'un radical alkynyle sur ledit radical phényle en position 11.

L'invention a pour objet les produits de formule I:

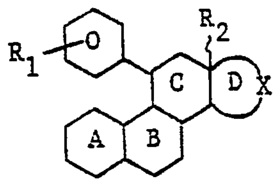

(I)

dans laquelle $R_1$ représente un radical alkynyle ayant de 2 à 8 atomes de carbone éventuellement substitué par un radical choisi parmi les radicaux hydroxyle, halogène, trialkylsilyle, alkoxy, alkylthio, dialkylamino et oxo, $R_2$ représente un radical alkyle ayant de 1 à 3 atomes de carbone, les cycles A et B ont l'une des structures suivantes;

a/. soit A et B représentent le groupement:

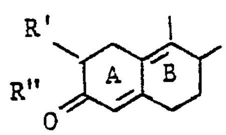

dans lequel R′ et R″ identiques ou différents représentent un atome d'hydrogène ou un radical alkyle ayant de 1 à 4 atomes de carbone;

b/. soit A et B représentent le groupement:

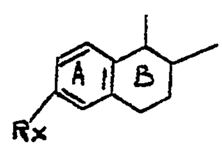

dans lequel Rx représente un atome d'hydrogène ou un groupement ORe dans lequel Re représente un atome d'hydrogène, un radical alkyle ayant de 1 à 6 atomes de carbone éventuellement substitué par un radical dialkylamino ou un radical acétyle ou propionyle;

c/. soit A et B représentent le groupement:

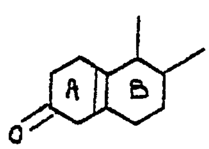

d/. soit A et B représentent le groupement:

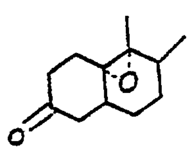

e/. soit A et B représentent le groupement:

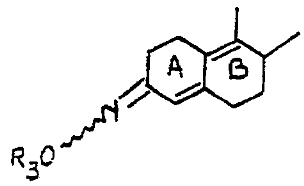

dans lequel $R_3$ représente un atome d'hydrogène un radical alkyle ayant de 1 à 8 atomes de carbone ou un radical aralkyle ayant de 7 à 15 atomes de carbone;
– le groupement de formule:

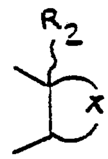

représente:
a/. soit:

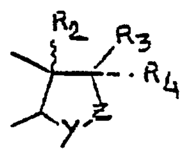

dans lequel $R_3$ et $R_4$ identiques ou différents représentent <u>ou bien</u> un atome d'hydrogène, un radical hydroxyle, un radical

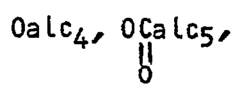

$alc_4$ et $alc_5$ représentant un radical alkyle renfermant de 1 à 8 atomes de carbone ou un radical benzyle; un radical alkyle, alkényle ou alkynyle ayant au plus 8 atomes de carbone ou un radical phényle, benzyle ou phénylethyle ou pyridyléthynyle, thiényle ou furyle, chacun de ces radicaux étant éventuellement substitué par un ou plusieurs radicaux choisis dans le groupe formé par les radicaux hydroxy, alkoxy et alkylthio ayant de 1 à 4 atomes de carbone, les atomes d'halogène et le radical dialkylamino,
<u>ou bien</u> $R_3$ et $R_4$ <u>forment ensemble l'un des radicaux suivants</u>:

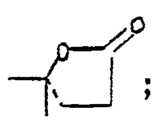 ; 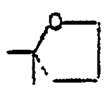 ; 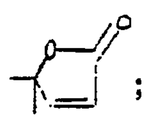 ; 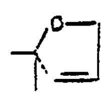 ; 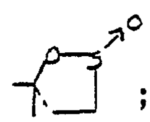 ; 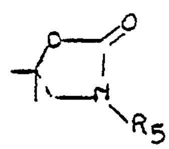 ;

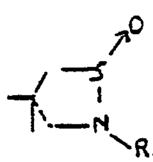 ; 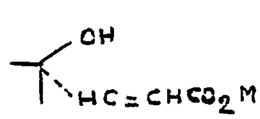 ; 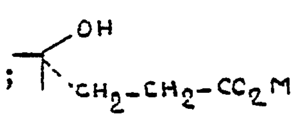 ;

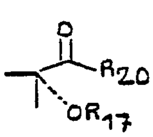 ; 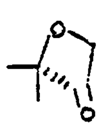 ; 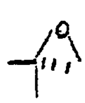 ; 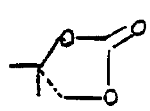 ; 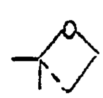

dans lesquels $R_5$ et $R_6$ représentent un atome d'hydrogène ou un radical alkyle ayant de 1 à 4 atomes de carbone, M représente un atome d'hydrogène, de sodium, de potassium ou de lithium, $R_{20}$ représente un radical alkyle éventuellement substitué par hydroxyle et $R_{17}$ représente un atome d'hydrogène ou un radical acétyle, Y et Z représentent les groupements suivants:

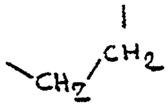 ; 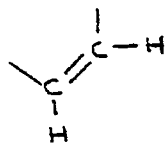 ; 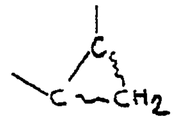 ; 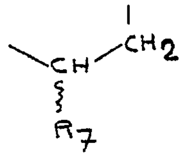 ;

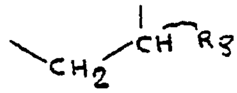

dans lesquels $R_7$ et $R_8$ représentent un radical alkyle ayant de 1 à 4 atomes de carbone;
b/. soit:

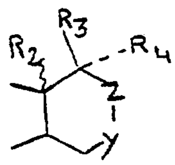

dans lequel $R_3$, $R_4$ et Y-Z ont les définitions indiquées précédemment, les traits ondulés signifient que les substituants $R_2$, $R_7$ et $R_8$ peuvent se trouver dans l'une ou l'autre des configurations possibles, ainsi que les sels d'addition des produits de formule I avec les acides et les bases.

Parmi les valeurs de $R_1$, on peut citer les radicaux éthynyle, propynyle, butynyle, pentynyle ou hexynyle. Parmi ces radicaux, on préfère les radicaux de formule $-C{\equiv}C-R_{1a}$ dans laquelle $R_{1a}$ représente un atome d'hydrogène ou un radical alkyle ayant de 1 à 6 atomes de carbone tel que les radicaux méthyle, éthyle, propyle, isopropyle, butyle, butyle secondaire, tert-butyle. La valeur préférée de $R_1$ est la valeur éthynyle en position para.

Ces radicaux peuvent être substitués par un radical hydroxyle ou un halogène tel que fluoro, chloro, bromo, iodo et de préférence chloro, un trialkylsilyle de préférence triméthylsilyle, ou par alkoxy, alkylthio, dialkylamino, tels que méthoxy, éthoxy, méthylthio, éthylthio, diméthylamino ou par un groupement oxo.

$R_2$ représente un radical méthyle, éthyle ou propyle et de préférence méthyle ou éthyle, plus préférentiellement encore méthyle.

R' et R" peuvent représenter un radical méthyle, éthyle, propyle, isopropyle, butyle linéaire, secondaire ou tertiaire.

De préférence R' et R" représentent chacun un atome d'hydrogène.

Re peut représenter l'une des valeurs alkyle citées précédemment. Lorsque ce radical alkyle est substitué, il peut notamment l'être par un radical dialkylamino, tel que diméthylamino, diéthylamino ou méthyléthylamino.

Le radical acyle que peut représenter Re peut notamment être aussi un radical acétyle ou propionyle.

Par les valeurs de $R_3$ ou $R_4$, on peut notamment citer les valeurs indiquées précédemment pour les radicaux alkyle et de préférence le radical propyle.

$R_3$ et $R_4$ peuvent également représenter un radical phenyle, benzyle ou phénéthyle éventuellement substitué par un radical choisi dans le groupe formé par les radicaux hydroxy, alkoxy, alkylthio, dialkylamino et les atomes d' halogène tels que méthoxy, méthylthio, chloro, fluoro ou hydroxy. $R_3$ et $R_4$ peuvent également représenter un radical thiényle ou furyle portant éventuellement les mêmes substituants que ceux indiqués ci-dessus.

Lorsque $R_3$ ou $R_4$ représente un radical Oalc$_4$ ou OCOalc$_5$, alc$_4$ et alc$_5$ représentent de préférence un radical méthyle, éthyle, n-propyle, butyle, pentyle, hexyle ou benzyle.

Lorsque $R_3$ ou $R_4$ représente un radical alkényle, il s'agit de préférence du radical vinyle, isopropényle, allyle 2-méthylallyle ou prop-1-ényle, ce dernier substituant étant préféré.

Lorsque $R_3$ ou $R_4$ représente un radical alkynyle, il s'agit de préférence du radical $-C{\equiv}CH$, ou $-C{\equiv}C$-alc$_9$, alc$_9$ représentant de préférence un radical méthyle, éthyle, propyle, isopropyle, isopropényle, butyle ou trifluorométhyle, de préférence encore méthyle.

Lorsque $R_3$ et $R_4$, de préférence $R_4$, représente un radical alkyle, alkényle ou alkynyle substitué, il s'agit de préférence du radical propyle, propényle ou propynyle, substitué par un radical halogène tel que chloro et plus préférentiellement d'un radical hydroxyle. La valeur préférée de $R_{20}$ est méthyle éventuellement substitué par hydroxyle. $R_{17}$ représente de préférence un atome d'hydrogène ou un radical acétyle.

Alc$_6$ représente de préférence une des valeurs préférentielles de alc$_4$ ou alc$_5$. Alc$_6$ représente de préférence un radical méthyle ou hydroxyméthyle.

Les composés préférés sont ceux pour lesquels les radicaux $R_3$ et $R_4$ sont différents.

Parmi les valeurs préférées du radical

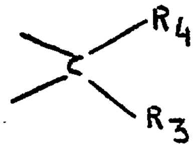 ,

on peut citer les radicaux :

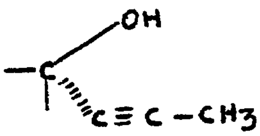
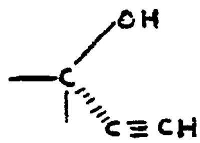
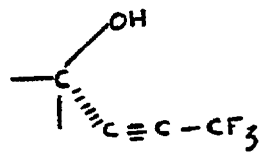

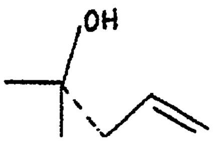 ; 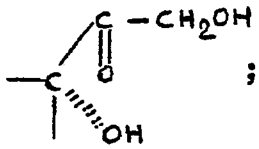 ; 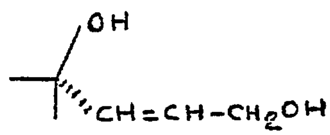 ;

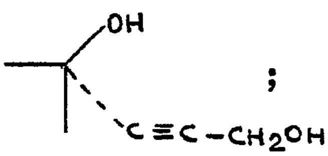 ; 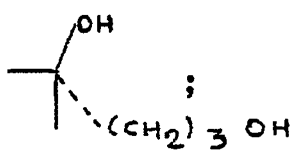 ; 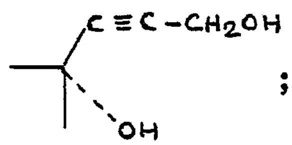 ;

E ou Z, de préférence Z ;

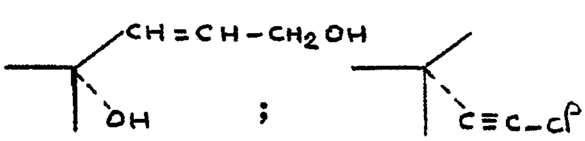 ; 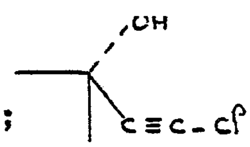 ; 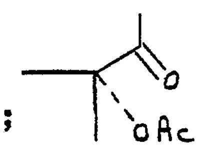

Compte-tenu des différentes valeurs des symboles Y et Z, le cycle D peut de préférence avoir les significations suivantes :

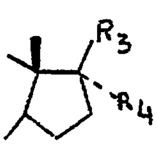 ; 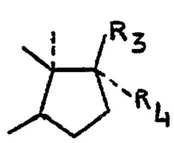 ; 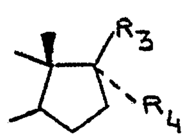 ; 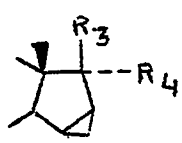 ;

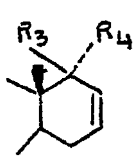

Compte tenu de la combinaison des valeurs $R_3$ et $R_4$ et des valeurs des symboles Y et Z, les valeurs suivantes peuvent être considérées comme les valeurs préférées du cycle D :

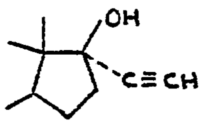 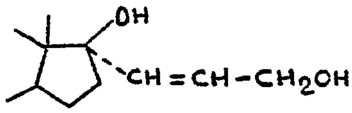 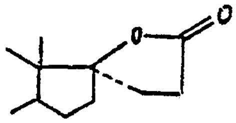

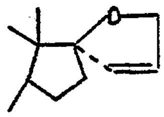 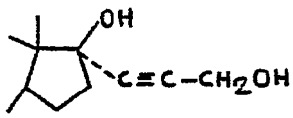 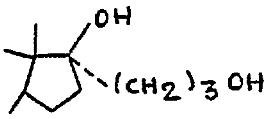

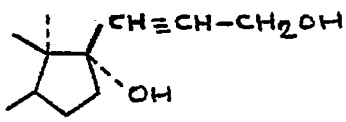 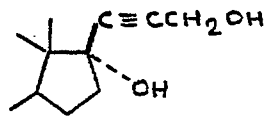 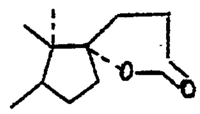

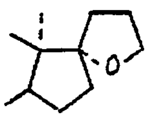 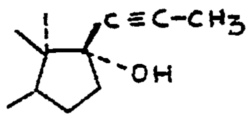 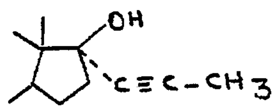 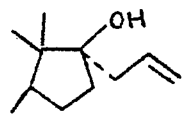

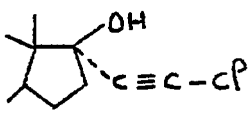 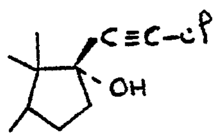 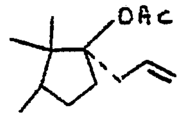 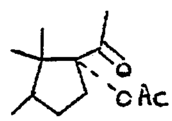

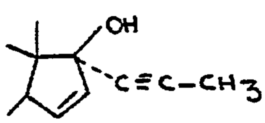 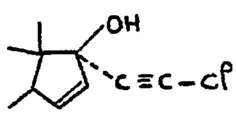 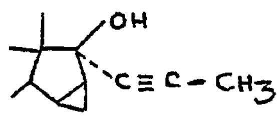

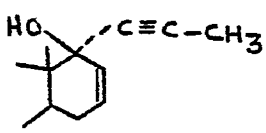 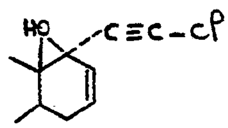 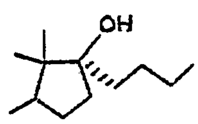 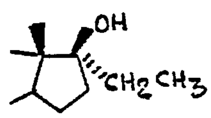

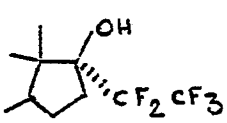 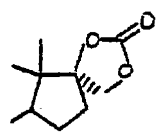 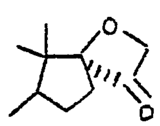

Les produits de formule I qui comportent une ou plusieurs fonctions salifiables par un acide peuvent se présenter sous forme de sels avec les acides, comme par exemple les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benène ou paratoluène sulfoniques et arylcarboxyliques.

Les produits de formule I qui comportent une ou plusieurs salifiables par une base peuvent se présenter sous forme de sels avec les bases. On peut citer les sels formés avec un équivalent de sodium, de potassium, de lithium, de calcium, de magnésium ou d'ammonium. On peut citer, parmi les bases organiques, la méthylamine, la propylamine, la triméthylamine, la diétylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

L'invention a plus particulièrement pour objet les produits de formule générale I' :

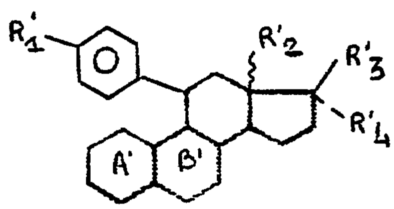

(I')

dans laquelle R' 1 représente un radical alkynyle ayant de 2 à 4 atomes de carbone éventuellement substitué par un radical choisi parmi les radicaux hydroxy, halogène ou triméthylsilyle ; $R'_2$ représente un radical méthyle ou éthyle, $R'_3$ et $R'_4$ représentent un radical hydroxyle éventuellement acylé, ou un radical alkyle, alkényle ou alkynyle ayant au plus 8 atomes de carbone et éventuellement substitué par un radical choisi parmi les radicaux hydroxyle ou halogène, ou $R'_3$ et $R'_4$ forment ensemble un radical :

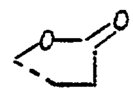

ou

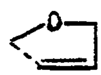

;

les cycles A' et B' ont l'une des structures suivantes :

a/. soit A' et B' représentent le groupement :

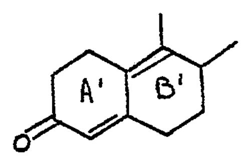

b/. soit A' et B' représentent le groupement :

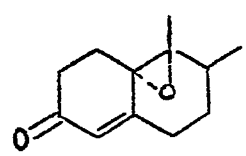

c/. soit A' et B' représentent le groupement :

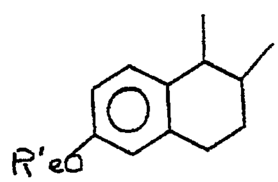

dans lequel R'e représente un atome d'hydrogène ou un radical alkyle ayant de 1 à 4 atomes de carbone.

Les valeurs préférées du radical $R'_1$ sont les valeurs éthynyle, propynyle, triméthylsilyléthynyle, de préférence éthynyle, $R'_2$ est de préférence un radical méthyle, $R'_3$ est de préférence un radical hydroxyle, acétoxy, $R'_4$ étant alors un radical propynyle, propényle, chrloroéthynyle ou hydroxy prop-1-ényle. $R'_4$ peut également représenter un radical hydroxyle lorsque $R'_3$ représente un radical éthynyle, propynyle, chloroéthynyle.

Parmi les produits de formule I', une autre classe de produits préférés est constituée par les produits de formule I' dans laquelle $R'_1$ représente un radical $-C{\equiv}C-R_{11}$ dans lequel $R_{11}$ représente un atome d'hydrogène ou un radical méthyle ou triméthylsilyle, $R'_3$ et $R'_4$ sont choisis parmi les radicaux hydroxyle, acétoxy, éthynyle et propynyle éventuellement substitués par un halogène ou par un hydroxyle, propyle et propényle éventuellement substitués par un hydroxy, ou $R'_3$ et $R'_4$ forment ensemble le radical :

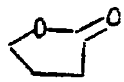

ou

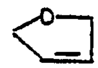

L'invention a plus particulièrement pour objet les produits décrits ci-après dans les exemples et notamment :

- La 11β -(4-éthynylphényl) 17β -hydroxy 17 α-(1-propynyl) estra 4,9-diène 3-one,
- La 11β -(4-éthynylphényl) 17α -allyl 17β-hydroxy estra 4,9-diène 3-one,
- La 17α-(chloroéthynyl) 11β-(4-éthynylphényl) 17β-hydroxy estra 4,9-diène 3-one,
- La 17α-(chloroéthynyl) 9α, 10α-époxy 11β-(4-éthynylphényl) 17β-hydroxy estr-4-ène 3-one.

L'invention a également pour objet un procédé de préparation des produits de formule I caractérisé en ce que l'on soumet un produit de formule II :

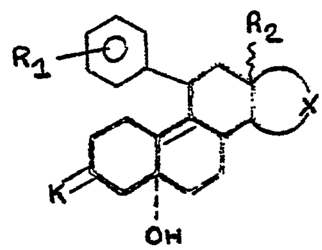

(II)

dans laquelle K représente une fonction cétone bloquée, $R_1$ et $R_2$ et X ont la signification indiquée ci-dessus, à l'action d'un réactif de déshydratation susceptible de libérer la fonction cétone pour obtenir un produit de formule $I_A$ :

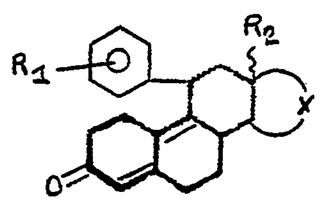

($I_A$)

produit de formule $I_A$ que l'on soumet éventuellement :

- a/. soit à l'action d'un agent réducteur, puis à un agent d'aromatisation acide pour obtenir un produit de formule $I_{B1}$ dans laquelle A et B représentent le groupement :

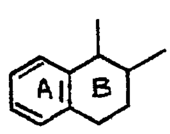

– b/. soit à l'action d'un agent d'aromatisation, puis de saponification puis éventuellement à un réactif d'alkylation ou d'acylation pour obtenir un produit de formule $I_{B2}$ dans laquelle A et B représentent le

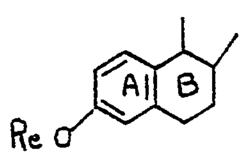

dans laquelle Re a la signification indiquée ci-dessus:

– c/. soit à l'action d'un agent réducteur pour obtenir un produit de formule $I_C$ dans laquelle A et B représentent le groupement:

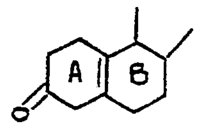

– d/. soit à l'action d'un agent d'oxydation pour obtenir les produits de formule $I_D$ dans laquelle A et B représentent le groupement:

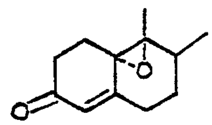

– e/. soit à l'action de l'hydroxylamine $NH_2OH$ libre ou bloquée sous forme $NH_2OR'_3$ dans laquelle $R'_3$ représente un radical alkyle ayant de 1 à 8 atomes de carbone ou un radical aralkyle ayant de 7 à 15 atomes de carbone pour obtenir les produits de formule $I_E$ dans laquelle A et B représentent le groupement:

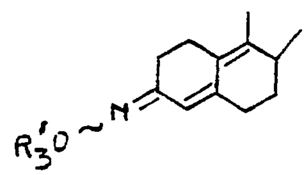

et produits de formule $I_A$, $I_{B1}$, $I_{B2}$, $I_C$, $I_D$ et $I_E$ que l'on soumet éventuellement à l'action d'un acide pour obtenir un sel.

K représente de préférence un groupement cétal ou cétal cyclique tel que de diméthyl ou diéthylcétal, éthylènedioxy ou un thiocétal.

Dans un mode de réalisation préféré du procédé, l'agent de déshydratation capable de libérer la fonction cétone est une résine sulfonique (forme acide), par exemple, une résine sulfonique du commerce à support de polystyrène ou à support de polymère styrène/divinylbenzène; mais on peut également utiliser un acide minéral tel que l'acide chlorhydrique dans l'acide sulfurique dans un alcanol inférieur ou l'acide perchlorique dans l'acide acétique, ou un acide sulfonique comme l'acide paratoluène sulfonique.

Lors de la réaction de déshydratation, les groupements protecteurs que peuvent comporter les produits de formule II sont généralement éliminés. Il en est notamment ainsi des groupements protecteurs de la fonction hydroxy que peuvent porter les substituants $R_3$ ou $R_4$ en position 17. Si tel n'est pas le cas, les groupements protecteurs peuvent être éliminés par les méthodes usuelles, telles que l'hydrolyse alcaline.

L'agent de réduction que l'on utilise pour préparer le produit de formule $I_{B1}$ est de préférence un borohydrure alcalin tel que le borohydrure de sodium et l'on opère au sein d'un alcanol, tel que le méthanol ou l'éthanol.

On obtient alors intermédiairement un produit dans lequel A et B représentent le groupement :

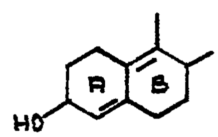

L'agent d'aromatisation que l'on utilise de préférence pour préparer les produits de formule $I_{B1}$ est un acide minéral tel que l'acide chlorhydrique ou l'acide sulfurique, ou encore un agent tel que le pentachlorure de phosphore, le tribromure de phosphore, l'oxychlorure de phosphore, ou encore un anhydride tel que l'anhydride acétique ou trifluoroacétique.

L'agent d'aromatisation que l'on utilise de préférence pour préparer les produits de formule $I_{B2}$ est un halogénure d'acyle par exemple, le bromure d'acétyle ou l'anhydride acétique ou un mélange de ces produits.

L'agent de saponification utilisé est de préférence une base alcaline comme la soude ou la potasse, l'amidure de sodium, le tertbutylate de potassium ou l'acétylure de lithium dans l'éthylène diamine. La réaction a lieu, de préférence au sein d'un alcool inférieur tel que le méthanol ou l'éthanol.

Selon les conditions employées et dans le cas où par exemple le substituant

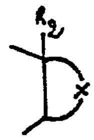

comprend une fonction réactionnelle, par exemple un radical hydroxyle en position 17β, on peut obtenir une acétylation partielle de cette fonction. On recueille alors, en plus du produit 17β -OH attendu, un pourcentage variable de produit 17β -OAc.

Ces produits 17-OH et 17-OAc peuvent être séparés par les méthodes usuelles telles que la chromatographie.

L'alkylation éventuelle est effectuée selon les méthodes usuelles. On utilise un réactif d'alkylation qui est de préférence un halogénure d'alkyle tel que l'iodure d'alkyle ou le sulfate d'alkyle.

On utilise de préférence le sulfate de méthyle.

L'acylation est également réalisée selon les méthodes usuelles.

On utilise de préférence un halogénure d'acyle.

L'agent réducteur que l'on utilise pour transformer les produits de formule $I_A$ en produits de formule $I_C$ est de préférence un métal alcalin dans l'ammoniac liquide. On utilise de préférence le lithium, mais le sodium peut également être envisagé.

Selon les quantités de métal employées, on peut également opérer des modifications à d'autres endroits de la molécule.

L'agent d'oxydation que l'on utilise pour préparer un produit de formule $I_D$ est de préférence un peracide comme l'acide métachloroperbenzoïque, l'acide paracétique ou l'acide perphtalique ou encore l'eau oxygénée seule ou en présence d'hexachloro ou d'hexafluoroacétone.

L'action de l'hydroxylamine ou d'un dérivé est effectuée dans les conditions usuelles.

Dans une variante du procédé décrit ci-dessus, on peut également soumettre un produit de formule $I_A$, $I_{B1}$, $I_{B2}$, $I_C$, $I_D$ et $I_E$ comportant, sur le cycle D, un radical alkynyle à l'action d'un agent de réduction pour obtenir le radical alkényle correspondant. Dans une autre variante du procédé, on peut soumettre un produit de formule $I_A$, $I_{B1}$, $I_{B2}$, $I_C$ et $I_D$ comportant sur le cycle D, un radical :

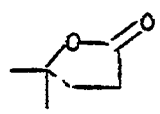

ou

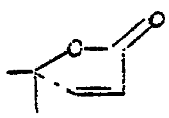

à l'action d'un hydroxyde alcalin ou de l'ammoniaque puis éventuellement à l'action d'un agent acide pour obtenir un produit comportant un radical :

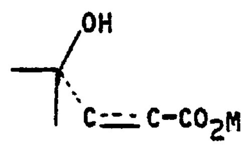

dans lequel le trait pointillé indique la présence éventuelle d'une seconde liaison et M a la signification précédente. Une telle réaction est décrite par exemple dans le brevet français BF. 2.496.669.

L'invention a également pour objet un procédé de préparation des produits de formule générale I comportant un radical acyloxy caractérisé en ce que l'on traite un produit de formule générale $I_A$, $I_{B1}$, $I_{B2}$, $I_C$, $I_D$ ou $I_E$ comportant un radical hydroxy libre par un dérivé d'un acide carboxylique.

Dans un mode préférentiel d'exécution de ce procédé, on traite le produit à acyler par un dérivé tel qu'un anhydride symétrique ou un chlorure d'acide en présence de pyridine ou de 4-dimétylaminopyridine.

On peut également opérer avec une catalyse acide telle que l'acide paratoluène sulfonique.

L'invention a plus particulièrement pour objet un procédé de préparation des produits de formule I' telle que définie ci-dessus caractérisé en ce que l'on met en oeuvre le procédé décrit précédemment au départ d'un produit de formule II' :

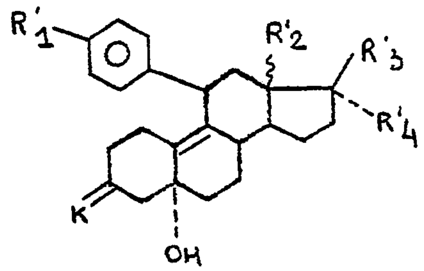

(II')

dans laquelle K, R'$_1$, R'$_2$, R'$_3$ et R'$_4$ ont la signification indiquée précédemment.

L'invention a également pour objet un procédé caractérisé en ce que le produit de départ de formule II est préparé :

- <u>soit</u> en soumettant un produit de formule III :

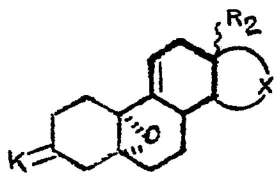

(III)

à l'action d'un produit choisi dans le groupe formé par les produits de formule

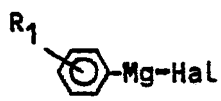 et de formule 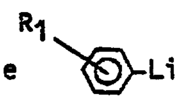

dans lesquelles R$_1$ a la signification indiquée ci-dessus et Hal représente un atome d'halogène, le cas échéant en présence d'halogénure cuivreux :

- <u>soit</u> en soumettant un produit de formule IV :

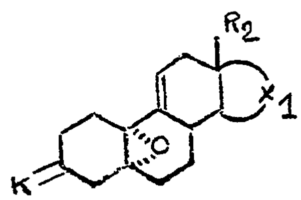

(IV)

dans laquelle le groupement de formule

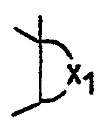

représente :

- soit :

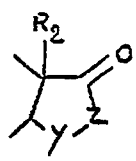

- soit :

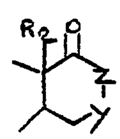

dans lesquels $R_2$, Y et Z ont la signification indiquée ci-dessus à l'action d'un produit choisi dans le groupe formé par les produits de formule

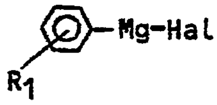 et de formule 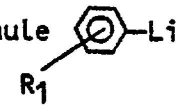

dans lesquelles $R_1$ et Hal ont les significations indiquées précédemment, le cas échéant en présence d'halogénure cuivreux pour obtenir un produit de formule $V_A$ :

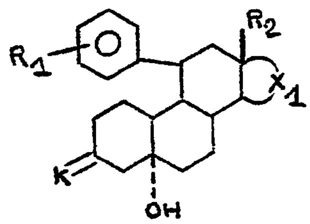

$(V_A)$

produit de formule $V_A$ que l'ou soumet éventuellement à l'action d'un rayonnement ultra-violet pour obtenir un produit de formule $V_B$ :

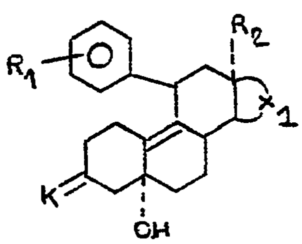

$(V_B)$

produits de formules $V_A$ ou $V_B$ que l'on soumet à l'une des réactions suivantes :
a/. <u>ou bien</u> l'on soumet on produit de formule $V_A$ ou $V_B$ à l'action d'un produit de formule $H\text{-}C{\equiv}C\text{-}CH_2\text{-}O\text{-}Gp$ dans lequel Gp représente un groupement protecteur du radical hydroxyle pour obtenir un produit de formule VI :

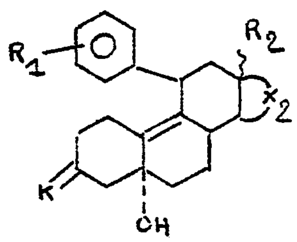

(VI)

dans laquelle

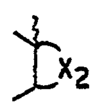

représente :
- soit

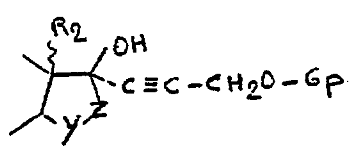

- soit

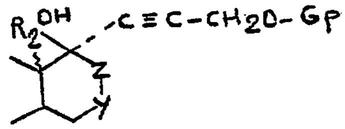

produit de formule VI que l'on soumet à un agent de réduction, puis à un agent de déprotection du radical hydroxyle pour obtenir un produit de formule VII :

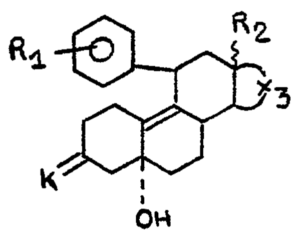

(VII)

dans laquelle

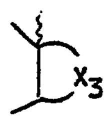

représente :
- soit

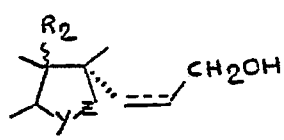

- soit

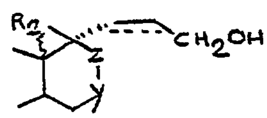

le trait pointillé indiquant la présence éventuelle d'une seconde liaison E ou Z entre les carbones qui le portent et produits de formule VII que l'on soumet :
- soit à un réactif d'oxydation pour obtenir un produit de formule $II_A$ :

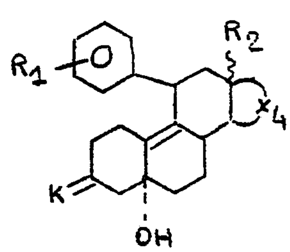

($II_A$)

dans laquelle $X_4$ représente :
- soit

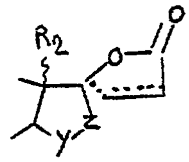

- soit

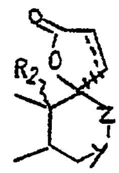

dans lequel le trait pointillé indique la présence éventuelle d'une seconde liaison entre les carbones qui le portent :
- soit l'on soumet le produit de formule VII à un réactif de cyclisation pour obtenir un produit de formule $II_B$ :

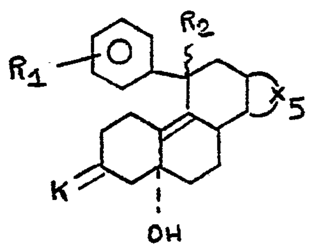

($II_B$)

dans laquelle

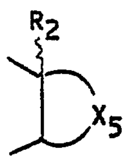

représente :
- soit

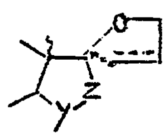

- soit

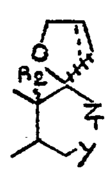

dans lequel le trait pointillé indique la présence éventuelle d'une seconde liaison entre les carbones qui le portent.
b/. ou bien l'on soumet un produit de formule $V_A$ ou $V_B$ d'abord à l'action d'un réactif de formation de l'oxiranne, puis à l'action d'un produit de formule

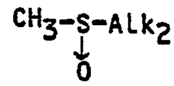

dans laquelle $Alk_2$ représente un radical alkyle ayant de 3 à 5 atomes de carbone et enfin à l'action d'un réactif de cyclisation pour obtenir un produit de formule $II_C$ :

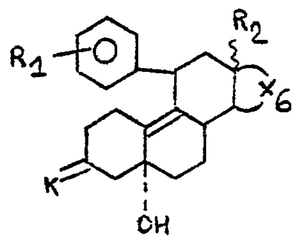

$(II_C)$

dans laquelle :

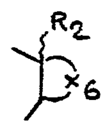

représente :
- soit

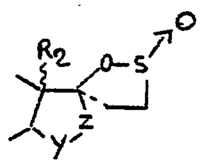

- soit

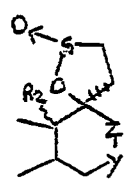

c/. ou bien l'on soumet un produit de formule $V_A$ ou $V_B$ d'abord à l'action d'un réactif de formation de l'oxiranne, puis à l'action d'une alkylamine de formule $H_2NR_5$, $R_5$ ayant la signification indiquée ci-dessus, enfin à l'action d'un dérivé de l'acide carbonique pour obtenir un produit de formule $II_D$ :

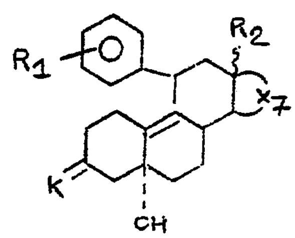

$(II_D)$

dans laquelle :

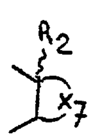

représente :
- soit

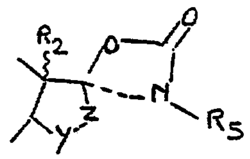

- soit

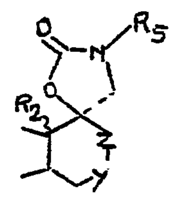

d/. ou bien l'on soumet un produit de formule $V_A$ ou $V_B$ d'abord à l'action d'un réactif de formation de l'oxiranne, puis à l'action d'une alkylamine de formule $H_2NR_5$, enfin à l'action du chlorure de thionyle pour obtenir un produit de formule $II_E$ :

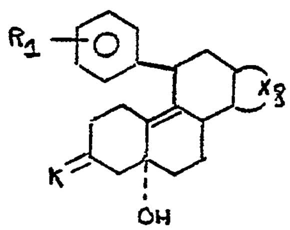

$(II_E)$

dans laquelle :

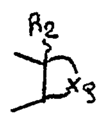

représente :
- soit

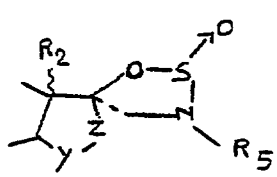

- soit

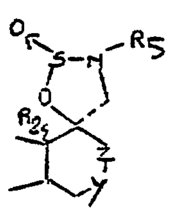

e/. ou bien l'on soumet un produit de formule $V_A$ ou $V_B$ à l'action d'un organomagnésien ou d'un organolithien pour obtenir un produit de formule $II_F$ :

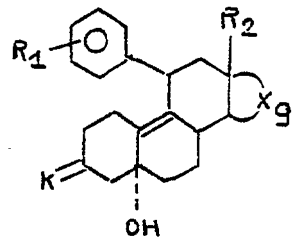

$(II_F)$

dans laquelle :

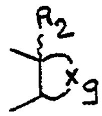

représente :
- soit

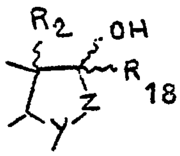

- soit

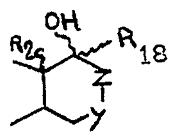

dans lesquels $R_{18}$ représente un radical alkyle, alkényle, alkynyle, ayant au plus 8 atomes de carbone ou un radical aryle, aralkyle, aralkényle ou aralkynyle carbo ou hétérocyclique éventuellement substitués par un ou plusieurs radicaux choisis dans le groupe formé par les radicaux hydroxy, alkoxy ou alkylthio ayant de 1 à 4 atomes de carbone ou halogène.

f/. ou bien l'on soumet un produit de formule $V_A$ ou $V_B$:
– soit à l'action d'un agent de cyanuration, puis à un agent de protection de la fonction hydroxy, puis enfin à l'action d'un magnésien ou d'un lithien,
– soit à l'action d'un lithien de formule

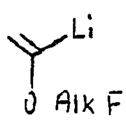

dans laquelle $Alk_f$ représente un radical alkyle ayant de 1 à 4 atomes de carbone, puis à l'action d'une base et d'un acide pour obtenir un produit de formule $II_G$:

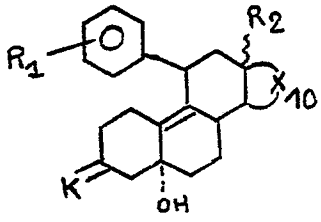

$(II_G)$

dans laquelle

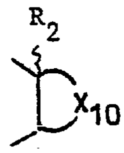

représente :

- soit

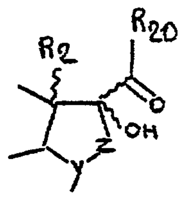

- soit

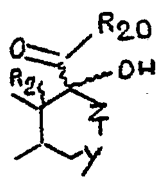

dans laquelle $R_{20}$ représente un radical alkyle éventuellement substitué par hydroxyle produit que l'on soumet éventuellement à l'action d'un réactif d'acylation.

g/. ou bien l'on soumet un produit de formule $V_A$ ou $V_B$ à l'action d'un halogénure de triméthylsulfonium ou du produit de formule $CH_2^{\ominus}S^{\oplus}(CH_3)_2$ en présence d'une base forte pour obtenir un produit de formule $II_H$ :

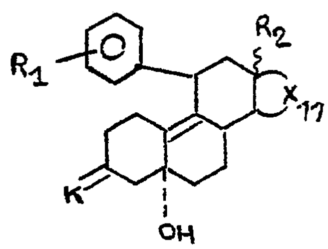

$(II_H)$

dans laquelle

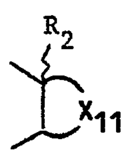

représente :

- soit

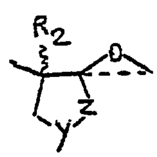

- soit

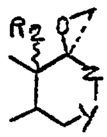

et produit de formule $II_H$ que l'on soumet éventuellement à l'action du produit de formule $CH_2^{-}$-$S^{+}$-$(CH_3)_2$ pour obtenir un produit de formule $II'_H$ :

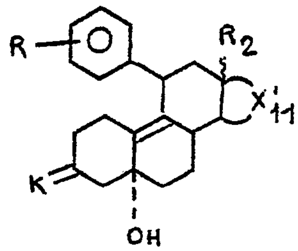

(II'$_H$)

dans laquelle

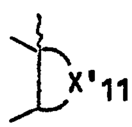

représente :
- soit

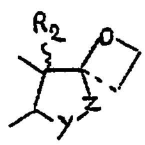

- soit

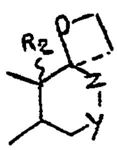

et produit de formule $II_H$ que l'on soumet éventuellement d'abord à une hydrolyse alcaline, puis à l'action d'un produit de formule Hal-$CO_2Alk_g$ dans laquelle Hal représente un atome d'halogène et $Alk_g$ représente un radical alkyle ayant de 1 à 4 atomes de carbone et enfin à l'action d'un carbonate de dialkyle pour obtenir unproduit de formule $II''_H$ :

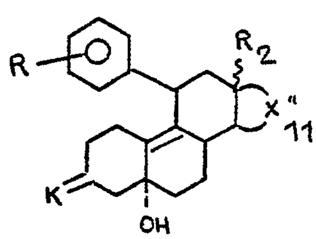

(II"$_H$)

dans laquelle

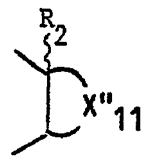

représente :
- soit

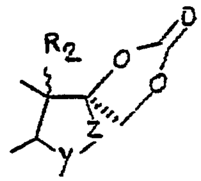

- soit

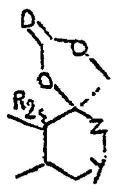

h/. ou bien l'on soumet un produit de formule $V_A$ ou $V_B$ à l'action d'abord d'un lithien de formule

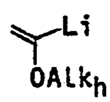

dans lequel $Alk_h$ représente un radical alkyle ayant de 1 à 4 atomes de carbone, puis à l'action d'un réactif d'halogénation, puis enfin à l'action d'une base pour obtenir un produit de formule $II_J$ :

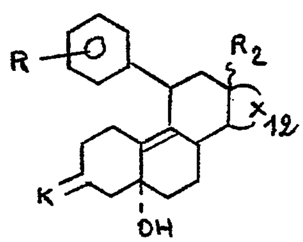

$(II_J)$

dans laquelle

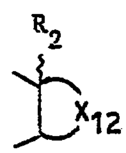

représente :
- soit

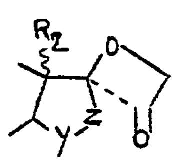

- soit

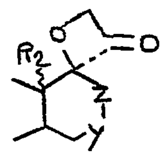

Dans un mode préférentiel d'exécution du procédé, l'action des produits de formules

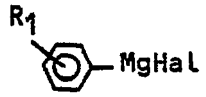

ou

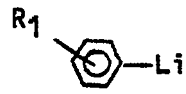

a lieu à température ambiante. Lorsque l'on utilise un magnésien, la réaction a lieu de préférence en présence de sels cuivreux tel que le chlorure cuivreux.

La transformation des produits de formule $V_A$ en produits de formule $V_B$ est effectuée dans de bonnes conditions de rendement. La durée d'exposition à la lampe ultra-violet peut être de l'ordre de 10 minutes à une heure. On utilise de préférence une lampe à quartz au mercure, à haute pression. On opère, par exemple, à température ambiante dans un solvant tel que dioxanne, le cyclohexane, le benzène, le toluène ou le tétrahydrofuranne ou dans un mélange de solvants. La concentration en produit à transformer peut être de l'ordre de 1% ou moins en poids.

Après la réaction, les produits peuvent être purifiés par chromatographie ou tout autre moyen connu de purification.

L'action du produit de formule H-C≡C-$CH_2$OGp est effectuée de préférence par l'intermédiaire d'un dérivé métallique tel que le lithium. On opère, par exemple, comme indiqué dans la demande européenne 0.116.974 en présence de butyllithium. Le groupement protecteur Gp est de préférence le tétrahydropyrannyle.

La réduction des produits de formule VI comportant un radical -C≡C-$CH_2$OGp en position 17α peut être effectuée par l'hydrogène à pression normale, à température ambiante, dans un solvant tel que le méthanol, l'éthanol, le propanol, l'acétate d'éthyle ou le tétrahydrofuranne. On opère en présence d'un catalyseur métallique tel que le palladium à 10% sur charbon. On obtient alors les produits de formule VII dans lesquels aucune insaturation ne subsiste sur les substituants en position 17.

Pour obtenir les produits de formule VII comportant un radical alkényle en position 17α, on opère dans les conditions énoncées par exemple, dans la demande européenne EP.0.147.361. Les produits de configuration Z sont obtenus en hydrogénant les produits acétyléniques à l'aide d'un catalyseur métallique désactivé comme par exemple du palladium à 10 % sur du sulfate de baryum en présence d'une amine ou du palladium sur du carbonate de calcium en présence d'acétate de plomb.

Les produits de configuration E sont obtenus selon les méthodes connues dans la littérature telles que par exemple le sodium dans l'ammoniac liquide ou le lithium dans une amine.

L'élimination du groupement protecteur Gp, si elle n'est pas intervenue au cours de la réaction d'hydrogénation est effectuée par les méthodes usuelles telle que par exemple l'hydrolyse acide en présence d'acide acétique ou chlorhydrique en solution aqueuse. On peut opérer à température ambiante ou à une température de l'ordre de 50°C.

Le réactif d'oxydation que l'on utilise pour préparer les produits de formule $II_A$ peut être choisi parmi les réactifs connus rappelés par exemple dans la demande eruopéenne EP. 0.116.974. On peut par exemple opérer à l'aide du réactif de Jones : anhydride chromique dans l'acide sulfurique dilué, le dichromate ou le chlorochromate de pyridinium, le carbonate d'argent en présence de célite, l'oxygène en présence de platine ou le complexe acide chromique-pyridine.

La cyclisation de produits de formule VII en produits de formule $II_B$ est effectuée à l'aide d'un réactif tel que par exemple le chlorure de tosyle en présence de pyridine.

La préparation des sultines de formule $II_C$ peut être effectuée selon les conditions décrites dans les brevets français BF. 2.285.137 et 2.344.286. La formation de l'oxiranne est effectuée de préférence à l'aide d'iodure de triméthyl sulfonium en présence d'une base forte telle que le tertbutylate de potassium. L'addition du produit de formule

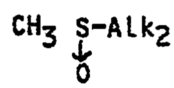

de préférence le méthyltertbutylsulfoxyde est effectuée également en présence d'une base forte par exemple le butyllithium. Le réactif de cyclisation que l'on utilise ensuite est de préférence le N-chloro ou le N-bromo succinimide. Cette dernière réaction peut avoir lieu à température ambiante en présence d'eau.

La réaction de préparation des produits de formule $II_D$ est effectuée en préparant l'oxiranne dans les conditions précédemment indiquées. L'alkylamine $H_2NR_5$, de préférence la méthylamine peut être ajoutée en présence d'acide paratoluène sulfonique, à haute température.

Le dérivé de l'acide carbonique que l'on utilise ensuite est de préférence le diméthyl ester. La réaction se produit au reflux de préférence en présence d'une base forte telle que le tert-butylate de potassium.

Pour la préparation des produits de formule $II_E$, on opère en fin de réaction, à l'aide de chlorure de thionyle en présence de préférence de triéthylamine.

Pour la préparation des produits de formule $II_F$, on opère dans les conditions usuelles décrites notamment ci-dessus et figurant également par exemple dans les brevets européens EP. 0.057.115 et 0.116.974. On peut par exemple opérer en présence de butyllithium.

Pour la préparation des produits de formule $II_G$ à l'aide d'un agent de cyanuration, puis d'un agent de protection de la fonction hydroxy, puis enfin à l'aide d'un magnésien ou d'un lithien, on opère également dans des conditions usuelles. Dans un mode préféré de préparation, on utilise comme agent de cyanuration le cyanure de potassium et comme agent de protection de la fonction hydroxyle, on préfère le chlorotriméthylsilane. La préparation de tels produits est décrite par exemple dans le brevet français BF. 2.082.129. L'acylation éventuelle est effectuée dans les conditions usuelles décrites ci-dessus.

Le lithien de formule

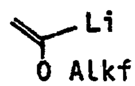

est de préférence le lithien du vinyl éthyl éther. On opère de préférence dans le trétrahydrofuranne. L'action de la base est de préférence, celle de la soude dans le méthanol, celle de l'acide est effectuée par de l'acide chlorhydrique dans le méthanol.

Les produits de formule $II_G$ peuvent également être préparés par hydratation en présence de sels mercuriques d'un produit de formule $II_F$ dans lequel $R_{17}$ représente un radical -C=CH.

La préparation des produits de formule $II_H$ est effectuée selon les méthodes usuelles. Comme base forte, on peut par exemple citer un alcoolate alkalin tel que l'éthylate de sodium ou le tertbutylate de potassium. On peut également utiliser un hydrure alcalin, tel que l'hydrure de sodium. Les conditions de préparation des produits de formule $II'_H$ sont identiques.

Les conditions opératoires préférentielles pour préparer les produits de formule $II''_H$ sont décrites par exemple dans la publication Act. Chim. Hung. 1984, 116(2) 111-23 (CA Vol. 101,1984, p. 801 n° 192272 p.). Le produit de formule $II_H$ est soumis à une hydrolyse alcaline; le produit de formule Hal-$CO_2$-Alkg que l'on fait agir sur le diol obtenu est Cl-$CO_2$-Et de préférence dans un solvant tel que la pyridine et le carbonate de dialkyke est de préférence le carbonate de diéthyle en présence de sodium métal.

La préparation des produits de formule $II_J$ est effectuée de préférence d'abord par le lithien du vinyléthyléther, puis par la N-bromo succinimide, puis enfin par une base telle que la soude.

Les produits de formule I ainsi que leurs sels d'addition avec les acides ou les bases pharmaceutiquement acceptables sont des produits particulièrement intéressants du point de vue pharmacologique.

L'étude des produits sur les récepteurs hormonaux a permis de mettre en évidence des activités progestomimétiques ou anti progestomimétiques, androgènes ou antiandrogènes.

Les produits de formule I possèdent en particulier une remarquable activité anti progestomimétique.

Les produits de formule I possèdent également une activité antiglucocorticoïde comme le montrent les résultats des tests exposés ci-après.

Certains produits montrent cependant une activité antiprogestromimétique supérieure à leur propriété antiglucocorticoïde.

Les produits de formule I ainsi que leurs sels d'addition avec les acides ou les bases pharmaceutiquement acceptables qui possèdent des propriétés anti progestomimétiques peuvent être utilisés comme contraceptifs ; ils peuvent être utilisés contre les dérèglements hormonaux ; ils peuvent par ailleurs présenter un intérêt dans le traitement des cancers hormono-dépendants.

Certains produits de formule I ainsi que leurs sels d'addition avec les acides ou les bases pharmaceutiquement acceptables peuvent également présenter des propriétés progestomimétiques et peuvent ainsi être employés dans le traitement des aménorrhées, des dysménorrhées et des insuffisances lutéales.

Les produits de formule I ainsi que leurs sels d'addition avec les acides ou les bases pharmaceutiquement acceptables peuvent donc être utilisés comme médicaments pour lutter principalement contre les effets secondaires des glucocorticoïdes, ils permettent de lutter également contre les troubles dus à une hypersécrétion de glucocorticoïdes et notamment contre le vieillissement en général et plus particulièrement contre l'hypertension, l'athérosclérose, l'ostéoporose, le diabète, l'obésité ainsi que l'immunodépression et l'insomnie.

Les produits de formule I ainsi que leurs sels d'addition avec les acides ou les bases pharmaceutiquement acceptables qui présentent des propriétés anti-androgènes peuvent être utilisés dans le traitement des hypertrophies et du cancer de la prostate, de l'hyperandrogènie, de l'anémie, de l'hirsutisme et de l'acné.

L'invention a donc pour objet à titre de médicament les produits de formule I pharmaceutiquement acceptables, c'est-à-dire non toxiques aux doses utilisées, ainsi que leurs sels d'addition avec les acides ou les bases pharmaceutiquement acceptables.

L'invention a plus particulièrement pour objet, à titre de médicament les produits de formule générale I':

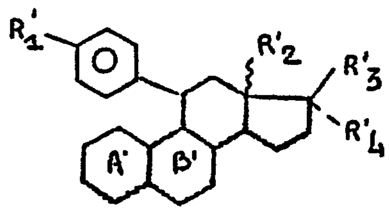

(I')

dans laquelle R'1 représente un radical alkynyle ayant de 2 à 4 atomes de carbone éventuellement substitué par un radical choisi parmi les radicaux hydroxy, halogène ou triméthylsilyle ; $R'_2$ représente un radical méthyle ou éthyle, $R'_3$ et $R'_4$ représentent un radical hydroxyle éventuellement acylé, ou un radical alkyle, alkényle ou alkynyle ayant au plus 8 atomes de carbone et éventuellement substitué par un radical choisi parmi les radicaux hydroxyle ou halogène ou $R'_3$ et $R'_4$ forment ensemble un radical :

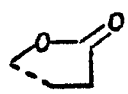

ou

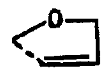

les cycles A' et B' ont l'une des structures suivantes :

a/. soit A' et B' représentent le groupement :

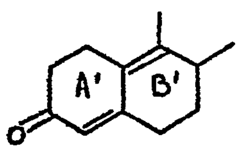

b/. soit A' et B' représentent le groupement :

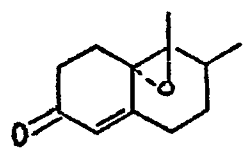

c/. soit A' et B' représentent le groupement :

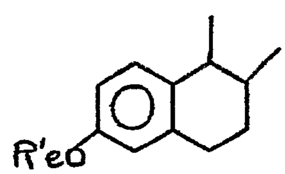

dans lequel R'e représente un atome d'hydrogène ou un radical alkyle ayant de 1 à 4 atomes de carbone et ceux dans laquelle $R'_1$ représente un radical -C≡C-$R_{11}$ dans lequel $R_{11}$ représente un atome d'hydrogène ou un radical méthyle ou triméthylsilyle, $R'_3$ et $R'_4$ sont choisis parmi les radicaux hydroxyle, acétoxy, éthynyle et propynyle éventuellement substitués par un halogène ou par un hydroxyle, propyle et propényle éventuellement substitués par un hydroxy, ou $R'_3$ et $R'_4$ forment ensemble le radical :

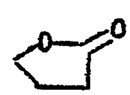

ou

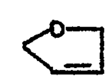

L'invention a plus particulièrement pour objet, à titre de médicaments, les produits de formule I décrits ci-après dans les exemples et plus particulièrement :

- La 11β -(4-éthynylphényl) 17β -hydroxy 17α-(1-propynyl) estra 4,9-dièn-3-one,
- La 11β -(4-éthynylphényl) 17α -allyl 17β-hydroxy estra 4,9-dièn-3-one,
- La 17α-(chloroéthynyl) 11β-(4-éthynylphényl) 17β-hydroxy estra 4,9-dièn-3-one.
- La 17α-(chloroéthynyl) 9α , 10α-époxy 11β-(4-éthynylphényl) 18β-hydroxy estr-4-èn-3-one.

La posologie utile varie en fonction de l'affection à traiter et de la voie d'administration ; elle peut varier par exemple de 10 mg à 1 g par jour chez l'adulte par voie orale.

Les nouveaux produits de formule I et leurs sels pharmaceutiquement acceptables, tels que définis ci-dessus peuvent être employés pour préparer des compositions pharmaceutiques renfermant, à titre de principie actif, l'un au moins desdits produits.

Les produits de formule I et leurs sels pharmaceutiquement acceptables sont utilisés par voie digestive, parentérale ou locale. Ils peuvent être prescrits sous forme de comprimés simples ou dragéifiés, de gélules, de granulés, de suppositoires, de préparations injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émusifiants, les conservateurs.

L'invention a donc pour objet les compositions pharmaceutiques renfermant comme principe actif au moins un produit de formule(I), ou au moins un de ses sels pharmaceutiquement acceptables.

Les produits de formule III ou IV sont connus ou peuvent être préparés par les méthodes usuelles à partir de produits connus. Des méthodes de préparation de ces produits sont décrites par exemple dans les demandes européennes EP. 0.116.974 ou EP. 0 156.284.

Les produits de formule III peuvent également être préparés en soumettant un produit de formule IV ou $IV_B$ :

(IV) 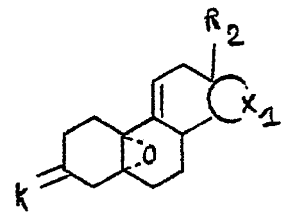

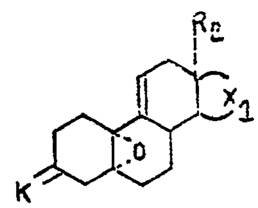 ($IV_B$)

dans laquelle K, $R_2$ et $X_1$ ont la signification précédente aux différentes réactions décrites ci-dessus pour les produits $V_A$ et $V_B$. Le produit $IV_B$ peut être obtenu comme décrit précédemment à partir des produits de formule IV.

En plus, des exemples suivants qui illustrent l'invention sans toutefois la limiter, les produits suivants constituent des produits pouvant être obtenus dans le cadre de la présente invention.

A) Les produits de formule :

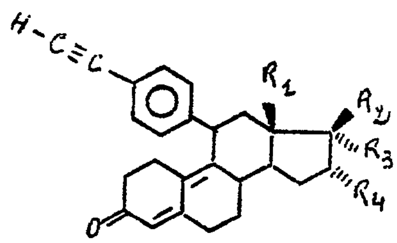

dans laquelle $R_1$, $R_2$, $R_3$ et $R_4$ ont les significations suivantes (le sigle " signifie que le substituant est le même que celui qui précède).

| $R^1$ | $R^2$ | $R^3$ | $R^4$ |
|---|---|---|---|
| $CH_3$ | OH | $C\equiv C-H$ | H |
| " | " | $C\equiv C-CH_2CH_3$ | H |
| " | " | $C\equiv C-(CH_2)_2CH_3$ | H |
| " | " | $C\equiv C$ 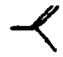 | H |
| " | " | $C\equiv C-CF_3$ | " |
| " | " | $C\equiv C-CF_2CF_3$ | " |
| " | " | $CH=CH_2$ | " |
| " | " | $CH_2-CH_3$ | " |
| " | " | $CH_3$ | " |
| " | " | $CH_2CH_2OH$ | " |
| " | " | $CH_2CH_2CH_2OH$ | " |
| " | " | $CH=CH-CH_3$ (E) | " |
| " | " | $CH=CH-CH_3$ (Z) | " |
| " | " | $CH_2-\underset{CH_3}{\underset{\mid}{C}}=CH_2$ | " |
| " | " | 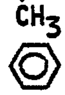 | " |
| " | " | $CF_2CF_3$ | " |
| " | " | $CH_2Ph$ | " |
| " | " | $CH_2CH_2-CO_2H$ | " |
| " | " | $C\equiv C-S\ CH_3$ | " |
| " | " | $CH=C=CH_2$ | " |
| " | " | $CH_2C\equiv C-H$ | " |
| " | " | 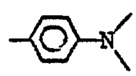 | " |

| $R^1$ | $R^2$ | $R^3$ | $R^4$ |
|---|---|---|---|
| " | O | $CH_2$ | " |
| " | $O-CH_2$ | $CH_2$ | " |
| " | $O-CH_2-CH_2-$ | $CH_2$ | " |
| " | OH | $C(=O)-CH_3$ | H |
| $CH_3$ | OH | $CF=CF_2$ | H |
| " | " | 2-pyridiyl | " |
| " | " | 3-pyridyl | " |
| " | " | 4-pyridyl | " |
| " | " | H | " |
| " | $O-CH_2-CH=CH-$ | | " |
| $C_2H_5$ | OH | $C\equiv C-H$ | H |
| " | " | $C\equiv C-CH_2-CH_3$ | H |
| " | " | $C\equiv C-(CH_2)-CH_3$ | H |
| " | " | $C\equiv C$ | " |
| " | " | $C\equiv C-CF_3$ | " |
| " | " | $C\equiv C-CF_2-CF_3$ | " |
| " | " | CH=CH2 | " |

| $R^1$ | $R^2$ | $R^3$ | $R^4$ |
|---|---|---|---|
| " | " | $CH_2CH_3$ | " |
| " | " | $CH_3$ | " |
| " | " | $CH_2-CH_2-CH_2-OH$ | " |
| " | " | $CH_2-CH_2OH$ | " |
| " | " | $CH=CH-CH_3$ (E) | " |
| | " | $CH=CH-CH_3$ (Z) | " |
| " | " | $CH_2-C(CH_3)=CH_2$ | " |
| | | | " |
| " | " | 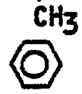 | " |
| " | " | $CF_2-CF_3$ | " |
| " | " | $CH_2-Ph$ | " |
| | | $CH_2-CH_2-CO_2H$ | " |
| " | " | $C=C-SCH_3$ | " |
| " | " | $CH=C=CH_2$ | " |
| " | " | $CH_2-C\equiv C-H$ | " |
| " | " | $(CH_2)_3CH_3$ | " |
| " | O | $CH_2$ | " |
| " | $O-CH_2$ | $CH_2$ | " |
| " | $O-CH_2-CH_2-$ | $CH_2$ | " |
| " | $O-C(=O)-NH-CH_2$ | $CH_2$ | " |
| | " | | $CH_2CH_3$ |
| " | OH | $C(=O)-CH_3$ | H |

| $R^1$ | $R^2$ | $R^3$ | $R^4$ |
|---|---|---|---|
| " | OH | $CF=CF_2$ | H |
| " | " | 2-pyridyl | " |
| " | " | 3-pyridyl | " |
| " | " | 4-pyridyl | " |
| " | " | H | " |
| " | $O-CH_2-CH=CH-$ | | " |

B/. Les produits de formule :

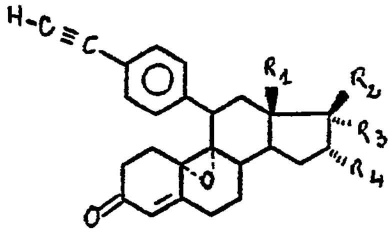

dans laquelle $R_1$, $R_2$, $R_3$ et $R_4$ ont les significations suivantes (le sigle " signifie que le substituant est le même que celui qui précède).

| $R^1$ | $R^2$ | $R^3$ | $R^4$ |
|---|---|---|---|
| $CH_3$ | OH | $C{\equiv}C{-}H$ | H |
| " | " | $C{\equiv}C{-}CH_2CH_3$ | H |
| " | " | $C{\equiv}C{-}(CH_2)_2CH_3$ | H |
| " | " | C≡C 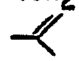 | H |
| " | " | $C{\equiv}C{-}CF_3$ | " |
| " | " | $C{\equiv}C{-}CF_2CF_3$ | " |
| " | " | $CH{=}CH_2$ | " |
| " | " | $CH_2{-}CH_3$ | " |
| " | " | $CH_3$ | " |
| " | " | $CH_2CH_2OH$ | " |
| " | " | $CH_2CH_2CH_2OH$ | " |

| $R^1$ | $R^2$ | $R^3$ | $R^4$ |
|---|---|---|---|
| " | " | $CH=CH-CH_3$ (E) | " |
| " | " | $CH=CH-CH_3$ (Z) | " |
| " | " | $CH_2-\underset{CH_3}{\underset{\mid}{C}}=CH_2$ | " |
| " | " | 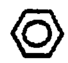 | " |
| " | " | $CF_2CF_3$ | " |
| " | " | $CH_2Ph$ | " |
| " | " | $CH_2CH_2-CO_2H$ | " |
| " | " | $C\equiv C-S\ CH_3$ | " |
| " | " | $CH\equiv C=CH_2$ | " |
| " | " | $CH_2C\equiv C-H$ | " |
| " | " | $(CH_2)_3CH_3$ | " |
| " | O | $CH_2$ | " |
| " | $O-CH_2$ | $CH_2$ | " |
| " | $O-CH_2-CH_2-$ | $CH_2$ | " |
| " | | $CH_2$ | " |
| " | $O-\underset{O}{\underset{\|}{C}}-NH-$ | $CH_2$ | " |
| " | OH | $\underset{O}{\underset{\|}{C}}-CH_3$ | H |
| $CH_3$ | OH | $CF=CF_2$ | H |
| " | " | 2-pyridyl | " |
| " | " | 3-pyridyl | " |
| " | " | 4-pyridyl | " |
| " | " | H | " |
| " | $O-CH_2-CH=CH-$ | | " |

C/. Les produits de formule :

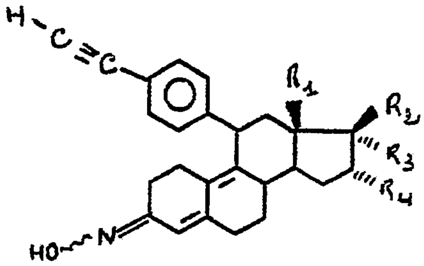

dans laquelle $R_1$, $R_2$, $R_3$ et $R_4$ ont les significations suivantes (le sigle " signifie que le substituant est le même que celui qui précède).

| $R^1$ | $R^2$ | $R^3$ | $R^4$ |
|---|---|---|---|
| $CH_3$ | OH | $C\equiv C-H$ | H |
| " | " | $C\equiv C-CH_2CH_3$ | H |
| " | " | $C\equiv C-(CH_2)_2CH_3$ | H |
| " | " | $C\equiv C$ 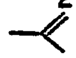 | H |
| " | " | $C\equiv C-CF_3$ | " |
| " | " | $C\equiv C-CF_2CF_3$ | " |
| " | " | $CH=CH_2$ | " |
| " | " | $CH_2-CH_3$ | " |
| " | " | $CH_3$ | " |
| " | " | $CH_2CH_2OH$ | " |
| " | " | $CH_2CH_2CH_2OH$ | " |
| " | " | $CH=CH-CH_3$ (E) | " |
| " | " | $CH=CH-CH_3$ (Z) | " |
| " | " | $CH_2-C=CH_2$<br>$CH_3$ | " |
| " | " | 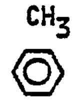 | " |

| $R^1$ | $R^2$ | $R^3$ | $R^4$ |
|---|---|---|---|
| " | " | $CF_2CF_3$ | " |
| " | " | $CH_2Ph$ | " |
| " | " | $CH_2CH_2-CO_2H$ | " |
| " | " | $C\equiv C-S\ CH_3$ | " |
| " | " | $CH=C=CH_2$ | " |
| " | " | $CH_2C\equiv C-H$ | " |
| " | " | $(CH_2)_3CH_3$ | " |
| " | O | $CH_2$ | " |
| " | $O-CH_2$ | $CH_2$ | " |
| " | $O-CH_2-CH_2-$ | $CH_2$ | " |
| " | $O-C(=O)-NH-$ | $CH_2$ | " |
| " | OH | $C(=O)-CH_3$ | H |
| $CH_3$ | OH | $CF=CF_2$ | H |
| " | " | 2-pyridyl | " |
| " | " | 3-pyridyl | " |
| " | " | 4-pyridyl | " |
| " | " | H | " |
| " | $O-CH_2-CH=CH-$ | | " |

D/. Les produits de formule :

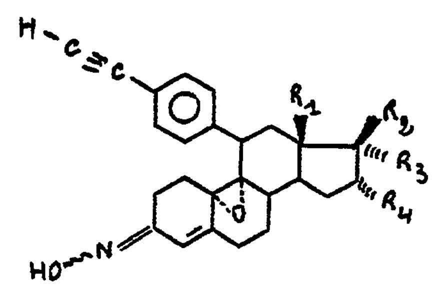

dans laquelle R1, R2, R3 et R4 ont les significations suivantes (le sigle " signifie que le substituant est le même que celui qui précède).

| $R^1$ | $R^2$ | $R^3$ | $R^4$ |
|---|---|---|---|
| $CH_3$ | OH | $C{\equiv}C-H$ | H |
| " | " | $C{\equiv}C-CH_2CH_3$ | H |
| " | " | $C{\equiv}C-(CH_2)_2CH_3$ | H |
| " | " | $C{\equiv}C$ 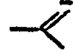 | H |
| " | " | $C{\equiv}C-CF_3$ | " |
| " | " | $C{\equiv}C-CF_2CF_3$ | " |
| " | " | $CH{=}CH_2$ | " |
| " | " | $CH_2-CH_3$ | " |
| " | " | $CH_3$ | " |
| " | " | $CH_2CH_2OH$ | " |
| " | " | $CH_2CH_2CH_2OH$ | " |
| " | " | $CH{=}CH-CH_3$ (E) | " |
| " | " | $CH{=}CH-CH_3$ (Z) | " |
| " | " | 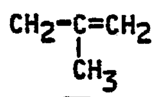 | " |
| " | " | 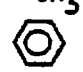 | " |

| $R^1$ | $R^2$ | $R^3$ | $R^4$ |
|---|---|---|---|
| " | " | $CF_2CF_3$ | " |
| " | " | $CH_2Ph$ | " |
| " | " | $CH_2CH_2-CO_2H$ | " |
| " | " | $C\equiv C-S\ CH_3$ | " |
| " | " | $CH=C=CH_2$ | " |
| " | " | $CH_2C\equiv C-H$ | " |
| " | " | $(CH_2)_3CH_3$ | " |
| " | O | $CH_2$ | " |
| " | $O-CH_2$ | $CH_2$ | " |
| " | $O-CH_2-CH_2-$ | $CH_2$ | " |
| " | $O-\underset{\underset{O}{\|}}{C}-NH-$ | $CH_2$ | " |
| " | OH | $\underset{\underset{O}{\|}}{C}-CH_3$ | H |
| $CH_3$ | OH | $CF=CF_2$ | H |
| " | " | 2-pyridyl | " |
| " | " | 3-pyridyl | " |
| " | " | 4-pyridyl | " |
| " | " | H | " |
| " | $O-CH_2-CH=CH-$ | | " |

E/. Les produits de formule :

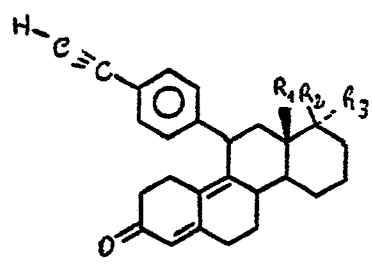

dans laquelle $R_1$, $R_2$, $R_3$ et $R_4$ ont les significations suivantes (le sigle " signifie que le substituant est le même que celui qui précède).

| $R^1$ | $R^2$ | $R^3$ | $R^4$ |
|---|---|---|---|
| $CH_3$ | OH | $C \equiv C-H$ | H |
| " | " | $C \equiv C-CH_2CH_3$ | H |
| " | " | $C \equiv C-(CH_2)_2CH_3$ | H |
| " | " | $C \equiv C$ 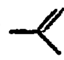 | H |
| " | " | $C \equiv C-CF_3$ | " |
| " | " | $C \equiv C-CF_2CF_3$ | " |
| " | " | $CH=CH_2$ | " |
| " | " | $CH_2-CH_3$ | " |
| " | " | $CH_3$ | " |
| " | " | $CH_2CH_2OH$ | " |
| " | " | $CH_2CH_2CH_2OH$ | " |
| " | " | $CH=CH-CH_3$ (E) | " |
| " | " | $CH=CH-CH_3$ (Z) | " |
| " | " | $CH_2-C=CH_2$<br>$CH_3$ | " |
| " | " | 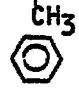 | " |
| " | " | $CF_2CF_3$ | " |
| " | " | $CH_2Ph$ | " |
| " | " | $CH_2CH_2-CO_2H$ | " |
| " | " | $C \equiv C-S\ CH_3$ | " |
| " | " | $CH=C=CH_2$ | " |
| " | " | $CH_2C \equiv C-H$ | " |

| $R^1$ | $R^2$ | $R^3$ | $R^4$ |
|---|---|---|---|
| " | " | $(CH_2)_3CH_3$ | " |
| " | O | $CH_2$ | " |
| " | $O-CH_2$ | $CH_2$ | " |
| " | $O-CH_2-CH_2-$ | $CH_2$ | " |
| " | $O-C(=O)-NH-$ | $CH_2$ | " |
| " | OH | $C(=O)-CH_3$ | H |
| $CH_3$ | OH | $CF=CF_2$ | H |
| " | " | 2-pyridyl | " |
| " | " | 3-pyridyl | " |
| " | " | 4-pyridyl | " |
| " | " | H | " |
| " | $O-CH_2-CH=CH-$ | | " |

Exemple 1 : 17β-hydroxy 17α-(1-propynyl) 11β-14-(1-propynyl) phényl/estra 4,9-dièn-3-one.

Stade A : 3,3-diméthoxy 11β-/4-(propynyl) phényl/ 17α-propynyl-estr-9-ène 5α,17β-diol.

A une solution de 1.49 g de 3,3-diméthoxy 17β-hydroxy 17α-(1-propynyl) estra 5,10-époxy 9(11)-ène dans 15 cm3 de tétrahydrofuranne anhydre, on ajoute sous atmosphère inerte, à +10°C, 150 mg de CuCl, ajoute à la suspension verte 20 cm3 de bromure de 1-propynyl phényl magnésium

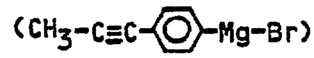

en solution dans le tétrahydrofuranne et titrant 0,75 M/l, agite pendant 1 heure et demie à 20°C, ajoute 20 cm3 d'une solution à 10% de $NH_4Cl$ et 50 cm3 d'eau, agite fortement en présence d'air

$$(Cu \xrightarrow{+O_2} Cu^{++} \xrightarrow{NH_3} Cu(NH_3)_2^{++}$$

soluble et bleu)
extrait par l'acétate d'éthyle, sèche, filtre, concentre à sec par distillation sous pression réduite, chromatographie le résidu sur silice en éluant avec un mélange de chlorure de méthylène et d'acétone (95-5) et isole 1.52 g de produit recherché.

Spectre IR (chloroforme)

absorption à $3602 cm^{-1}$ et $2250 cm^{-1}$ 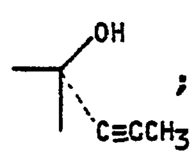 ; $3470 cm^{-1}$ -OH ;

$2220 cm^{-1}$ ø $-C\equiv C-CH_3$.

Spectre RMN (deutérochloroforme)

pic à 0,45 ppm : hydrogènes du 18 Me

pics à 1,06-1,89 ppm : hydrogènes de $\equiv C-CH_3$

pics à 3,21-3,23 ppm : hydrogènes des 2-$OCH_3$

pics à 4,24-4,33 ppm : hydrogène en 11

pic à 4,71 ppm : hydrogène de OH

pics à 7,08-7,17 ppm
pics à 7,24-7,33 ppm } hydrogènes aromatiques

Stade B : 17β-hyroxy 17α-(1-propynyl) 11β-/4-(1-propynyl) phényl/estra 4,9-dièn-3-one.

On mélange 1 g de 3,3-diméthoxy 11β-/4-(propynyl) phényl/ 17α-propynyl estra-9-ène 5α,17β-diol obtenu au stade A, 10 cm3 d'éthanol, 2 g de résine "cedex" préalablement lavée à l'éthanol, porte le mélange réactionnel au reflux, l'y maintient pendant 1 heure et 30 minutes, filtre, concentre le filtrat à sec par distillation sous pression réduite, chromatographie le résidu sur silice en éluant avec un mélange chlorure de méthylène-acétate d'éthyle (97,5-2,5) et obtient 803 mg de composé recherché brut, dissout celui-ci dans 5 cm3 d'éther, ajoute 5 cm3 d'éther isopropylique, amorce la cristallisation, distille l'éther sous pression réduite jusqu'à un volume de 5 cm3 environ, essore, lave à l'éther isopropylique, sèche et isole 539 mg de composé recherché. F = 120-130°C. /α/$_D$ = +147° (c = 0,5%, $CHCl_3$).

Spectre IR (chloroforme)

absorption à 3603$^{cm-1}$ : OH ;

absorption à 2220$^{cm-1}$ et 2250$^{cm-1}$ : C≡C ;

absorption à 1654$^{cm-1}$ et 1600$^{cm-1}$ : C=O et C=C ;

Spectre RMN (deutérochloroforme)

pic à 0,5 ppm : hydrogènes du 18 Me

pics à 1,92 et 2,07 ppm : hydrogènes de $\equiv C-CH_3$

pics de 4,37 à 4,44 ppm : hydrogène en 11

pic à 5,8 ppm : hydrogène en 4

pics à 7,09 et 7,311 ppm: 4H aromatiques quadruplet J = 8 Hz

Spectre UV (éthanol)

λ max : 246 ε = 22000 ;

λ max : 255 ε = 22900 ;

λ max : 301 ε = 18100.

Dichroïsme circulaire (EtoH)

λ = 250 nm Δε = -27,5

λ = 256 nm Δε = -31,2

λ = 301 nm Δε = +22,8

λ = 356 nm Δε = -0,75

Préparation du 1-(4-bromophényl) propyne.

Le 1-(4-bromophényl) propyne qui sert à préparer le magnésien, utilisé au stade A de l'exemple 1, peut être préparé comme suit :

On mélange sous atmosphère inerte 75 g de p-bromo propiophénone et 78,5 g de pentaclhorure de phosphore, chauffe progressivement pour arriver vers 100°C en 1 heure, observe un fort dégagement d'acide chlorhydrique pendant environ 20 minutes, distille le trichlorure de phosphore formé sous pression réduite puis reactifie et obtient 63 g de produit recherché encore impur. On introduit ce dernier dans un mélange de 250 cm3 d'éthanol et 65 g de potasse en pastille, porte au reflux, maintient le reflux pendant 2 heures 30 minutes, verse le mélange réactionnel dans 1 litre d'eau et de glace, extrait à l'éther, lave à l'eau salée, sèche, concentre à sec par distillation sous pression réduite, rectifie et obtient 24,2 g. Eb : 0,1 mbar = 72/74°C.

Exemple 2 : 11β-(3-éthynylphényl) 17β-hydroxy 17α-(1-propynyl) estra 4,9-dièn-3-one.

Stade A: 3,3-diméthoxy 11β-(3-triméthylsilyléthynylphényl) 17α-propynyl estr-9-èn 5α,17β-diol.

On place sous atmosphère inerte 5 g de 3-(triméthylsilyléthynyl) bromobenzène dans 100 cm3 d'éther, ajoute en 5 minutes environ à -5°C, 12,5 cm3 de suspension de n-Buti à 1,6M dans l'hexane, agite pendant 45 minutes à -2°C, refroidit à -7°C et ajoute en 5 minutes par petites fractions 1,9 g de CuI, agite pendant 30 minutes à -5°C, ajoute alors en 5 minutes une solution de 1,5 g de 3,3-diméthoxy 17 -hydroxy 17 -(1-propynyl) estra 5,10-époxy 9(11)-ène dans 15 cm3 d'éther, laisse revenir à température ambiante, agite, essore 1 heure, verse le mélange réactionnel dans 200 cm3 de $NH_4Cl$ M, ajoute 2,5 cm3 d'ammoniaque concentré, agite vigoureusement en présence d'air

$$(Cu^+ \xrightarrow{O_2} Cu^{++} \xrightarrow{NH_4OH} Cu^{++}\ (NH_3)_2$$

bleu soluble), agite pendant 15 minutes, extait à l'éther, sèche, concentre à sec par distillation sous pression réduite, chromatographie le résidu sur silice en éluant avec un mélange chlorure de méthylène-acétate d'éthyle-triéthylamine (97-3-0,4) et isole 720 mg de produit recherché. /α/D = -74,5° (c = 0,6% $CHCl_3$).

Spectre IR (chloroforme)

absorption à 3473 $cm^{-1}$ : OH associé ;

absorption à 2156 $cm^{-1}$ : C≡C-Si≤ ;

absorption à 1237 $cm^{-1}$ et 846 $cm^{-1}$ : $SiMe_3$ ;

absorption à 1102 $cm^{-1}$, 1047 $cm^{-1}$ : cétal ;

absorption à 1596 $cm^{-1}$, 1573 $cm^{-1}$ et 1479 $cm^{-1}$ : aromatiques ;

absorption à 3603 $cm^{-1}$

absorption à 2136 $cm^{-1}$ 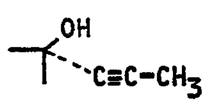

Spectre RMN (deutérochloroforme)

pic à 0,25 ppm : hydrogènes de $Si(CH_3)_3$

pic à 0,45 ppm : hydrogènes du 18 Me

pic à 1,89 ppm : hydrogènes du méthyle de $C{\equiv}C{-}CH_3$

pics à 4,25-4,73 ppm : hydrogène en 11

pics à 7,11-7,37 ppm : hydrogènes du noyau aromatique.

Stade B: 11β-(3-éthynylphényl) 17β-hydroxy 17α-(1-propynyl) estra 4,9-dièn-3-one.

On introduit 680 mg de 3,3-diméthoxy 11β-(3-triméthylsilyléthynyl phényl) 17α-propynyl estr-9-ène 5α,17β-diol, 20 cm3 de méthanol, 0,7 cm3 de solution aqueuse 2N de soude, agite pendant 30 minutes à 20°C, ajoute 1 cm3 de solution aqueuse 2N d'acide chlorhydrique, agite pendant 1 heure à température ambiante, ajoute 1 cm3 de solution aqueuse M de $CO_3HK$, concentre sous pression réduite, ajoute au résidu de l'acétate d'éthyle, lave à l'eau, sèche et concentre à sec par distillation sous pression réduite, chromatographie sur silice en éluant avec un mélange chlorure de méthylène-acétate d'éthyle (95-5) et obtient 383 mg de produit attendu. /α/$_D$ = +62° (c = 0,5% chloroforme).

Spectre IR (chloroforme)

absorption à 3600$^{cm-1}$ : OH ;

absorption à 3304$^{cm-1}$ et 2100$^{cm-1}$ : -C≡C-CH ;

absorption à 2235$^{cm-1}$ : -C≡C-C ;

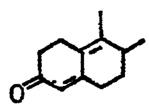

C=O 1675$^{cm-1}$

C=C 1596$^{cm-1}$

absorption à 1576$^{cm-1}$ et 1480$^{cm-1}$ : aromatiques ;

Spectre RMN (deutérochloroforme)

pic à 0,53 ppm : hydrogènes du 18 Me

pic à 1,93 ppm : hydrogènes du méthyle de C≡C-$CH_3$

pic à 3,08 ppm : hydrogène de H-C≡C-<

pics de 4,4 à 4,47 ppm : hydrogène en 11

pic à 5,82 ppm : hydrogène en 4

pics de 7,2 à 7,36 ppm : hydrogènes du noyau aromatique.

Spectre UV (chloroforme)

max : 238 ε = 18300 ;

max : 247 ε = 16100 ;

max : 301 ε = 19800.

Dichroïsme circulaire (dioxane)

214 nm Δε = -6

248 nm Δε = -4,4

280 nm Δε = +9,7

286 nm Δε = +12

300 nm Δε = +14,5

350 nm Δε = -1

Préparation du 3-(triméthylsilyléthynyl) bromobenzène.

Le 3-(triméthylsilyléthynyl) bromobenzène utilisé au départ du stade A de l'exemple 2 peut être préparé comme suit :

Stade A : 1-bromo 3-éthynylbenzène.

Stade 1 : On introduit 38 g de m-bromoacétophénone, 42 g de pentachlorure de phosphore, laisse réagir pendant 15 minutes, chauffe à 70-75° C pendant 1 heure, on distille l'oxychlorure de phosphore formé sous 100 mg de Hg, puis distille à sec sous pression réduite et obtient 37 g de dérivés chlorés intermédiaires. Eb : 5mm/Hg = 90-100° C.

Stade 2 : On introduit 45 g de potasse en pastilles, 150 cm3 d'alcool, agite pendant 30 minutes jusqu'à dissolution totale à 30°C, ajoute en une seule fois 37 g de produit obenu au stade 1, maintient le reflux pendant 2 heures, verse le mélange réactionnel dans 1 litre d'eau glacée, extrait à l'éther, sèche, concentre à sec par distillation sous pression réduite, ajoute 200 cm3 de chlorure de méthylène au résidu, traite au charbon actif, agite, sèche, filtre, concentre le filtrat à sec par distillation sous pression réduite, reactifie sous 10.15 mHg et obtient 9,5 g de produit recherché. Eb 10-15 mm/Hg = 80-85°C.

Stade B : 3-(triméthylsilyléthynyl) bromobenzène.

On introduit sous atmosphère inerte 9,5 g de composé obtenu au stade 2 précédent, dans 100 g de tétrahydrofuranne, ajoute à 25° C ± 3° C en 15 minutes environ 69 cm3 d'une solution 0,8M de bromure d'éthyl magnésium, agite pendant 15 minutes à 25° C puis ajoute en 2 minutes sans refroidir 8 cm3 de chlorure de triméthylsilyle, agite pendant 30 minutes à 25°C, verse le mélange réactionnel sur une solution aqueuse 2M de $ClNH_4$, extrait à l'éther, sèche, filtre, concentre le filtrat à sec par distillation sous pression réduite, rectifie et obtient 12,1 g de produit recherché. Eb : 0,05 mm/Hg = 78-84° C.

Spectre IR (chloroforme)

absorption à 2160$^{cm-1}$ : C≡C ;

absorption à 1250$^{cm-1}$, 874$^{cm-1}$ et 845$^{cm-1}$ : $Si-Me_3$ ;

absorption à 1590$^{cm-1}$, 1581$^{cm-1}$, 1520$^{cm-1}$ et 1503$^{cm-1}$ : noyau aromatique.

Spectre RMN (deutérochloroforme)

pic à 0,23 ppm : hydrogènes à $Me_3Si$

pics de 6,98 à 7,62 ppm : hydrogènes des noyaux aromatiques.

Stade A : 3,3-diméthoxy 17α-propynyl 11β-/4-/(triméthylsilyl) éthynyl/ phényl/ estr-9-èn 5α,17β-diol.

Stade 1 : Préparation du magnésien du (p-bromophényléthynyl) triméthylsilane.

On introduit sous atmosphèere inerte 2 g de magnésium en tournures, 5 cm3 de tétrahydrofuranne anhydre, quelques gouttes de réactif bromé, on amorce la réaction par quelques gouttes de dibromoéthane et chauffage, introduit en 20 minutes environ de manière à maintenir le reflux 18,6 g de (p-bromophényléthynyl) triméthylsilane en solution dans 70 cm3 de tétrahydrofuranne, agite encore 15 minutes au reflux, puis laisse reposer et obtient un magnésien titrant 1M/l.

Stade 2 : Condensation.

On introduit à 0°C sous atmosphère inerte 1,86 g de 3,3-diméthoxy 17β-hydroxy 17α-(1-propynyl) estra 5,10-époxy 9(11)-ène, 20 cm3 de tétrahydrofuranne anhydre, ajoute 100 mg de $Cl_2Cu_2$ puis rapidement 30 cm3 de la solution de magnésien obtenue au stade 1 ci-dessus, on refroidit à 0°C, agite pendant 1 heure à cette température, laisse remonter à +20°C en 30 minutes, verse le mélange réactionnel sur une solution aqueuse saturée de chlorure d'ammonium, extrait à l'acétate d'éthyle, lave à l'eau, sèche, concentre le filtrat à sec par distillation sous pression réduite, chromatographie le résidu sur silice en éluant par un mélange de chlorure de méthylène-acétate d'éthyle (97-3) et obtient 2 g du produit attendu.

<u>Spectre IR</u> (chloroforme)

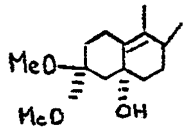

OH associé à $3480^{cm-1}$ ; MeO à $2840^{cm-1}$ ; diméthoxycétal à $1118^{cm-1}$, $1102^{cm-1}$, $1072^{cm-1}$.

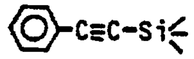

C≡C à $2160^{cm-1}$ ;

aromatiques à $1602^{cm-1}$, $1502^{cm-1}$, $1556^{cm-1}$ ;
$Si(Me)_3$ à $1250^{cm-1}$, $872^{cm-1}$ et $843^{cm-1}$ ;

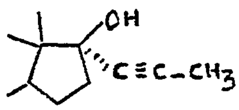

OH à $3600^{cm-1}$
C≡C à $2245^{cm-1}$ ;

<u>Spectre RMN</u> (deutérochloroforme)

pic à 0,24 ppm : hydrogènes de $SiMe_3$
pic à 0,42 ppm : hydrogènes du 18-Me
pic à 1,87 ppm : hydrogènes de ≡C-Me
pics à 3,20 et 3,40 ppm : hydrogènes OMe
pic à 4,28 ppm : hydrogène en 11
pic à 4,70 ppm : hydrogène de OH
pics de 7,15 à 7,37 ppm : hydrogènes aromatiques.

<u>Stade B</u> : 11β-(4-éthynylphényl) 17β-hydroxy 17α-(1-propynyl) estra 4,9-dièn-3-one.

<u>Stade 1</u> : Coupure du silyl.

On introduit sous atmosphère d'azote 1 g de dérivé silylé obtenu au stade A, 95 cm3 de méthanol, 5 cm3 d'eau, agite jusqu'à dissolution, ajoute 1 cm3 d'ammoniaque 28% (12N), porte le mélange réactionnel au reflux et agite pendant 2 heures, ajoute 0,2 cm3 de solution aqueuse 10N de soude, agite pendant 15 minutes au reflux et obtient la solution A.

<u>Stade 2</u> : Déshydratation, décétalysation.

On laisse revenir la solution A vers 40°C, ajoute 15 cm3 de solution aqueuse 2N d'acide chlorhydrique, agite pendant 15 minutes à 40°C, refroidit, dilue à l'eau, extrait à l'acétate d'éthyle, lave au bicarbonate de sodium puis à l'eau, sèche, concentre le filtrat à sec par distillation sous pression réduite, ajoute 5 cm3 d'éther au résidu, amorce et laisse cristalliser 16 heures, essore, lave, sèche et obtient 383 mg de produit recherché. F = 155°C.
$/\alpha/_D$ = +140,5° (c = 0,5%, chloroforme).

Les liqueurs mères sont chromatographiées sur silice en éluant par le mélange benzène-acétate d'éthyle (8-2), on isole 335 mg de produit brut que l'on cristallise dans l'éther et obtient 208 mg de produit recherché. F = 155°C.

Spectre IR (chloroforme).

-OH à 3600 $cm^{-1}$ ;

-C≡C-Me à 2240 $cm^{-1}$ ;

-C≡C-H { ≡CH à 3305 $cm^{-1}$ ; C≡C à 2105 $cm^{-1}$ }

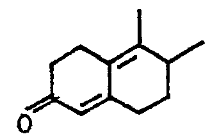

C=O à 1657 $cm^{-1}$

C=C à 1600 $cm^{-1}$ ;

C ø C à 1590 $cm^{-1}$, 1505 $cm^{-1}$ et 835 $cm^{-1}$.

Spectre RMN (deutérochloroforme)

pic à 0,5 ppm : hydrogènes du 18-Me

pic à 1,92 ppm : hydrogènes du ≡C-Me

pic à 3,06 ppm : hydrogènes de ≡C-H

pics de 4,41 à 4,48 ppm : hydrogènes en 11

pic à 5,82 ppm : hydrogène en 4

pics de 7,12 à 7,20 ppm } hydrogènes du noyau aromatique

pics de 7,15 à 7,37 ppm }

Spectre UV (éthanol)

244 nm $\varepsilon$ = 22300 ;

253 nm $\varepsilon$ = 22300 ;

302 nm $\varepsilon$ = 20200.

Dichroïsme circulaire (dioxane)

248 nm $\Delta\varepsilon$ = -30

254 nm $\Delta\varepsilon$ = -32

300 nm $\Delta\varepsilon$ = +21,2

355 nm $\Delta\varepsilon$ = -0,9.

Préparation

Le parabromophényléthynyl triméthylsilane utilisé au stade 1 du stade A de l'exemple 3 peut être préparé comme suit :

Stade A : p-bromophényl acétylène.

Stade 1 : Préparation de dérivés chlorés intermédiaires par action du pentachlorure de phosphore sur la p-bromoacétophénone.

On introduit 95 g de p-bromoacétophénone, 107 g de pentachlorure de phosphore, chauffe à 70°C, observe, après fusion, un important dégagement d'acide chlorhydrique, maintient à cette température

pendant 10 minutes, rectifie sous pression réduite et obtient une première fraction (10 g ) correspondant au produit monochloré

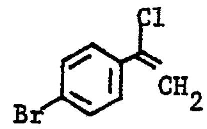

et distillant à 95°C sous 5 mm/Hg et une deuxième fraction eb : 100-102°C sous 5 mm/Hg et correspondant au dérivé chloré

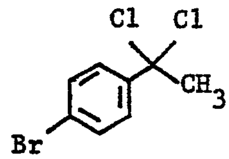

Les deux produits conduisant après traitement au même dérivé, nous les avons joints ; poids total 83,5 g (mélange A).

<u>Stade 2</u> : Préparation du p-bromophénylacétylène par action de la potasse sur les dérivés halogénés intermédiaires.

On introduit le mélange A obtenu au stade 1 dans 430 g de solution à 25% de potasse dans l'éthanol, porte au reflux et maintient pendant 3 heures, refroidit, verse le mélange réactionnel sur 2,5 l d'eau glacée, extrait à l'éther, lave à l'eau, sèche, filtre, concentre le filtrat à sec par distillation sous pression réduite, rectifie sous pression réduite et obtient 26,5 g de produit attendu.
Eb : 10 mm/Hg = 90°C.

<u>Stade B</u> : p-bromophényléthynyl triméthylsilane.

<u>Stade 1</u> : Préparation du bromure d'éthyl magnésium.

On introduit sous atmosphère inerte 8,5 g de tournure de magnésium, 20 cm3 d'éther, ajoute quelques gouttes de bromure d'éthyle, chauffe pour amorcer la réaction, ajoute en 1 heure de manière à maintenir le reflux 25 cm3 de bromure d'éthyle dans 300 cm3 d'éther, agite encore pendant 30 minutes après la fin de l'introduction puis laisse reposer. titre : 0,7 mole/l.

<u>Stade 2</u> : Préparation du dérivé silylé.

On ajoute goutte à goutte en 30 minutes 26,4 g de p-bromophényl acétylène en solution dans 100 cm3 de tétrahydrofuranne dans 215 cm3 de la solution de magnésien préparée ci-dessus. La réaction est vive et immédiatement accompagnée d'un dégagement important d'éthane et d'une élévation de la température. On ramène la température à 15-20°C, agite encore pendant 10 minutes et ajoute goutte à goutte en 5 minutes 20 cm3 de chlorotriméthylsilane ; on laisse revenir à température ambiante, agite pendant 30 minutes, dilue par addition de 200 cm3 de solution aqueuse saturée de $ClNH_4$, décante, extrait au tétrahydrofuranne, lave à l'eau salée, sèche et concentre à sec par distillation sous pression réduite. On reprend le résidu huileux par 150 cm3 de méthanol, amorce la cristallisation, essore, lave, sèche et obtient 18,67 g de produit recherché. F≃ 65°C.

<u>Exemple 4 : 11β-(4-éthynylphényl) 17α-allyl 17β-hydroxy estra-4,9-dièn-3-one.</u>

<u>Stade A</u> : 3,3-éthylènedioxy 5α-hydroxy 11β-/4-(2-triméthylsilyléthynyl) phényl/ 17-céto estr-9-ène.

On introduit sous atmosphère d'azote 22,8 g de 3,3-éthylènedioxy 5,10-époxy 17-céto estr-9-ène, 276 cm3 de tétrahydrofuranne, agite jusqu'à dissolution, amène la température à 0°C, verse 1,38 g de CuCl, agite pour dissoudre, on verse rapidement 182 cm3 d'une solution de Br

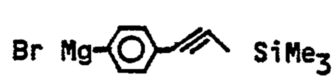

titrant 0,75 mole/l. On laisse la température redescendre à 0°C et on agite pendant 1 heure à cette température. On laisse remonter à 20°C en 30 minutes. On ajoute 1400 cm3 de solution saturée de $NH_4Cl$, ex-

trait à l'acétate d'éthyle, lave par une solution aqueuse saturée de chlorure de sodium, sèche, filtre, concentre le filtrat à sec par distillation sous pression réduite, chromatographie le résidu sous pression en éluant avec un mélange chlorure de méthylène-acétate d'éthyle (9-1) et recueille 5,9 g de produit recherché. F = 182°C.

Spectre IR (chloroforme)

OH à 3510cm-1 ;
C≡C fort à 2155cm-1 ;
aromatiques 1602-1555-1500cm-1 ;
17-cétone à 1732cm-1 ;
$SiMe_3$ : probable.

Spectre RMN (deutérochloroforme)

pic à 0,24 ppm : hydrogènes de 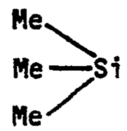 ;

pic à 0,47 ppm : hydrogènes du 17-Me

pic à 3,99 ppm : hydrogènes de 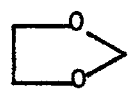

pics de 4,29 à 4,38 ppm : hydrogènes en 11
pic à 4,41 ppm : hydrogène de OH en 5
pics de 7,14 à 7,23 ppm }
pics de 7,37 à 7,46 ppm } hydrogènes du noyau aromatique

Préparation.

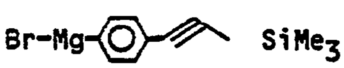

utilisé au stade A de l'exemple 4 peut être préparé comme suit :
On prépare un mélange de 43,74 g de

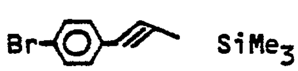

SiMe3 dans 165 cm3 de tétrahydrofuranne (mélange A), et verse sous atmosphère inerte quelques gouttes de ce mélange dans 4,70 g de magnésium en poudre en suspension dans 12 cm3 de tétrahydrofuranne. On amorce par léger chauffage à 72°C et continue l'addition de façon à maintenir la température à 68-71°C, puis porte au reflux pendant 35 minutes. On obtient une solution du magnésien désiré titrant 0,75 mole/l.

Stade B : 3,3-éthylènedioxy 5α-hydroxy 11β-/4-(2-triméthylsilyléthynyl) phényl/ 17β-hydroxy 17α-allyl estr-9-ène.

Dans un ballon, on introduit la totalité du magnésien préparé à partir de 43 mmoles de bromure d'allyle soit 26 mmoles environ d'allyl Mg-Br. On ajoute goutte à goutte en agitant 3,01 g de 3,3-éthylènedioxy 5α-hydroxy 11β-/4-(2-triméthylsilyléthynyl) phényl/ 17-céto estr-9-ène en solution dans 32 cm3 de tétrahydrofuranne en 7 minutes. Après addition, on agite pendant 1 heure à 20°C. On ajoute 62 cm3 de solution aqueuse saturée de $NH_4Cl$, extrait à l'éther, lave par une solution aqueuse saturée de chlorure de sodium, sèche, filtre, concentre le filtrat à sec par distillation sous pression réduite, chromatographie le résidu sur silice en éluant par un mélange chlorure de méthylène-acétate d'éthyle (95-5) puis par un mélange chlorure de méthylène-acétate d'éthyle (85-15) et obtient 2,37 g de produit recherché. F = 192-193°C.

Spectre IR

OH associé à $3505 cm^{-1}$ ;

cétal ;

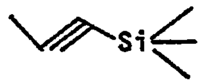

| | |
|---|---|
| Si-C≡C | $2155 cm^{-1}$ très forte |
| SiMe | $1250 cm^{-1}$, $844 cm^{-1}$, $834 cm^{-1}$ ; |

allyle

| | |
|---|---|
| =C-H | $3077 cm^{-1}$ |
| C=C | $1635 cm^{-1}$ |
| déformation | $1000 cm^{-1}$ épaulement. |

Spectre RMN (deutérochloroforme)

pic à 0,24 ppm : hydrogènes de 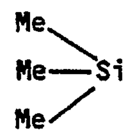

pic à 0,49 ppm : hydrogènes en 18

pic à 3,98 ppm : hydrogènes de 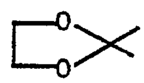

pics de 4,28 à 4,34 ppm : hydrogènes en 11

pic à 4,41 ppm : hydrogène de OH en 5

pics à 5,05-5,24 ppm : hydrogène de $-CH=CH_2$

pics à 5,83-6,22 ppm : hydrogènes de $-CH=CH_2$

pics à 7,11-7,21 ppm et pics à 7,34-7,44 ppm : hydrogènes du noyau aromatique.

Stade C : 11β-(4-éthylnylphényl) 17α-allyl 17β-hydroxy estra-4,9-dièn-3-one.

1ère étape : coupure du silyle.

On introduit 2,14 g de 3,3-éthylènedioxy 5α-hydroxy 11β-/4-(2-triméthylsilyléthynyl) phényl/ 17β-hydroxy 17α-allyl estr-9-ène dans 200 cm3 d'un mélange méthanol-eau (95-5). On chauffe à 40°C en agitant jusqu'à dissolution totale, ajoute 2,17 cm3 de $NH_4OH$ ( ≃ 12N), porte au reflux, pendant 2 heures et 15 minutes, ajoute 0,78 cm3 de solution aqueuse 5N de soude, agite encore pendant 15 minutes au reflux, laisse la température redescendre à 38°C.

2ème étape : déblocage de la cétone et déshydratation.

Au mélange réactionnel à 38°C obtenu ci-dessus, on ajoute 33 cm3 de solution aqueuse 2N d'acide chlorhydrique, agite pendant 15 minutes à 20°C, dilue par 200 cm3 d'eau légèrement salée, extrait à l'acétate d'éthyle, lave par une solution aqueuse saturée de chlorure de sodium, sèche, concentre le filtrat à sec par distillation sous pression réduite, chromatographie le résidu sur silice en éluant avec un mélange chlorure de méthylène-acétate d'éthyle (95-5) et obtient 1,35 g de produit recherché. F = 116°C.

Spectre IR

OH complexe 3620 cm$^{-1}$, 3600 cm$^{-1}$, 3560 cm$^{-1}$

-C≡C-H | ≡C-H 3305 cm$^{-1}$
C≡C 2108 cm$^{-1}$

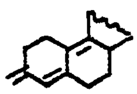 | C=O 1655 cm$^{-1}$
C=C 1603 cm$^{-1}$

.groupe allyle | =C-H 3080 cm$^{-1}$
C=C 1440 cm$^{-1}$ épaulement
déformation 998 cm$^{-1}$
" 919 cm$^{-1}$

noyau aromatique 1593 cm$^{-1}$ épaulement
1558 cm$^{-1}$
1503 cm$^{-1}$

Spectre RMN (deutérochloroforme)

pic à 0,55 ppm : hydrogènes de 18-Me
pic à 3,07 ppm : hydrogènes de H-C≡C-
pics à 4,41-4,48 ppm : hydrogènes en 11
pics à 5,12-5,31 ppm : hydrogène de -CH=$CH_2$
pic à 5,81 ppm : hydrogène en 4
pics de 5,83 à 6,22 ppm : hydrogènes de -CH=$CH_2$
pics à 7,11-7,21 ppm et
pics à 7,35-7,49 ppm } hydrogènes du noyau aromatique.

Exemple 5 : 17α-(chloroéthynyl) 11β-(4-éthynylphényl) 17β-hydroxy estra-4,9-dièn-3-one.

Stade A : 3,3-éthylènedioxy 17α-chloroéthynyl 11β-/4-/(triméthylsilyl) éthynyl/ phényl/ estr-9-ène 5α,17β-diol.

Dans 10 cm3 de n-butyllithium dans l'hexane 1,6M, on ajoute lentement à -5°C, 25 cm3 d'éther puis goutte à goutte 0,67 cm3 de cis dichloroéthylène en solution dans 5 cm3 d'éther, agite pendant 10 minutes la suspension obtenue, ajoute rapidement 1,01 g de 3,3-éthylènedioxy 5α-hydroxy 11β-/4-(2-triméthylsilyléthynyl) phényl/ 17-céto estr-9-ène en solution dans 5 cm3 de tétrahydrofuranne, laisse revenir à 20°C, agite pendant 30 minutes, verse le mélange réactionnel dans une solution aqueuse de $NH_4Cl$, extrait à l'éther, sèche, filtre, concentre le filtrat à sec par distillation sous pression réduite, chromatographie le résidu sur silice en éluant par un mélange chlorure de méthylène-acétate d'éthyle (95-5), cristallise dans l'éther isopropylique et obtient 884 mg de produit recherché.

Spectre IR (chloroforme)

Présence de 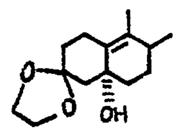 OH associé à 3500 $cm^{-1}$

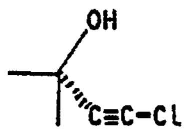 OH 3600 $cm^{-1}$

C≡C 2220 $cm^{-1}$

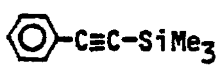 C≡C 2157 $cm^{-1}$

aromatiques 1602 $cm^{-1}$

1555 $cm^{-1}$

1500 $cm^{-1}$

$SiMe_3$ 1250 $cm^{-1}$

867 $cm^{-1}$

845 $cm^{-1}$

Spectre RMN (deutérochloroforme)

pic à 0,25 ppm : hydrogènes de $Me_3Si$-

pic à 0,45 ppm : hydrogènes du 18-Me

pics à 3,74-4,44 ppm : hydrogènes des OH

pic à 3,97 ppm : hydrogènes de 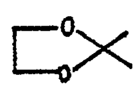

pics à 4,3-4,37 ppm : hydrogène en 11

pics à 7,17-7,2 ppm
pics à 7,33-7,43 ppm } hydrogènes du noyau aromatique.

Stade B: 17α-(chloroéthynyl) 11β-(4-éthynylphényl) 17β-hydroxy estra-4,9-dièn-3-one.

A une solution de 860 mg de 3,3-éthylènedioxy 17α-chloroéthynyl 11β-/4-(triméthylsilyl) éthynyl/ phényl/ estr-9-ène 5α,17β-diol obtenu au stade A dans 30 cm3 de méthanol, on ajoute 3 cm3 de solution aqueuse N de soude, agite à 50°C pendant 20 minutes puis pendant 1 heure à 20°C. On ajoute 7 cm3 de solution aqueuse N d'acide chlorhydrique et 30 cm3 de méthanol, laisse à 20°C pendant 30 minutes, réduit le volume de solvant par distillation sous pression réduite, ajoute de l'éther, verse le mélange réactionnel dans une solution aqueuse saturée de chlorure de sodium, extrait à l'éther, sèche, filtre, concentre le filtrat à sec par distillation sous pression réduite, chromatographie le résidu sur silice en éluant par un mélange chlorure de méthylène-acétate d'éthyle (95-5), empâte dans un mélange d'éthanol et d'eau, sèche et obtient 450 mg de produit recherché.

Spectre IR (chloroforme)

Absence de cétal

| | | |
|---|---|---|
| Présence de OH | $3600 cm^{-1}$ | |
| C≡C-Cl | $2212 cm^{-1}$ | (C≡C) |
| C≡C-H | $3302 cm^{-1}$ | (≡C-H) |
| | $2100 cm^{-1}$ | (C≡C-) |
| diénone | $1657 cm^{-1}$ | (C=O) |
| | $1602 cm^{-1}$ | (C=C) |
| aromatique | $1555 cm^{-1}$ | |
| | $1503 cm^{-1}$ | |

Spectre RMN (deutérochloroforme)

| | |
|---|---|
| pic à 0,5 ppm | : hydrogènes de 18-Me |
| pic à 3,05 ppm | : hydrogènes du H-C≡C- |
| pic à 4,44 ppm | : hydrogènes en 11 |
| pic à 5,8 ppm | : hydrogènes en 4 |
| pics à 7,11-7,21 ppm<br>pics à 7,38-7,48 ppm | hydrogènes du noyau aromatique. |

Exemple 6 : 17-(chloroéthynyl) 11β-(4-éthynylphényl) 17 -hydroxy 13α -estra-4,9-dièn-3-one.

Stade A : 3,3-éthylènedioxy 5α-hydroxy 11β-/4-/triméthylsilyléthynyl/ phényl/ 13α-estr-9-èn-17-one.

Une solution de 3,7 g de 3,3-éthylènedioxy 5α-hydroxy 11β-/4-(2-triméthylsilyléthynyl) phényl/ 17-céto estr-9-ène dans 650 cm3 de dioxane est irradiée pendant 3 heures avec une lampe à vapeur de mercure plongeante (Hanan TQ150) en maintenant la température de la solution à 22-24°C, on concentre à sec par distillation sous pression réduite, chromatographie le résidu sur silice en éluant par un mélange hexane-acétate d'éthyle (4-6) à 0,1% de triéthylamine et obtient 1,6 g de produit recherché. F = 182°C.

<u>Spectre IR</u> (chloroforme)

Présence de 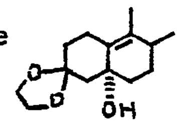 OH associé à $3508^{cm^{-1}}$

$-C\equiv C-SiMe_3$   $C\equiv C$   $2155^{cm^{-1}}$

$SiMe_3$   $1250^{cm^{-1}}$, $864^{cm^{-1}}$, $844^{cm^{-1}}$

17-céto   $1730^{cm^{-1}}$

<u>Spectre RMN</u> (deutérochloroforme)

pic à 1,11 ppm : hydrogènes de 18-Me

pic à 3,94 ppm : hydrogènes de 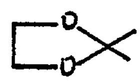

pic à 4,33 ppm : hydrogènes de l'hydroxyle en 5

pics à 7,03-7,12 ppm
pics à 7,33-7,43 ppm
hydrogènes du noyau aromatique.

<u>Stade B</u> : Isomère A et isomère B du 17-chloroéthynyl 17-hydroxy 11β-/4-(triméthylsilyléthynyl) phényl/ 13α-estra-4,9-dièn-3-one.

On introduit 10 cm3 de suspension de n-Buli dans l'hexane, titrant 1,6M, agite sous atmosphère inerte à -5°CC, ajoute lentement 25 cm3 d'éther puis goutte à goutte 0,67 cm3 de <u>cis</u> dichloroéthylène en solution 5 cm3 d'éther, obtient une suspension, agite encore pendant 10 minutes, ajoute 950 mg de 3,3-éthylènedioxy 5α-hydroxy 11β-/4-(triméthylsilyléthynyl) phényl/ 13α-estr-9-èn-17-one, agite pendant 30 minutes, verse le mélange réactionnel dans une solution aqueuse de $NH_4Cl$, extrait à l'éther, sèche, filtre, concentre le filtrat à sec par distillation sous pression réduite, chromatographie le résidu sur silice en éluant avec un mélange chlorure de méthylène-acétate d'éthyle (9-1) à 0,2% de triéthylamine et obtient 115 mg de l'isomère A du 17-chloroéthynyl 17-hydroxy 11β-/4-(triméthylsilyléthynyl) phényl/ 13α-estra-4,9-dièn-3-one et 235 mg de l'isomère B du 17-chloroéthynyl 17-hydroxy 11β-/4-(triméthylsilyléthynyl) phényl/ 13α-estra-4,9-dièn-3-one.

<u>Spectre IR de l'isomère A</u> (chloroforme)

| | |
|---|---|
| OH | : 3600 $cm^{-1}$ |
| C≡C de C≡C-Cl | : 2212 $cm^{-1}$ |
| C=O conjugué | 1654 $cm^{-1}$<br>1604 $cm^{-1}$ |
| C≡C de -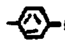-C≡C-SiMe$_3$ | 2155 $cm^{-1}$ |
| aromatique | 1604 $cm^{-1}$<br>1504 $cm^{-1}$ |
| SiMe$_3$ | 1250 $cm^{-1}$<br>864 $cm^{-1}$<br>845 $cm^{-1}$ |

<u>Spectre IR de l'isomère B</u> (chloroforme)

| | |
|---|---|
| OH | : 3601 $cm^{-1}$ |
| C≡C de C≡C-Cl | : 2250 $cm^{-1}$ |
| C=O conjugué | 1653 $cm^{-1}$<br>1621 $cm^{-1}$ |
| C≡C de -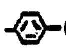-C≡C-SiMe$_3$ | 2156 $cm^{-1}$ |
| aromatique | 1605 $cm^{-1}$<br>1503 $cm^{-1}$ |

<u>Stade C</u> : Isomère A du 17-chloroéthynyl 11β-(4-éthynylphényl) 17-hydroxy 13α-estra-4,9-dièn-3-one.

A une solution de 100 mg de l'isomère A du 17-chloroéthynyl 17-hydroxy 11 β-/4-/(triméthylsilyl) éthynyl/ phényl/ 13α-estra-4,9-dièn-3-one dans le méthanol, on ajoute sous atmosphère inerte 0,4 cm3 de solution aqueuse N de soude, laisse à 20°C pendant 40 minutes, ajoute 1 cm3 de solution aqueuse N d'acide chlorhydrique, évapore une partie du solvant, ajoute de l'éther, lave par une solution de bicarbonate de sodium, sèche, filtre, concentre à sec par distillation sous pression réduite, empâte le résidu dans l'éther isopropylique et obtient 55 mg de composé attendu dont on n'a pas déterminé la configuration en 17. D'après la littérature (Stéroids<u>44</u>, p. 349) il devrait s'agir du 17β-OH car provenant du produit minoritaire lors de la substitution en 17.

Spectre UV (éthanol)

max. 241 nm $E^1_1$ = 591 $\varepsilon$ = 25500 ;

max. 251 nm $E^1_1$ = 569 $\varepsilon$ = 24500 ;

Infl. 276 nm $E^1_1$ = 207 ;

Infl. 285 nm $E^1_1$ = 304 ;

max. 306 nm $E^1_1$ = 492 $\varepsilon$ = 21200.

Spectre RMN (deutérochloroforme)

pic à 1,18 ppm : hydrogènes de 18-Me

pic à 3,05 ppm : hydrogènes du H-C≡C-

pic de 3,75 à 3,97 ppm : hydrogènes en 11

pic à 5,71 ppm : hydrogènes en 4

pics à 7,08-7,17 ppm } hydrogènes du noyau aromatique.
pics à 7,4 -7,5 ppm }

Stade D : Isomère B du 17-chloroéthynyl 11β -(4-éthynylphényl) 17-hydroxy 13α-estra-4,9-dièn-3-one.

On dissout 225 mg de l'isomère B du 17-chloroéthynyl 17-hydroxy 11β-/4-/(triméthylsilyl) éthynyl/ phényl/ 13α-estra-4,9-dièn-3-one dans 10 cm3 de méthanol, fait barboter de l'azote, ajoute 1 cm3 de solution aqueuse N de soude, agite pendant 30 minutes sous azote à 20°C, ajoute 3 cm3 de solution aqueuse N d'acide chlorhydrique , évapore une partie du solvant sous pression réduite, ajoute de l'eau salée, extrait à l'éther, sèche, filtre, concentre le filtrat à sec par distillation sous pression réduite, chromatographie le résidu sur silice en éluant par un mélange chlorure de méthylène-acétate d'éthyle (9-1) et obtient 166 mg du composé attendu.

La configuration en 17 de ce produit n'a pas été démontrée. Si l'on se base sur les données de la littérature (Stéroids 44, p. 349), il devrait s'agir de l'isomère 17α OH.

Spectre IR (chloroforme)

| | |
|---|---|
| OH | : $3601 cm^{-1}$ |
| C≡C de C≡C-Cl | : $2215 cm^{-1}$ |
| ≡C-H | : $3302 cm^{-1}$ |
| C≡C de C≡C-H | : $2100 cm^{-1}$ |
| C=O conjugué | $1653 cm^{-1}$ |
| C=C | : $1621 cm^{-1}$ |
| aromatique | $1606 cm^{-1}$ |
| | $1504 cm^{-1}$ |
| | $1557 cm^{-1}$ |

Spectre UV (éthanol)

max. 241 nm $E^1_1$ = 552 ε = 23800 ;
max. 250 nm $E^1_1$ = 526 ε = 22700 ;
Infl. 277 nm $E^1_1$ = 203 ;
Infl. 282 nm $E^1_1$ = 258 ;
Infl. 285 nm $E^1_1$ = 297 ;
max. 305 nm $E^1_1$ = 466 ε = 20100.

Spectre RMN (deutérochloroforme)

| | |
|---|---|
| pic à 1,12 ppm | : hydrogènes de 18-Me |
| pic à 3,07 ppm | : hydrogènes du H-C≡C- |
| pic de 3,8 à 4,01 ppm | : hydrogènes en 11 |
| pic à 5,75 ppm | : hydrogènes en 4 |
| pics à 7,11-7,21 ppm<br>pics à 7,43-7,53 ppm | hydrogènes du noyau aromatique. |

Exemple 7 : 17α-chloroéthynyl 17β-hydroxy 11β-/4-/(triméthylsilyl) éthynyl/ phényl/ estra-4,9-dièn-3-one.

On dissout 100 mg de 3,3-éthylènedioxy 17α-chloroéthynyl 11β-/4-(triméthylsilyl) éthynyl/ phényl/ estr-9-èn 5α,17β -diol dans 3 cm3 de méthanol, ajoute 0,5 cm3 de solution aqueuse N d'acide chlorhydrique, laisse reposer à 20°C pendant 5 heures. On concentre à sec par distillition sous pression réduite, ajoute du chloroforme, agite, lave à l'eau puis par une solution aqueuse M de bicarbonate de potassium, sèche, filtre, concentre le filtrat à seo sous pression réduite, chromatographie le résidu sur silice en éluant par un mélange chlorure de méthylène-acétate d'éthyle (95-5) et obtient 53 mg de produit attendu.

Spectre IR (chloroforme)

Présence de 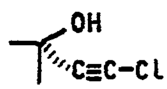 OH : $3600^{cm^{-1}}$

C≡C : $2212^{cm^{-1}}$

dioxane 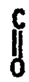 $1657^{cm^{-1}}$

C=C $1601^{cm^{-1}}$

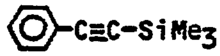 C≡C : $2156^{cm^{-1}}$

aromatique : $1503^{cm^{-1}}$

$SiMe_3$ : $1251^{cm^{-1}}$

$864^{cm^{-1}}$

$844^{cm^{-1}}$

Spectre UV (éthanol)

infl. 235 nm $E^1_1$ = 286 ;

infl. 245 nm $E^1_1$ = 474 ;

max. 255 nm $E^1_1$ = 627 $\varepsilon$ = 31500 ;

max. 264 nm $E^1_1$ = 580 $\varepsilon$ = 29200 ;

Infl. 280 nm $E^1_1$ = 303 ;

max. 300 nm $E^1_1$ = 427 $\varepsilon$ = 21500.

Spectre RMN (deutérochloroforme)

pic à 0,23 ppm : hydrogènes du $Me_3Si$

pic à 0,48 ppm : hydrogènes de 18-Me

pic à 4,44 ppm : hydrogènes en 11

pic à 5,83 ppm : hydrogènes en 4

pics à 7,1 -7,2 ppm
pics à 7,39-7,49 ppm } hydrogènes du noyau aromatique.

Exemple 8 : 17α-chloroéthynyl 9α, 10α-époxy 11β-(4-éthynylphényl) 17β-hydroxy estr-4-èn-3-one.

A une solution de 215 mg de 17α-chloroéthynyl) 11β-(4-éthynylphényl) 17β-hydroxy estra-4,9-dièn-3-one dans le chlorure de méthylène, on ajoute en plusieurs fractions 120 mg d'acide métachloroperbenzoïque, agite pendant 1 heure à 20°C, lave par une solution M de bicarbonate de potassium, sèche, filtre, concentre à sec par distillation sous pression réduite, chromatographie le résidu sur silice en éluant par un mélange chlorure de méthylène-acétate d'éthyle (95-5) et obtient 165 mg de produit recherché.

Spectre IR (chloroforme)

| | | |
|---|---|---|
| OH | | : 3600 $cm^{-1}$ |
| C≡C-Cl | C≡C | : 2222 $cm^{-1}$ |
| -C≡C-H | C≡CH | : 3303 $cm^{-1}$ |
| | C≡C | : 2120 $cm^{-1}$ |
| C=O | | : 1608 $cm^{-1}$ |
| $\Delta_4$ C=C | | : 1623 $cm^{-1}$ |
| aromatiques | | 1608 $cm^{-1}$ |
| | | 1557 $cm^{-1}$ |
| | | 1505 $cm^{-1}$ |

bande forte à 908 $cm^{-1}$ typique de 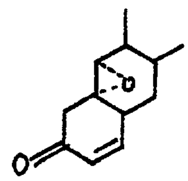

Spectre UV (éthanol)

infl. 240 nm $E^1_1$ = 670 ;
max. 244 nm $E^1_1$ = 733 $\varepsilon$ = 32800 ;
max. 254 nm $E^1_1$ = 636 $\varepsilon$ = 28400 ;
infl. 272 nm $E^1_1$ = 42 ;
infl. 280 nm $E^1_1$ = 16.

Spectre RMN (deutérochloroforme)

| | |
|---|---|
| pic à 0,46 ppm | : hydrogènes de 18-Me |
| pic à 3,08 ppm | : hydrogènes de H-C≡C- |
| pics à 3,23-3,30 ppm | : hydrogènes en 11 |
| pic à 6,15 ppm | : hydrogènes en 4 |
| pics à 7,19-7,29 ppm | hydrogènes du noyau aromatique. |
| pics à 7,45-7,55 ppm | |

Exemple 9 : 17-acétate de 11β-(4-éthynylphényl) 17α-(2-propényl) estra 1,3,5(10)-trièn-3,17β -diol et 11β-(4-éthynylphényl) 17α-(2-propényl) estra-1,3,5(10)-trièn-3,17β-diol.

A une solution de 500 mg de 11β-(4-éthynylphényl) 17α-allyl 17β-hydroxy estra-4,9-dièn-3-one dans 3 cm3 de chlorure de méthylène, on ajoute à 0°C, 0,45 cm3 d'anhydride acétique, 0,25 cm3 de bromure d'acétyle, agite pendant 1 heure à 0°C, verse le mélange réactionnel dans 5 cm3 de solution aqueuse M de bicarbonate de potassium, extrait au chlorure de méthylène, sèche et concentre le filtrat à sec par distillation sous pression réduite, on dissout le résidu dans 20 cm3 de méthanol, dégaze par barbotage d'azote et ajoute 2 cm3 de lessive de soude à 32%, agite pendant 2 heures à 20°C sous atmosphère

inerte, verse le mélange réactionnel dans une solution aqueuse saturée de chlorure de sodium additionnée de 15 cm3 de solution aqueuse 2N d'acide chlorhydrique, extrait au chloroforme, sèche, filtre, concentre le filtre à sec par distillation sous pression réduite, chromatographie le résidu sur silice en éluant par un mélange chlorure de méthylène-acétate d'éthyle (95-5) et obtient 231 mg du 17-acétate de 11β-(4-éthynylphényl) 17α-(2-propényl) estra-1,3,5(10)-trièn-3,17β-diol et 68 mg de 11β-(4-éthynylphényl) 17α-(2-propényl) estra-1,3,5(10)-trièn-3,17β-diol.

Caractéristiques physiques de l'acétate :

<u>Spectre IR</u> (chloroforme)

OH phénolique : $3599^{cm-1}$
C≡C-H : $3302^{cm-1}$
C≡C conjugué : $2100^{cm-1}$

$\underset{\underset{O}{\|}}{C}$ acétate : $1724^{cm-1}$

Me : $1368^{cm-1}$
C-O-C : $1245^{cm-1}$

Allyl =C-H : $3080^{cm-1}$
C=C : $1640^{cm-1}$
déf. | $998^{cm-1}$
| $920^{cm-1}$.

<u>Spectre UV</u> (éthanol)

infl. 240 nm $E^1_1$ = 401 ;
max. 245 nm $E^1_1$ = 455 ε = 20700 ;
max. 256 nm $E^1_1$ = 409 ε = 18600 ;
max. 277 nm $E^1_1$ = 66 ε = 3000 ;
max. 283 nm $E^1_1$ = 68 ε = 3100 ;
max. 288 nm $E^1_1$ = 60 ε = 2700 ;
infl. 309 nm $E^1_1$ = 15 ;
infl. 323 nm $E^1_1$ = 12.

<u>Spectre RMN</u> (deutérochloroforme)

pic à 0,36 ppm : hydrogènes de 18-Me
pic à 1,92 ppm : hydrogènes de $O-\overset{\overset{O}{\|}}{C}=CH_3$
pic à 2,97 ppm : hydrogènes de H-C≡C-.
pic à 4,03 ppm : hydrogènes en 11

pics de 5,0 à 5,12 ppm : hydrogènes de 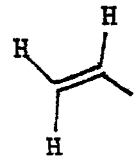

pics de 5,55 à 6,11 ppm : hydrogènes de 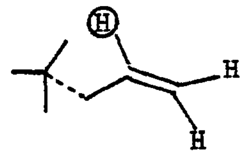

pics à 6,37-6,49 ppm : hydrogènes en 2
pics à 6,63-6,65 ppm : hydrogènes en 4
pics à 6,77-6,87 ppm : hydrogènes en 1
pics à 7,04-7,13 ppm } hydrogènes du noyau aromatique.
pics à 7,21-7,3 ppm }

**Caractéristiques physiques de l'alcool.**

Spectre IR (nujol)

absorption générale région NH/OH

C≡C-H : 3308 $cm^{-1}$
C≡C : 2100 $cm^{-1}$

Allyl =C-H : 3080 $cm^{-1}$
C=C : 1640 $cm^{-1}$
déf. 990 $cm^{-1}$
910 $cm^{-1}$

aromatiques 1618 $cm^{-1}$
1605 $cm^{-1}$
1587 $cm^{-1}$
1500 $cm^{-1}$.

Spectre de Masse : en accord.

Exemple 10 : 17α-acétyloxy 11β-/4-(1-propynyl) phényl/ 19-nor pregna-4,9-dièn-3,20-dione.

Stade A : 3,3-éthylènedioxy 5α-hydroxy 11β-/4-(1-propynyl) phényl/17α-hydroxy 19-nor-pregna-9-èn-20-one.

a) Préparation du magnésien.

On mélange 3 g de magnésium en tournures, 10 cm3 de tétrahydrofuranne, amorce avec quelques gouttes de la solution de bromure de méthyle, puis ajoute 30 cm3 d'une solution de bromure de méthyle dans le tétrahydrofuranne, titrant 100 mmoles/30 cm3 de manière à maintenir le reflux, agite encore 15 minutes puis laisse au repos pendant 1 heure. Titre 2M/l.

b) Condensation.

On introduit sous atmosphère inerte 2 g de 3,3-éthylènedioxy 5α-hydroxy 11β-/4-(1-propynyl) phényl/ 17α-triméthylsilyloxy 17β-cyano estr-9-ène puis rapidement en une seule fois 25 cm3 de solution de magnésien préalablement obtenue. On chauffe au reflux pendant 24 heures puis encore 16 heures après avoir concentré à environ 15 cm3; après retour à la température ambiante, on obtient une pâte assez épaisse dans laquelle on ajoute avec précaution environ 100 cm3 de glace et de chlorure d'ammonium, extrait à l'acétate d'éthyle, lave à l'eau salée, sèche, filtre, concentre à sec par distillation sous pression réduite, chromatographie le résidu sur silice en éluant par un mélange chlorure de méthylène-acétate d'éthyle (8-2) et obtient 810 mg de produit recherché.

Spectre IR (chloroforme)

| | | |
|---|---|---|
| 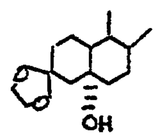 | OH associé : | $3510 cm^{-1}$ |
| | cétal | $1120 cm^{-1}$, $1099 cm^{-1}$, $1074 cm^{-1}$ |
| 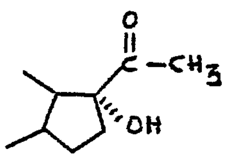 | OH | $3610 cm^{-1}$ |
| | C=O | $1705 cm^{-1}$, $1690 cm^{-1}$ |
| 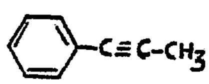 | C≡C : | $2255 cm^{-1}$ |
| | aromatique | $1558 cm^{-1}$, $1508 cm^{-1}$ |

Spectre RMN (deutérochloroforme)

| | |
|---|---|
| pic à 0,33 ppm | : hydrogènes de 18-Me |
| pic à 2,05 ppm | : hydrogènes de $\equiv C-CH_3$ |
| pic à 2,22 ppm | : hydrogènes de $COCH_3$ |
| pic à 4,0 ppm | : cétal |
| pics à 4,3-4,35 ppm | : hydrogènes en 11 |
| pic à 3,35 ppm | : hydrogènes de OH |
| pics à 7,07-7,17 ppm | hydrogènes du noyau aromatique. |
| pics à 7,24-7,33 ppm | |

Stade B : 11β-/4-(1-propynyl) phényl/ 17α-hydroxy 19-nor-pregna-4,9-dièn-3,20-dione (décétalysation, conjugaison).

On introduit sous atmosphère inerte 650 mg de 3,3-éthylènedioxy 5α-hydroxy 11β-/4-(1-propynyl) phényl/ 17α-hydroxy 19-nor-pregn-9-èn-20-one, 10 cm3 d'éthanol 96%, 1 cm3 d'eau, obtient la dissolution en tiédissant, ajoute 1 g de résine Redex CF, chauffe au reflux pendant 1 heure 30 minutes, filtre la résine et concentre le filtrat à sec sous pression réduite, chromatographie le résidu sur silice en éluant par un mélange chlorure de méthylène-acétate d'éthyle (8-2), reprend le résidu à l'éther, amorce la cristallisation, essore et obtient 355 mg de produit recherché. F = 150°C.

Spectre IR (chloroforme)

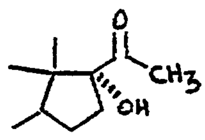

OH + associé 3608 $cm^{-1}$

C=O 1706 $cm^{-1}$

1690 $cm^{-1}$

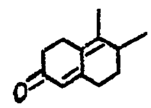

C=O 1655 $cm^{-1}$

C=C 1600 $cm^{-1}$

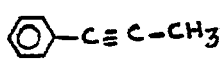

C≡C : 2255 $cm^{-1}$

aromatique 1555 $cm^{-1}$

1505 $cm^{-1}$

Stade C : 11β-/4-(1-propynyl) phényl/ 17α-acétoxy 19-nor-pregna-4,9-dièn-3,20-dione (acétylation).

On introduit sous atmosphère inerte 490 mg de 11β-/4-(1-propynyl) phényl/ 17α-hydroxy 19-nor-pregna-4,9-dièn-3,20-dione, 7 cm3 d'acide acétique, après dissolution on ajoute 2 cm3 d'anhydride trifluoroacétique, 120 mg d'acide paratoluène sulfonique, agite à 20°C pendant 3 heures, verse le mélange réactionnel sur 100 cm3 d'eau glacée, on essore, lave à l'eau, reprend le résidu au chlorure de méthylène, lave par une solution aqueuse M de bicarbonate de potassium, à l'eau salée, sèche, filtre, concentre le filtrat à sec par distillation sous pression réduite, chromatographie le résidu sur silice en éluant par un mélange chlorure de méthylène-acétate d'éthyle (9-1), empâte à l'éther isopropylique et obtient 300 mg de produit recherché. F = 185°C.
$/\alpha/_D$ = +173° (c = 0,5% chloroforme).

Spectre IR (chloroforme)

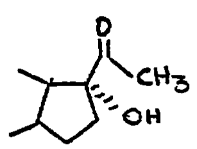

cétone C=O : 3510 $cm^{-1}$

$CH_3$ : 1120 $cm^{-1}$

acétate C=O : 1099 $cm^{-1}$

$CH_3$ : 1074 $cm^{-1}$

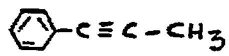

C≡C : 2216 $cm^{-1}$

aromatique 1552 $cm^{-1}$ ; 1506 $cm^{-1}$

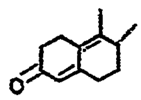

C=O 1644 $cm^{-1}$

C=C 1601 $cm^{-1}$

<u>Spectre UV</u> (éthanol)

max. 246 nm $E^1_1$ = 483 $\varepsilon$ = 22700 ;

max. 255 nm $E^1_1$ = 493 $\varepsilon$ = 23200 ;

max. 300 nm $E^1_1$ = 409 $\varepsilon$ = 19200.

<u>Dichroïsme circulaire</u> (éthanol)

infl. 247 nm $\Delta\varepsilon$ = -21 ;

max. 256 nm $\Delta\varepsilon$ = -25 ;

max. 301 nm $\Delta\varepsilon$ = +21 ;

max. 354 nm $\Delta\varepsilon$ = - 0,68.

<u>Spectre RMN</u> (deutérochloroforme)

pic à 0,31 ppm : hydrogènes de 18-Me

pic à 2,04 ppm : hydrogènes de ≡C-Me

pics à 2,09-2,13 ppm : hydrogènes de COMe

pics à 4,42-4,5 ppm : hydrogènes en 11

pic à 5,83 ppm : hydrogènes en 4

pics à 7,04-7,13 ppm }
pics à 7,27-7,37 ppm } hydrogènes du noyau aromatique.

Exemple 11 : 17α-acétyloxy 11β-4-(éthynylphényl) 19-nor-pregna-4,9-dièn-3,20-dione.

Stade A : 11β-(4-éthynylphényl) 17α-hydroxy 19-nor-pregna-4,9-dièn-3,20-dione.

Ce stade se décompose ainsi :

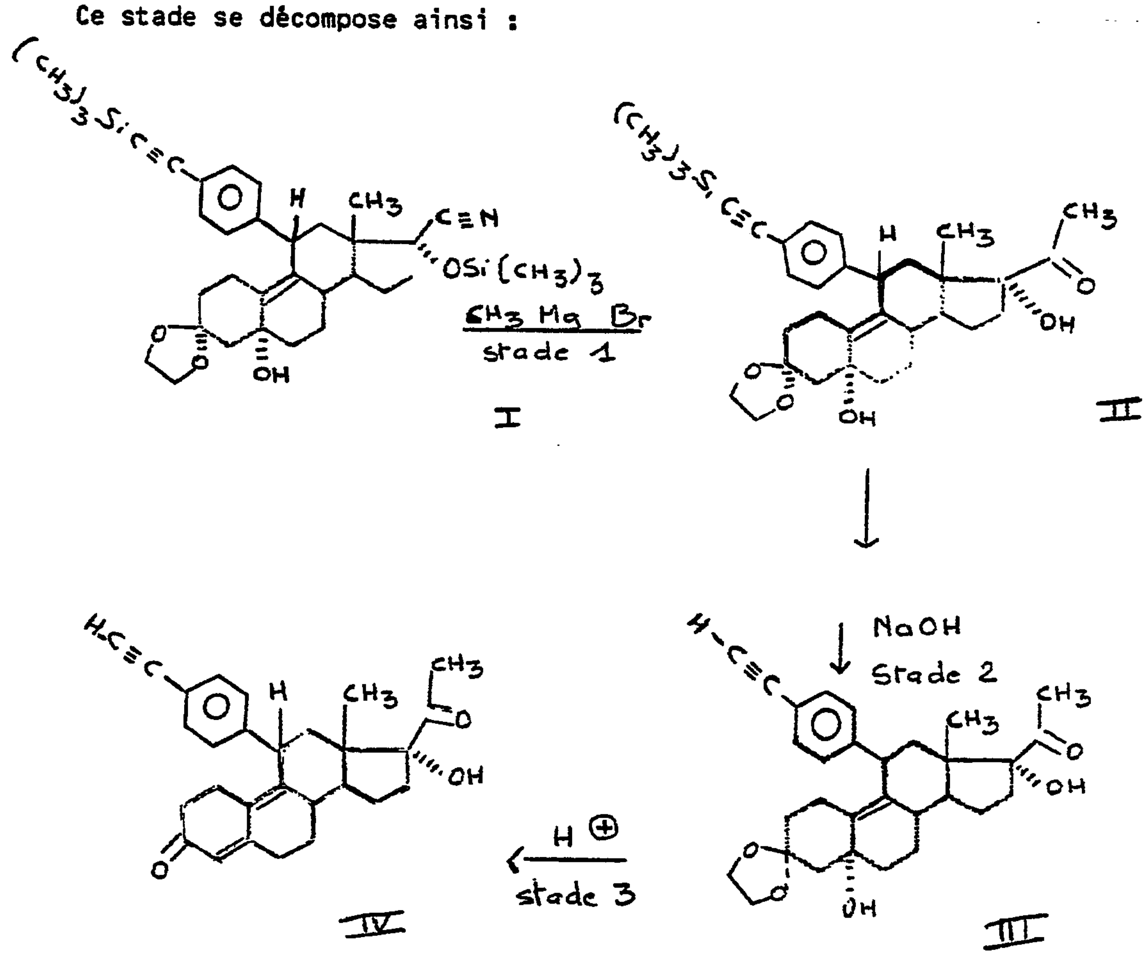

I
II
III
IV

Stade 1 :

On introduit sous atmosphère inerte 1,2 g de magnésium en tournures, ajoute lentement à 30°C 20 cm3 d'une solution 2,1M de bromure de méthyle dans le tétrahydrofuranne, en fin d'introduction, on laisse monter jusqu'à 50°C puis laisse revenir à 35°C et ajoute 1,79 g de 3,3-éthylènedioxy 5α-hydroxy 11β-/4-(triméthylsilyléthynyl) phényl/ 17α-triméthylsilyloxy 17β-cyano estr-9-ène, porte la solution au reflux pendant 24 heures, refroidit, verse le mélange réactionnel sur 100 cm3 de solution aqueuse de chlorure d'ammonium à 10%, extrait à l'acétate d'éthyle, sèche, filtre, concentre le filtrat à sec, reprend le résidu à l'éther isopropylique, triture, essore, lave les cristaux à l'éther isopropylique et obtient ainsi un premier jet de 910 mg de stéroïde II. Un second jet est obtenu par évaporation des liqueurs mères et traitement à l'éther isopropylique (120 mg), soit au total 1,03 g.

Stade 2 :

Sous atmosphère inerte, on place 1,03 g de stéroïde II, 20 cm3 de méthanol, 2 cm3 de solution aqueuse N de soude, porte à 60°C, refroidit à 20°C, ajoute 2 cm3 de solution aqueuse N d'acide chlorhydrique et concentre sous pression réduite à moitié du volume initial, verse le mélange réactionnel dans l'eau, extrait au chlorure de méthylène, lave la phase organique avec une solution aqueuse 1M de bicarbonate de potassium, sèche, filtre, concentre à sec par distillation sous pression réduite et obtient 1 g de produit qui contient déjà un peu de diénone IV.

Stade 3 :

On place sous atmosphère inerte 1 g de stéroïde III, 20 cm3 de méthanol, 2 cm3 d'eau, 1,3 g de résine Redex CF préalablement lavée au méthanol, porte au reflux pendant 1 heure sous agitation, filtre et concentre le filtrat à sec par distillation sous pression réduite, chromatographie le résidu sur silice en éluant avec un mélange chlorure de méthylène-acétone (9-1), concentre la fraction intéressante, on empâte le résidu à l'éther isopropylique et isole finalement 628 mg de stéroïde IV. F = 120-140°C (décomposition).

Spectre IR ($CHCl_3$)

| | |
|---|---|
| OH libre + associé | : 3600$^{cm-1}$ et 3500$^{cm-1}$ |
| H-C≡C | : 3300$^{cm-1}$ |
| -C≡C- | : 2100$^{cm-1}$ |
| C=O | : 1700$^{cm-1}$ épaulement et 1690$^{cm-1}$ |
| C=O conjugué | : 1655$^{cm-1}$ |
| -C=C conjugué | : 1600$^{cm-1}$. |

Spectre RMN (deutérochloroforme)

| | |
|---|---|
| pic à 0,375 ppm | : hydrogènes de 18-Me |
| pic à 2,22 ppm | : hydrogènes du $CH_3$ 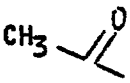 |
| pic à 3,12 ppm | : hydrogènes de 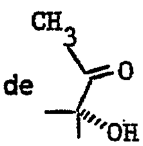 |
| pic à 3,0 ppm | : hydrogènes de H-C≡C- |
| pic à 4,37 ppm | : hydrogènes en 11 |
| pic à 5,75 ppm | : hydrogènes en 4 |
| pics à 7,37-7,05 ppm | : hydrogènes du noyau aromatique. |

Stade B : 17α-acétyloxy 11β-4-(éthynylphényl) 19-nor-pregna-4,9-dièn-3,20-dione.

On place sous atmosphère inerte 500 mg de 11β-(4-éthynylphényl) 17α-hydroxy 19-nor-pregna-4,9-dièn-3,20-dione (stéroïde IV) obtenu ci-dessus, 7 cm3 d'acide acétique, 2 cm3 d'anhydride trifluoroacétique, 120 mg d'acide paratoluène sulfonique, agite pendant 1 heure et 30 minutes à température ambiante puis verse le mélange réactionnel dans 100 cm3 d'eau, le précipité formé est essoré et lavé à l'eau. On redissout le solide dans 50 cm3 de chlorure de méthylène et lave la solution organique par une solution aqueuse M de bicarbonate de potassium, sèche la phase organique, filtre, concentre à sec par distillation sous pression réduite. On chromatographie le résidu sur silice en éluant avec le mélange chlorure de méthylène-acétate d'éthyle (9-1), concentre à sec la fraction intéressante, empâte le résidu à l'éther isopropylique et obtient 295 mg de produit recherché. F > 260°C.
/α/D = +170,5° (c = 0,4% $CHCl_3$).

Spectre IR (chloroforme)

H-C≡C : 3303$^{cm-1}$
-C≡C- : 2108$^{cm-1}$

C=O + C= conjugué : $1658^{cm^{-1}}$ et $1605^{cm^{-1}}$
C=O cétone : $1718^{cm^{-1}}$
C=O acétate : $1733^{cm^{-1}}$
aromatiques : $1605^{cm^{-1}}$, $1558^{cm^{-1}}$, $1503^{cm^{-1}}$, $840^{cm^{-1}}$
méthyle de l'acétate : $1370^{cm^{-1}}$
méthyle de l'acétone : $1355^{cm^{-1}}$.

Spectre RMN (deutérochloroforme)
pic à 0,31 ppm : hydrogènes de 18-Me

pic à 2,10 ppm : hydrogènes du 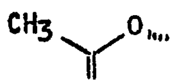

pic à 2,13 ppm : hydrogènes de 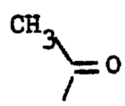

pic à 3,05 ppm : hydrogènes de H-C≡C-
pic à 4,45 ppm : hydrogènes en 11
pic à 5,80 ppm : hydrogènes en 4
pics à 7,11-7,4 ppm : hydrogènes du noyau aromatique.

Spectre UV
max. 243 nm ε = 23400 ;
max. 252 nm ε = 22900 ;
max. 300 nm ε = 21200.

Exemple 12 : 11β-(4-éthynylphényl) 17β-hydroxy 17α-(3-hydroxy 1-propynyl) estra-4,9-dièn-3-one.

Stade A : 3,3-éthylènedioxy 11β-/4-(2-triméthylsilyléthynyl) phényl/17α-/3-tétrahydropyranyloxy 1-propynyl/ 5α,17β-diol estra-9-ène.

On mélange 840 mg de 3-tétrahydropyranyloxy 1-propyne, 25 cm3 d'éther, ajoute à 0°C en 15 minutes environ 3 cm3 de n-butyllithium 1,65M dans l'hexane, agite pendant 15 minutes à 0°C, ajoute rapidement 1,09 g de 3,3-éthylènedioxy 5α-hydroxy 11β-/4-(2-triméthylsilyléthynyl) phényl/ 17-céto estra-9-ène obtenu au stade A de l'exemple 4, en solution dans 20 cm3 de tétrahydrofuranne, agite pendant 30 minutes à 0°C pendant 1 heure à 20°C, verse le mélange réactionnel dans une solution aqueuse saturée de chlorure d'ammonium, extrait à l'éther, sèche, concentre à sec par distillation sous pression réduite, chromatographie le résidu sur silice en éluant avec un mélange de chlorure de méthylène-acétate d'éthyle (75-25) et obtient 1,239 g de produit recherché.

Spectre IR

| | | |
|---|---|---|
| OH | : $3599^{cm-1}$ et $3510^{cm-1}$ |
| C≡C | : $2230^{cm-1}$ et $2155^{cm-1}$ |
| aromatiques | : $1600^{cm-1}$ et $1499^{cm-1}$. |

Spectre RMN ($CDCl_3$)

| | |
|---|---|
| pic à 0,25 ppm | : hydrogènes des méthyles du triméthylsilyl |
| pic à 0,47 ppm | : hydrogènes du 18-Me |
| pic à 3,0 ppm | : hydrogènes des hydroxyles |
| pic de 3,13 à 4,0 ppm | : hydrogènes de 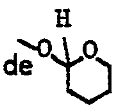 |
| pics à 4,29-4,37 ppm | : hydrogènes en 11 |
| pic à 3,7 ppm | : hydrogènes du méthylène de $-C\equiv CH-CH_2-O-$ |
| pic à 3,97 ppm | : hydrogènes des méthylènes de l'acétal cycli |
| pic à 4,84 ppm | : hydrogène de 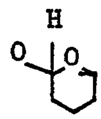 |
| pics à 7,11-7,21 ppm et pics à 7,34-7,44 ppm | hydrogènes de $(CH_3)_3-Si-C\equiv C-$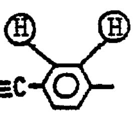 |

**Stade B** : 11β-(4-éthynylphényl) 17β-hydroxy 17α-(3-hydroxy 1-propynyl) estra-4,9-dièn-3-one.

On mélange 500 mg de 3,3-éthylènedioxy 11β-/4-(2-triméthylsilyléthynyl) phényl/ 17α-(3-tétrahydropyranyloxy 1-propynyl) 5α,17β-diol estra-9-ène obtenu au stade A, 5 cm3 de méthanol, ajoute à 20°C rapidement 0,5 cm3 de solution aqueuse 2N de soude, agite pendant 30 minutes à 20°C, ajoute 0,75 cm3 de solution aqueuse 2N d'acide chlorhydrique, agite pendant 5 heures à 20°C, ajoute 0,5 cm3 d'une solution aqueuse saturée de bicarbonate de potassium, ajoute de l'eau, extrait à l'acétate d'éthyle, sèche, concentre à sec par distillation sous pression réduite, chromatographie le résidu sur silice en éluant avec un mélange chlorure de méthylène-acétate d'éthyle (75-25), obtient 285 mg de produit recherché brut que l'on empâte dans 3 cm3 d'éther et obtient 201 mg de produit recherché. F = 120-130°C.

Spectre IR (chloroforme)

| | |
|---|---|
| OH | : $3605^{cm-1}$ |
| C≡C | : $2090^{cm-1}$ |
| ≡C-H | : $3302^{cm-1}$ |
| $\underset{\underset{O}{\|}}{-C-}$ conjugué | : $1657^{cm-1}$ |

C= conjugué : $1602 cm^{-1}$

aromatique : $1552 cm^{-1}$ et $1503 cm^{-1}$.

Spectre UV (éthanol)

max. 242 nm $\varepsilon$ = 25000 ;

max. 251 nm $\varepsilon$ = 23500 ;

max. 300 nm $\varepsilon$ = 21100.

Dichroïsme circulaire (éthanol)

220 nm $\Delta\varepsilon$ = + 3,5 ;

245 nm $\Delta\varepsilon$ = -26 ;

252 nm $\Delta\varepsilon$ = -28 ;

296 nm $\Delta\varepsilon$ = +17 ;

315 nm $\Delta\varepsilon$ = +15

350 nm $\Delta\varepsilon$ = - 0,9.

Analyse : $C_{29}H_{30}O_3$ (426,53)

Calculés : C% 81,66 H% 7,09

Trouvés : 81,3 7,1.

Spectre RMN ($CDCl_3$)

pic à 0,52 ppm : hydrogènes de 18-Me

pic à 3,07 ppm : hydrogènes de l'éthynyle

pic à 3,43 ppm : hydrogènes des hydroxyles

pic à 4,41 ppm : hydrogènes de $-C\equiv C-CH_2-OH$

pic à 4,39 ppm : hydrogènes en 11

pic à 5,81 ppm : hydrogènes en 4

pics à 7,08-7,17 ppm et
pics à 7,39-7,47 ppm } hydrogènes de H-C≡C- 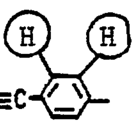

Exemple 13 : (E) 11β-(4-éthynylphényl) 17β hydroxy 17α-(3-hydroxy 1-propényl) estra-4,9-dièn-3-one.

Stade A : 3,3-éthylènedioxy 11β-/4-(2-triméthylsilyléthynyl) phényl/17α-(3-tétrahydropyranyloxy 1-propényl) 5α,17β-diol estra-9-ène.

On mélange 1,4 cm3 d'hydrure de tributyl étain, 0,65 g de 3-tétrahydropyranyloxy 1-propyne, 6 mg d'azoisobutyronitrile, chauffe à 65°C, attend que la réaction démarre, maintient la température à 85-90°C, laisse revenir à 20°C, ajoute 25 cm3 de tétrahydrofuranne, refroidit à -65°C, ajoute en 5 minutes environ 3 cm3 de n-butyllithium 1,65M dans l'hexane, agite pendant 1 heure à -65°C, ajoute 800 mg de 3,3-éthylènedioxy 5α-hydroxy 11β-/4-(2-triméthylsilyléthynyl) phényl/ 17-céto estra-9-ène obtenu au stade A de l'exemple 4, laisse revenir à 20°C, verse le mélange réactionnel sur une solution aqueuse saturée de chlorure d'ammonium, extrait à l'acétate d'éthyle, sèche, concentre à sec par distillation sous pression réduite, chromatographie le résidu sur silice en éluant par un mélange chlorure de méthylène-acétone (90-10) et obtient 289 mg de produit recherché.

<u>Spectre IR</u> (chloroforme)

OH : 3600$^{cm-1}$ et 3510$^{cm-1}$

C≡C : 2155$^{cm-1}$

aromatique : 1602$^{cm-1}$, 1555$^{cm-1}$ et 1500$^{cm-1}$.

<u>Spectre RMN</u> ($CDCl_3$)

pic à 0,24 ppm : hydrogènes des méthyles du triméthylsilyl

pic à 0,53 ppm : hydrogènes de 18-Me

pic à 3,4 ppm : hydrogènes en 11

pic à 4,3 ppm : hydrogènes de 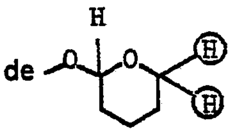

pic à 4,66 ppm : hydrogène de 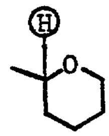

pics à 5,75 et 5,93 ppm : hydrogène éthylénique (J = 15,5 Hz).

<u>Stade B</u> : (E) 11β-(4-éthynylphényl) 17β-hydroxy 17α-(3-hydroxy 1-propényl) estra-4,9-dièn-3-one.

On mélange 270 mg de 3,3-éthylènedioxy 11β-/4-(2-triméthylsilyléthynyl) phényl/ 17α-(3-tétrahydropyranyloxy 1-propényl) 5α,17β-diol estra-9-ène obtenu au stade A, 3 cm3 de méthanol, ajoute à 20°C rapidement 0,25 cm3 de solution aqueuse 2N de soude, agite pendant 30 minutes à 20°C, ajoute 0,35 cm3 de solution aqueuse 2N d'acide chlorhydrique, agite pendant 7 heures à 20°C, ajoute une solution aqueuse de bicarbonate de potassium, ajoute de l'eau, extrait à l'acétate d'éthyle, sèche, concentre à sec par distillation sous pression réduite, chromatographie le résidu sur silice en éluant avec un mélange chlorure de méthylène-acétone (75-25) et obtient 104 mg de produit recherché.
/α/$_D$ = +201° (c = 0,4% chloroforme).

Spectre IR (chloroforme)

OH : $3609^{cm-1}$

≡C-H : $3302^{cm-1}$

C≡C : $2100^{cm-1}$

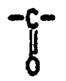 conjugué : $1657^{cm-1}$

C=C conjugué : $1602^{cm-1}$

aromatique : $1555^{cm-1}$ et $1503^{cm-1}$.

Spectre UV (éthanol)

max. 243 nm $\varepsilon$ = 23200 ;

max. 253 nm $\varepsilon$ = 22500 ;

max. 302 nm $\varepsilon$ = 19900.

Dichroïsme circulaire (éthanol)

219 nm $\Delta\varepsilon$ = + 4 ;

245 nm $\Delta\varepsilon$ = -24 ;

252 nm $\Delta\varepsilon$ = -25,7 ;

298 nm $\Delta\varepsilon$ = +16 ;

317 nm $\Delta\varepsilon$ = +12,5 ;

351 nm $\Delta\varepsilon$ = - 0,9.

Spectre RMN ($CDCl_3$)

pic à 0,58 ppm : hydrogènes de 18-Me

pic à 3,07 ppm : hydrogènes de l'éthynyle

pic à 4,19 ppm : hydrogènes de 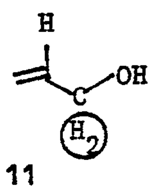

pic à 4,41 ppm : hydrogènes en 11

pics de 5,82 à 5,93 ppm : hydrogènes en 4 et hydrogènes éthyléniques

pics à 7,1-7,2 ppm
pics à 7,4 -7,49 ppm } hydrogènes de 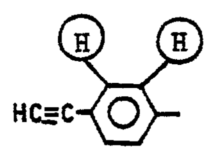

Exemple 14 : γ -lactone de l'acide 11β-(4-éthynylphényl) 17β-hydroxy 3-oxo 19-nor 17α-pregna-4,9-dièn-21-carboxylique.

Stade A : γ -lactone de l'acide 3,3-éthylènedioxy 11β-/4-(2-triméthylsilyléthynyl) phényl/-5α-hydroxy 19-nor 17α-pregna-9-èn-21-carboxylique.

On mélange sous atmosphère inerte 5,5 cm3 de n-butyllithium 1,6M dans l'hexane, refroidit à -70°C,

ajoute en 10 minutes environ à -70°C, 15 cm3 de tétrahydrofuranne, ajoute goutte à goutte à -70°C, 0,86 cm3 de

$$/(CH_3)_2N/_2-\overset{\overset{O}{\uparrow}}{P}-O-CH_2-CH=CH_2$$

en solution dans 5 cm3 de tétrahydrofuranne, agite pendant 30 minutes à -50°C, ajoute en 10 minutes environ 1,08 g de 3,3-éthylènedioxy 5α-hydroxy 11β-/4-(2-triméthylsilyléthynyl) phényl/ 17-céto estr-9-ène obtenu au stade A de l'exemple 4 en solution dans 7 cm3 de tétrahydrofuranne, laisse la température revenir à 20°C en 20 minutes environ, agite pendant 16 heures à 20°C, verse sur une solution aqueuse 1M de chlorure d'ammonium, ajuste le pH à 6 à l'aide de 4 cm3 de solution aqueuse 2N d'acide chlorhydrique, extrait à l'acétate d'éthyle, sèche, concentre à sec par distillation sous pression réduite, chromatographie le résidu sur silice en éluant avec un mélange éther-acétone (9-1) et obtient 185 mg du produit attendu que l'on utilise tel quel pour le stade suivant.

<u>Spectre IR</u> (chloroforme)

| | |
|---|---|
| -C=O | : 1763 $cm^{-1}$ |
| OH | : 3505 $cm^{-1}$ |
| C≡C-Si | : 2156 $cm^{-1}$ |

<u>Spectre RMN</u> ($CDCl_3$)

| | |
|---|---|
| pic à 0,5 ppm | : hydrogènes de 18-Me |
| pic à 0,23 ppm | : hydrogènes des méthyles du triméthylsilyl |
| pic à 3,44 ppm | : hydrogènes de l'hydroxyle en 5 |
| pic à 3,95 ppm | : hydrogènes des méthyles de l'acétal cyclique |
| pics à 4,27-4,36 ppm | : hydrogènes en 11 |

pics à 7,08-7,18 ppm et
pics à 7,37-7,47 } hydrogènes de -C≡C- 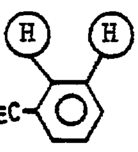

Stade B : γ-lactone de l'acide 11β-/4-(éthynylphényl/ 17β-hydroxy 3-oxo 19-nor-pregna-4,9-dièn-21-carboxylique.

On mélange 170 mg de γ-lactone de l'acide 3,3-éthylènedioxy 11β-/4-(2-triméthylsilyléthynyl) phényl/ 5α-hydroxy 19-nor 17α-pregna-9-èn-21-carboxylique obtenu au stade A, 3 cm3 de méthanol, ajoute à 20°C en 1 minute environ 0,35 cm3 de solution aqueuse 2N de soude, agite pendant 30 minutes à 20°C, ajoute 0,5 cm3 de solution aqueuse 2N d'acide chlorhydrique, agite pendant 2 heures à 20°C, ajoute une solution aqueuse de bicarbonate de potassium, ajoute de l'eau, extrait à l'acétate d'éthyle, sèche, concentre à sec par distillation sous pression réduite, chromatographie le résidu sur silice en éluant avec un mélange chlorure de méthylène-acétone (93-7) et obtient 116 mg de produit recherché.
F = 120-130°C.
/α/D = +97° (c = 0,35% chloroforme).

Spectre IR (chloroforme)

C≡C-H : 3306 $cm^{-1}$

C≡C : 2100 $cm^{-1}$

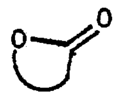 : 1767 $cm^{-1}$

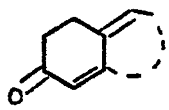 C=O : 1658 $cm^{-1}$

C=C : 1603 $cm^{-1}$

aromatiques : 1557 $cm^{-1}$ et 1500 $cm^{-1}$.

Spectre UV (éthanol)

max. 242 nm $\varepsilon$ = 20400 ;

max. 252 nm $\varepsilon$ = 19500 ;

max. 300 nm $\varepsilon$ = 15600.

Dichroïsme circulaire (éthanol)

219 nm $\Delta\varepsilon$ = + 6 ;

245 nm $\Delta\varepsilon$ = -18,5 ;

252 nm $\Delta\varepsilon$ = -20,2 ;

301 nm $\Delta\varepsilon$ = +17 ;

349 nm $\Delta\varepsilon$ = - 0,7.

Spectre RMN ($CDCl_3$)

pic à 0,6 ppm : hydrogènes de 18-Me

pics à 4,4-4,49 ppm : hydrogènes en 11

pic à 5,84 ppm : hydrogènes en 4

pics à 7,1 -7,19 ppm et
pics à 7,41-7,5 ppm } hydrogènes de H-C≡C- 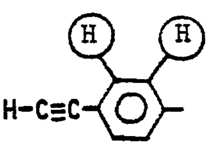

En utilisant les procédés décrits ci-dessus dans la description et les exemples précédents, on a obtenu les produits suivants :

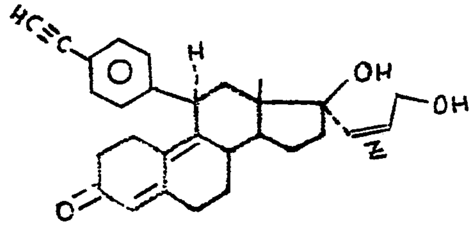

$/\alpha/_D = +254° \pm 3°$ (c = 0,5% $CHCl_3$)

Spectre IR ($CHCl_3$)

| | |
|---|---|
| OH libre | : 3603 $cm^{-1}$ |
| associé | : 3410 $cm^{-1}$ |
| -C≡C-H | : 3302 $cm^{-1}$ |
| C=O conjugué | : 1657 $cm^{-1}$ |
| C=C conjugué | : 1602 $cm^{-1}$ |
| aromatiques | : 1605 $cm^{-1}$ et 1504 $cm^{-1}$. |

Spectre RMN ($CDCl_3$)

| | |
|---|---|
| pic à 0,57 ppm | : hydrogènes de 18-Me |
| pic à 3,06 ppm | : hydrogènes de l'éthynyle |
| pic à 4,37 ppm | : hydrogènes en 11 |
| pic à 5,78 ppm | : hydrogènes en 4 |
| pics à 7,11-7,41 ppm | : hydrogènes aromatiques. |

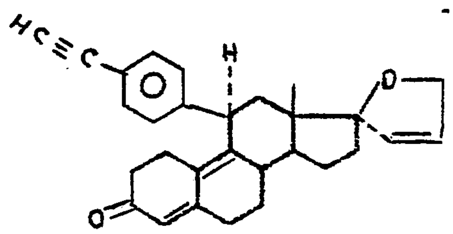

$/\alpha/_D = +252° \pm 3°$ (c = 0,4% $CDCl_3$)

Spectre IR ($CHCl_3$)

pas d'OH

| | |
|---|---|
| -C≡C-H | : 3302 $cm^{-1}$ |
| cétone conjuguée | : 1657 $cm^{-1}$ et 1602 $cm^{-1}$ |
| région C-O-C | : 1081 $cm^{-1}$ et 1040 $cm^{-1}$. |

Spectre RMN ($CDCl_3$)

| | |
|---|---|
| pic à 0,56 ppm | : hydrogènes de 18-Me |
| pic à 3,06 ppm | : hydrogènes de l'éthynyle |
| pic à 4,35 ppm | : hydrogènes en 11 |
| pic à 4,60 ppm | : O-$C\underline{H_2}$- |
| pic à 5,82 ppm | : hydrogènes en 4 |
| pics à 7,16-7,45 ppm | : hydrogènes aromatiques. |

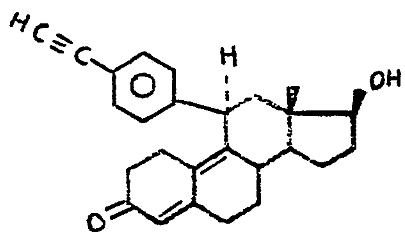

Spectre RMN ($CDCl_3$)

| | |
|---|---|
| pic à 0,416 ppm | : hydrogènes de 18-Me |
| pic à 3,07 ppm | : hydrogènes de l'éthynyle |
| pic à 3,66 ppm | : hydrogènes en 17 |
| pic à 4,39 ppm | : hydrogènes en 11 |
| pic à 5,81 ppm | : hydrogènes en 4 |
| pics à 7,13-7,50 ppm | : hydrogènes aromatiques. |

<u>Spectre UV</u> (EtOH)

max. 244 nm ε = 21500

max. 253 nm ε = 20700

max. 302 nm ε = 19400.

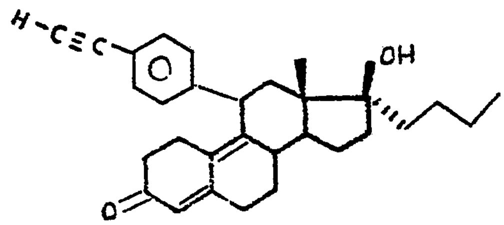

$/\alpha/_D$ = +209,5° ± 3,5° (c = 0,5% $CHCl_3$)

<u>Spectre RMN</u> ($CDCl_3$)

pic à 0,52 ppm : hydrogènes de 18-Me

pic à 0,95 ppm : hydrogènes du $CH_3$ de la chaîne

pic à 3,07 ppm : hydrogènes de l'éthynyle

pic à 4,43 ppm : hydrogènes en 11

pic à 5,81 ppm : hydrogènes en 4

pics à 7,11-7,49 ppm : hydrogènes aromatiques.

Spectre UV (EtOH)

max. 244 nm $\varepsilon = 24300$

max. 253 nm $\varepsilon = 23400$

max. 302 nm $\varepsilon = 20700$.

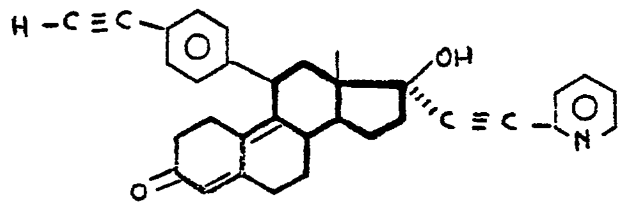

$/\alpha/_D = +25¤ \pm 1{,}5¤$ (c = 0,5% $CHCl_3$)

Spectre RMN ($CDCl_3$)

pic à 0,566 ppm : hydrogènes de 18-Me

pic à 3,07 ppm : hydrogènes de l'éthynyle

pic à 4,45 ppm : hydrogènes en 11

pic à 5,80 ppm : hydrogènes en 4

pic à 8,64 ppm : hydrogènes $H_6$ de la pyrroline

pics à 7,13-7,81 ppm : les autres hydrogènes aromatiques.

Spectre UV (EtOH)

max. 243 nm $\varepsilon = 40600$

infl. 252 nm

max. 280 nm $\varepsilon = 23100$

max. 287 nm $\varepsilon$ = 24300
max. 303 nm $\varepsilon$ = 21300.

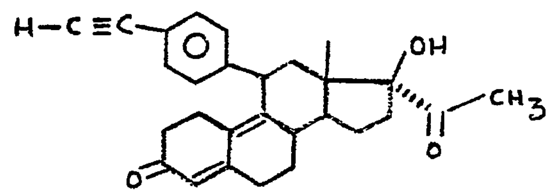

/α/$_D$ = +217° ± 4° (c = 0,6% $CHCl_3$)

Spectre RMN ($CDCl_3$)

pic à 0,588 ppm : hydrogènes de 18-Me
pic à 2,33 ppm : hydrogènes du 20-Me
pic à 3,07 ppm : hydrogènes de l'éthynyle
pic à 4,33 ppm : hydrogènes en 11
pic à 5,81 ppm : hydrogènes en 4
pics à 7,13-7,49 ppm : hydrogènes aromatiques.

Spectre UV (EtOH)

max. 243 nm $\varepsilon$ = 23800
max. 253 nm $\varepsilon$ = 22900
max. 300 nm $\varepsilon$ = 21300.

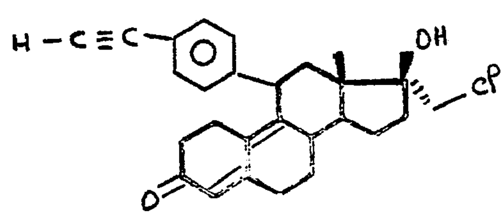

Spectre RMN ($CDCl_3$)

pic à 0,60 ppm : hydrogènes de 18-Me
pic à 3,07 ppm : hydrogènes de l'éthynyle
pics de 3,57 à 3,92 ppm : hydrogènes de $CH_2Cl$
pic à 4,45 ppm : hydrogènes en 11
pic à 5,83 ppm : hydrogènes en 4
pics à 7,12-8,63 ppm : hydrogènes aromatiques.

ETUDE PHARMACOLOGIQUE

Etude de l'activité des produits sur les récepteurs hormonaux.

Récepteur Progestogène de l'utérus de lapine.

Des lapines impubères d'environ 1 kg reçoivent une application cutanée de 25 ug d'estradiol. 5 jours après ce traitement, les animaux sont sacrifiés ; les utérus sont prélevés, pesés et homogénéisés à 0°C à l'aide d'un Potter polytétrafluoroéthylène-verre dans une solution tamponnée TS (Tris 10mM saccharose 0,25 M, HCl pH 7,4) (1 g de tissu pour 50 cm3 de TS).

L'homogénat est ensuite ultracentrifugé (105 000 g x 90 mn à 0°C. Des aliquotes du surnageant ainsi obtenu sont incubés à 0°C pendant un temps (t), avec une concentration constante (T) de Produit R tritié (17,21-diméthyl 19-nor-4,9-pregnadièn-3,20-dione) en présence de concentrations croissantes (0 - 2500. $10^{-9}$M) soit de R froid, soit de progestérone froide, soit du produit froid à tester. La concentration de R tritié lié (B) est ensuite mesurée dans chaque incubat par la technique d'adsorption au charbon dextran.

Récepteur glucocorticoïde du thymus de rat.

Des rats mâles Sprague Dawley EOPS de 160 à 200 g sont surrénalectomisés. 4 à 8 jours après cette ablation, les animaux sont sacrifiés, et les thymus sont prélévés et homogénéisés à 0°C dans un tampon Tris 10 mM, saccharose 0,25 M, dithiothreitol 2mM, HCl pH 7,4, à l'aide d'un Potter polytétrafluoroéthylène-verre (1 g de tissu pour 10 cm3 de TS). L'homogénat est ensuite ultracentrifugé (105 000 x 90 mn) à 0°C. Des aliquotes du surnageant ainsi obtenu sont incubées à 0°C pendant un temps (t) avec une concentration constante (T) de dexaméthasone tritiée en présence de concentrations croissantes (0 - 2500. $10^{-9}$M) soit de dexaméthasone froide, soit du produit froid à tester. La concentration de la dexaméthasone tritiée liée (B) est ensuite mesurée dans chaque incubat par la technique d'adsorption au charbon dextran.

Calcul de l'affinité relative de liaison.

Le calcul de l'affinité relative de liaison (ARL) est identique pour tous les récepteurs.

On trace les 2 courbes suivantes : le pourcentage de l'hormone tritiée liée

$$\frac{B}{T}$$

en fonction du logarithme de la concentration de l'hormone de référence froide et

$$\frac{B}{T}$$

en fonction du logarithme de la concentration du produit froid testé.

On détermine la droite d'équation

$$I_{50} = \frac{B}{T} max + \frac{B}{T} min)/2.$$

$$\frac{B}{T}$$

max = Pourcentage de l'hormone tritiée liée pour une incubation de cette hormone tritiée à la concentration (T).

$$\frac{B}{T}$$

min = Pourcentage de l'hormone tritiée liée pour une incubation de cette hormone tritiée à la concentration (T) en présence d'un grand excès d'hormone froide (2500.$10^{-9}$M).

Les intersections de la droite $I_{50}$ et des courbes permettent d'évaluer les concentrations de l'hormone de référence froide (CH) et du produit froid testé (CX) qui inhibent de 50% la liaison de l'hormone tritiée sur le récepteur.

L'affinité relative de liaison (ARL) du produit testé est déterminée par l'équation

$$ARL = 100 \frac{(CH)}{(CX)}.$$

Les résultats obtenus sont les suivants :

| Produit de l'exemple | Temps d'incubation à 0#C | Progestogène | | Glucocorticoïde | |
|---|---|---|---|---|---|
| | | 2 H | 24 H | 4 H | 24 H |
| Produit de l'exemple 3 | | 44 | 167 | 107 | 124 |
| Produit de l'exemple 4 | | 41 | 124 | 65 | 57 |
| Produit de l'exemple 5 | | 51 | 325 | 84 | 153 |
| Produit de l'exemple 8 | | 38 | 285 | 175 | 233 |

Conclusion :

Les produits étudiés et plus particulièrement les produits des exemples 3, 4, 5 et 8 présentent une affinité très marquée pour les récepteurs glucocorticoïde et progestogène.

Des résultats obtenus, on peut conclure que les produits peuvent présenter un activité agoniste au antagonite des glucocorticoïdes et des progestogènes.

Activité antiglucocorticoïde.

La technique utilisée découle de la méthode décrite par Daune et Coll dans Molecular Pharmacology 13, 948-955 (1977) "The relationship between glucocorticoïd structure and effects upon thymocytes", pour des thymocytes de souris.

Des thymocytes de rats surrenalectomisés sont incubés à 37⌑C pendant 3 heures, dans un milieu nutritif renfermant $5.10^{-8}$M de dexaméthasone, en présence ou non d'un produit à étudier à différentes concentrations. On ajoute l'uridine tritiée, et poursuit l'incubation pendant 1 heure. On refroidit les incubats, les traite avec une solution d'acide trichloroacétique à 5%, les filtre sur papier Whatman GF/A, les lave trois fois à l'aide d'une solution d'acide trichloroacétique à 5%. On détermine la radioactivité retenue par le filtre.

Les glucocorticoïdes et, en particulier la dexaméthasone, provoquent une diminution de l'incorporation d'uridine tritiée. Les produits testés et, plus particulièrement, les produits des exemples 4, 5 et 8 s'opposent à cet effet.

| Produit de l'exemple | $5.10^{-8}$ Dexaméthasone et Produit testé | % d'inhibition de l'effet de la Dexaméthasone |
|---|---|---|
| 4 | $10^{-8}$M | 46 |
| | $10^{-7}$M | 71 |
| | $10^{-6}$M | 99 |
| 5 | $10^{-8}$M | 47 |
| | $10^{-7}$M | 84 |
| | $10^{-6}$M | * |
| 8 | $10^{-8}$M | 7 |
| | $10^{-7}$M | 37 |
| | $10^{-6}$M | 77 |

* A la dose de $10^{-6}$, l'inhibition de l'effet de la dexaméthasone a été totale.

Il a par ailleurs été constaté qu'utilisés seuls, les produits testés ne provoquent aucun effet du type glucocorticoïde.

Conclusion :

Les produis étudiés présentent une activité antiglucocorticoïde très marquée, tout en étant dépourvus d'activité glucocorticoïde.

Activité abortive chez la ratte.

On détermine le Jour J 1 de la gestation par la présence de spermatozoïdes dans les frottis vaginaux. Au Jour J 9 de la gestation, on administre le produit par voie orale en suspension dans la carboxyméthyl cellulose contenant 0,5% de Tween.

Les animaux sont sacrifiés 72 heures après le traitement et l'utérus est examiné pour déterminer l'état de gestation.

On constate un avortement complet sur tous les animaux du groupe avec les produits des exemples 3, 4, 5 et 8 administrés à la dose de 3 mg/kg.

Compositions pharmaceutiques.

On a préparé des comprimés répondant à la formule suivante :

| | |
|---|---|
| **Produit de l'exemple 5** | **50 mg** |
| **Excipient (talc, amidon, stéarate de magnésium)** | **120 mg** |
| **q.s. pour un comprimé terminé à** | |

**Revendications**

1. Les produits de formule I:

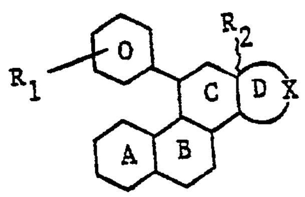

dans laquelle $R_1$ représente un radical alkynyle ayant de 2 à 8 atomes de carbone éventuellement substitué par un radical choisi parmi les radicaux hydroxyle, halogène, trialkylsilyle, alkoxy, alkylthio, dialkylamino et oxo, $R_2$ represente un radical alkyle ayant de 1 à 3 atomes de carbone, les cycles A et B ont l'une des structures suivantes;

a/. soit A et B représentent le groupement:

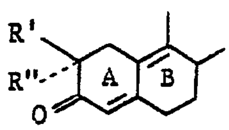

dans lequel R′ et R″ identiques ou différents représentent un atome d'hydrogène ou un radical alkyle ayant de 1 à 4 atomes de carbone;

b/. soit A et B représentent le groupement:

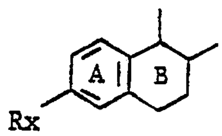

dans lequel Rx représente un atome d'hydrogène ou un groupement ORe dans lequel Re représente un atome d'hydrogène, un radical alkyle ayant de 1 à 6 atomes de carbone éventuellement substitué par un radical dialkylamino ou un radical acétyle ou propionyle;

c/. soit A et B représentent le groupement:

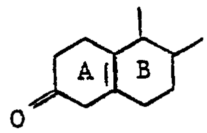

d/. soit A et B représentent le groupement:

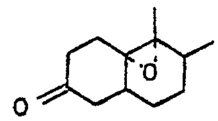

e/. soit A et B représentent le groupement:

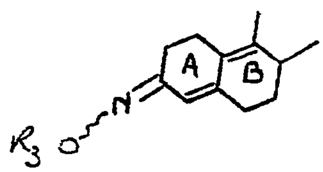

dans lequel $R_3$ représente un atome d'hydrogène un radical alkyle ayant de 1 à 8 atomes de carbone ou un radical aralkyle ayant de 7 à 15 atomes de carbone;
– le groupement de formule:

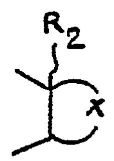

représente:
a/. soit:

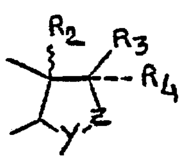

dans lequel $R_3$ et $R_4$ identiques ou différents représentent ou bien un atome d'hydrogène, un radical hydroxyle, un radical

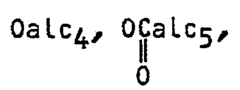

$alc_4$ et $alc_5$ représentant un radical alkyle renfermant de 1 à 8 atomes de carbone ou un radical benzyle; un radical alkyle, alkényle ou alkynyle ayant au plus 8 atomes de carbone ou un radical phényle, benzyle ou phényléthyle ou pyridyléthynyle, thiényle ou furyle, chacun de ces radicaux étant éventuellement substitué par un ou plusieurs radicaux choisis dans le groupe formé par les radicaux hydroxy, alkoxy et alkylthio ayant de 1 à 4 atomes de carbone, les atomes d'halogène et le radical dialkylamino,
ou bien $R_3$ et $R_4$ forment ensemble l'un des radicaux suivants:

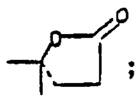 ; 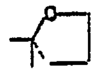 ; 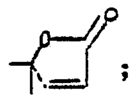 ; 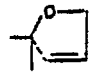 ; 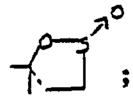 ; 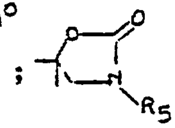 ;

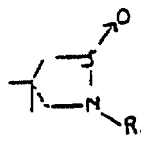 ; 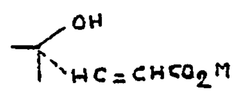 ; 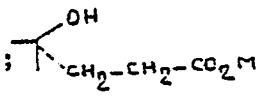 ;

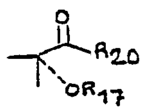 ; 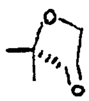 ; 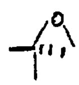 ; 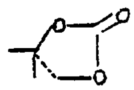 ; 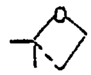

dans lesquels $R_5$ et $R_6$ représentent un atome d'hydrogène ou un radical alkyle ayant de 1 à 4 atomes de carbone, M représente un atome d'hydrogène, de sodium, de potassium ou de lithium, $R_{20}$ représente un radical alkyle éventuellement substitué par hydroxyle et $R_{17}$ représente un atome d'hydrogène ou un radical acétyle, Y et Z représentent les groupements suivants:

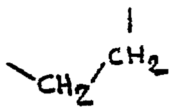 ; 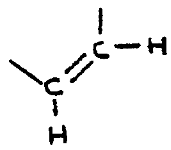 ; 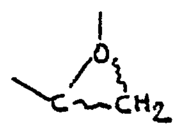 ; 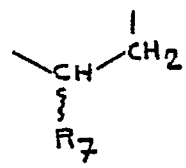 ;

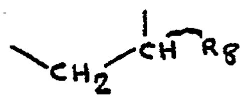

dans lesquels $R_7$ et $R_8$ représentent un radical alkyle ayant de 1 à 4 atomes de carbone;
b/. soit:

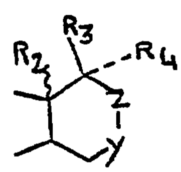

dans lequel $R_3$, $R_4$ et Y–Z ont les définitions indiquées précédemment, les traits ondulés signifient que les substituants $R_2$, $R_7$ et $R_8$ peuvent se trouver dans l'une ou l'autre des configurations possibles, ainsi que les sels d'addition des produits de formule I avec les acides et les bases.

2. Les produits de formule (I'):

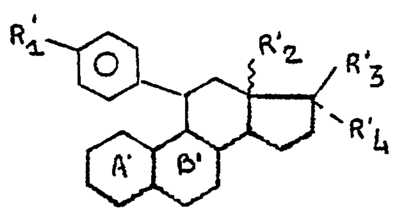

(I')

dans laquelle $R'_1$ représente un radical alkynyle ayant de 2 à 4 atomes de carbone éventuellement substitué par un radical choisi parmi les radicaux hydroxy, halogène ou triméthylsilyle; $R'_2$ représente un radical méthyle ou éthyle, $R'_3$ et $R'_4$ représentent un radical hydroxyle éventuellement acylé, ou un radical alkyle, alkényle ou alkynyle ayant au plus 8 atomes de carbone et éventuellement substitué par un radical choisi parmi les radicaux hydroxyle ou halogène, ou $R'_3$ et $R'_4$ forment ensemble un radical:

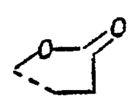 ou 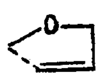 ;

les cycles A′ et B′ ont l'une des structures suivantes:
a/. soit A′ et B′ représentent le groupement:

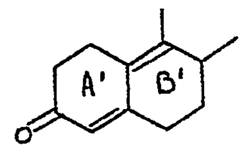

b/. soit A′ et B′ représentent le groupement:

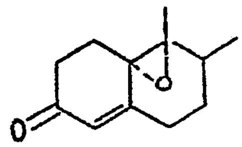

c/. soit A′ et B′ représentent le groupement:

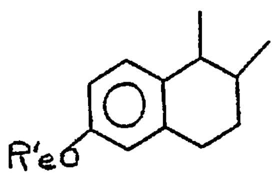

dans lequel R'e représente un atome d'hydrogène ou un radical alkyle ayant de 1 à 4 atomes de carbone.

3. Les produits de formule I′ telle que définie à la revendication 2 dans laquelle $R'_1$ représente un radical $-C{\equiv}C-R_{11}$ dans lequel $R_{11}$ représente un atome d'hydrogène ou un radical méthyle ou triméthylsilyle, $R'_3$ et $R'_4$ sont choisis parmi les radicaux hydroxyle, acétoxy, éthynyle et propynyle éventuellement substitués par un halogène ou par un hydroxyle, propyle et propényle éventuellement substitués par un hydroxy, ou $R'_3$ et $R'_4$ forment ensemble le radical:

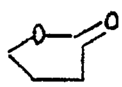 ou 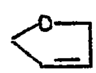

4. Les produits de formule générale (I) telle que définie à la revendication 1 dont les noms suivent:
– La 11β-(4-éthynylphényl) 17β-hydroxy 17α-(1-propynyl) estra 4,9-diène 3-one,
– La 11β-(4-éthynylphényl) 17α-allyl 17β-hydroxy estra 4,9-diène 3-one,
– La 17α(chloroéthynyl) 11β-(4-éthynylphényl) 17β-hydroxy estra 4,9-diène 3-one,
– La 17α(chloroéthynyl) 9α, 10α-époxy 11β-(4-éthynylphényl) 17β-hydroxy est r-4-ène 3-one.

5. Procédé de préparation des produits de formule générale (I) telle que définie à la revendication 1, caractérisé en ce que l'on soumet un produit de formule II:

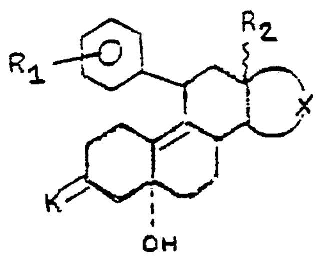

II

dans laquelle K représente une fonction cétone bloquée, $R_1$ et $R_2$ et X ont la signification indiquée à la revendication 1, à l'action d'un réactif de déshydratation susceptible de libérer la fonction cétone pour obtenir un produit de formule $I_A$:

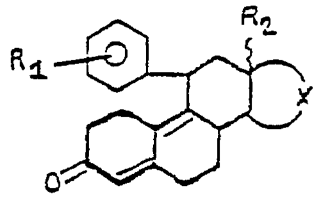

$I_A$

produit de formule $I_A$ que l'on soumet éventuellement:

– a/. soit à l'action d'un agent réducteur, puis à un agent d'aromatisation acide pour obtenir un produit de formule $I_{B1}$ dans laquelle A et B représentent le groupement:

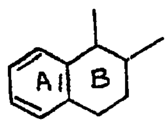

– b/. soit à l'action d'un agent d'aromatisation, puis de saponification puis éventuellement à un réactif d'alkylation ou d'acylation pour obtenir un produit de formule $I_{B2}$ dans laquelle A et B représentent le groupement:

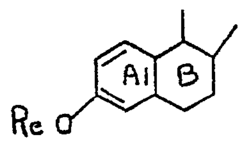

dans laquelle Re a la signification indiquée à la revendication 1:

– c/. soit à l'action d'un agent réducteur pour obtenir un produit de formule $I_C$ dans laquelle A et B représentent le groupement:

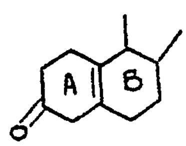

– d/. soit à l'action d'un agent d'oxydation pour obtenir les produits de formule $I_D$ dans laquelle A et B représentent le groupement:

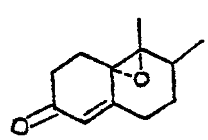

– e/. soit à l'action de l'hydroxylamine $NH_2OH$ libre ou bloquée sous forme $NH_2OR'_3$ dans laquelle $R'_3$ représente un radical alkyle ayant de 1 à 8 atomes de carbone ou un radical aralkyle ayant de 7 à 15 atomes de carbone pour obtenir les produits de formule $I_E$ dans laquelle A et B représentent le groupement:

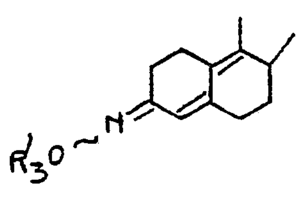

et produits de formule $I_A$, $I_{B1}$, $I_{B2}$, $I_C$, $I_D$ et $I_E$ que l'on soumet éventuellement à l'action d'un acide pour obtenir un sel.

6. Procédé selon la revendication 5 de préparation des produits de formule générale I comportant un radical acyloxy caractérisé en ce que l'on traite un produit de formule générale $I_A$, $I_{B1}$, $I_{B2}$, $I_C$, $I_D$ ou $I_E$ comportant un radical hydroxy libre par un dérivé d'un acide carboxylique.

7. Procédé selon la revendication 5 ou 6, caractérisé en ce que le produit de départ de formule II est préparé:

– soit en soumettant un produit de formule III:

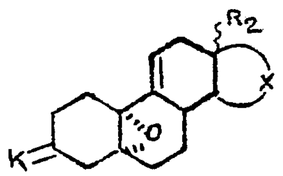

(III)

à l'action d'un produit choisi dans le groupe formé par les produits de formule

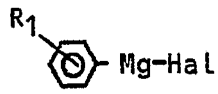

et de formule

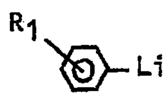

dans lesquelles $R_1$ a la signification indiquée à la revendication 1 et Hal représente un atome d'halogène, le cas échéant en présence d'halogénure cuivreux:
– soit en soumettant un produit de formule IV:

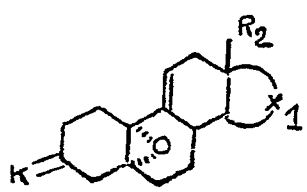

(IV)

dans laquelle le groupement de formule

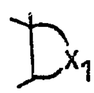

représente:
– soit un cycle

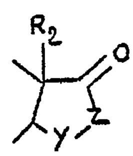

– soit un cycle

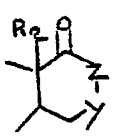

dans lesquels $R_2$, Y et Z ont la signification indiquée à la revendication 1 à l'action d'un produit choisi dans le groupe formé par les produits de formule

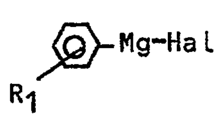

et de formule

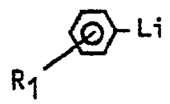

dans lesquelles $R_1$ et Hal ont les significations indiquées précédemment, le cas échéant en présence d'halogénure cuivreux pour obtenir un produit de formule $V_A$:

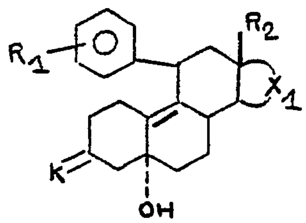

($V_A$)

produit de formule $V_A$ que l'ou soumet éventuellement à l'action d'un rayonnement ultra-violet pour obtenir un produit de formule $V_B$:

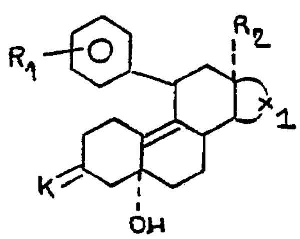

($V_B$)

produits de formules $V_A$ ou $V_B$ que l'on soumet à l'une des réactions suivantes:

a/. <u>ou bien</u> l'on soumet un produit de formule $V_A$ ou $V_B$ à l'action d'un produit de formule $H-C=C-CH_2-O-Gp$ dans lequel Gp représente un groupement protecteur du radical hydroxyle pour obtenir un produit de formule VI:

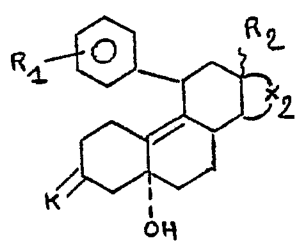

(VI)

dans laquelle

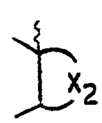

représente:

– soit

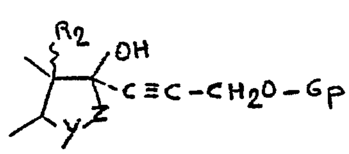

– soit

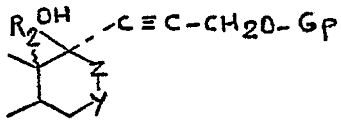

produit de formule VI que l'on soumet à un agent de réduction, puis à un agent de déprotection du radical hydroxyle pour obtenir un produit de formule VII:

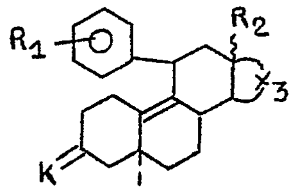

(VII)

dans laquelle

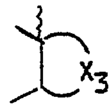

représente:

– soit

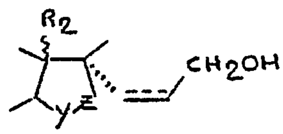

– soit

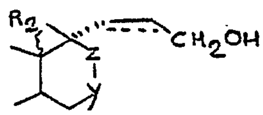

le trait pointillé indiquant la présence éventuelle d'une seconde liaison E ou Z entre les carbones qui le portent et produits de formule VII que l'on soumet:

– soit à un réactif d'oxydation pour obtenir un produit de formule $II_A$:

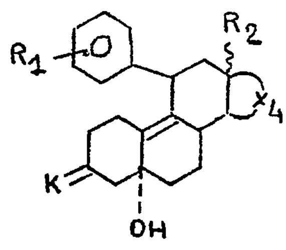

($II_A$)

dans laquelle $X_4$ représente:

– soit

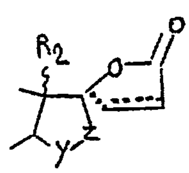

– soit

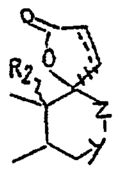

dans lequel le trait pointillé indique la présence éventuelle d'une seconde liaison entre les carbones qui le portent:
– soit l'on soumet le produit de formule VII à un réactif de cyclisation pour obtenir un produit de formule $II_B$:

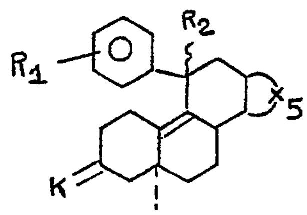

($II_B$)

dans laquelle

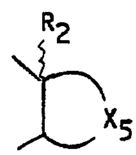

représente:
– soit

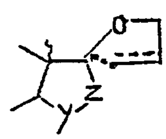

– soit

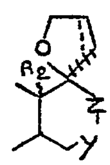

dans lequel le trait pointillé indique la présence éventuelle d'une seconde liaison entre les carbones qui le portent.
b/. ou bien l'on soumet un produit de formule $V_A$ ou $V_B$ d'abord à l'action d'un réactif de formation de l'oxiranne, puis à l'action d'un produit de formule

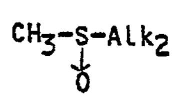

dans laquelle $Alk_2$ représente un radical alkyle ayant de 3 à 5 atomes de carbone et enfin à l'action d'un réactif de cyclisation pour obtenir un produit de formule $II_C$:

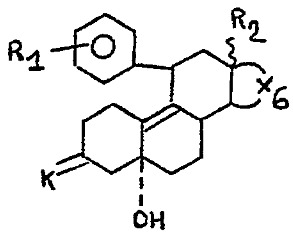

(II$_C$)

dans laquelle:

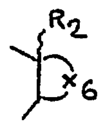

représente:
– soit

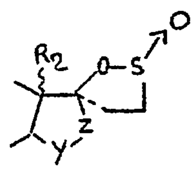

– soit

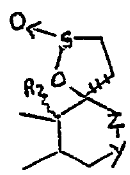

c/. ou bien l'on soumet un produit de formule $V_A$ ou $V_B$ d'abord à l'action d'un réactif de formation de l'oxiranne, puis à l'action d'une alkylamine de formule $H_2NR_5$, $R_5$ ayant la signification indiquée ci-dessus, enfin à l'action d'un dérivé de l'acide carbonique pour obtenir un produit de formule $II_D$:

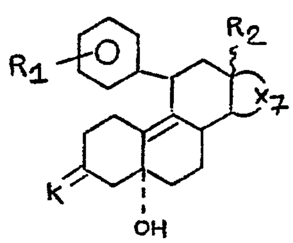

(II$_D$)

dans laquelle:

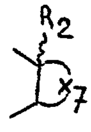

représente:
– soit

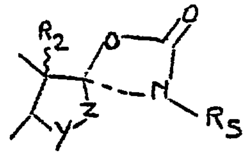

– soit

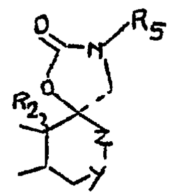

d/. ou bien l'on soumet un produit de formule $V_A$ ou $V_B$ d'abord à l'action d'un réactif de formation de l'oxiranne, puis à l'action d'une alkylamine de formule $H_2NR_5$, enfin à l'action du chlorure de thionyle pour obtenir un produit de formule $II_E$:

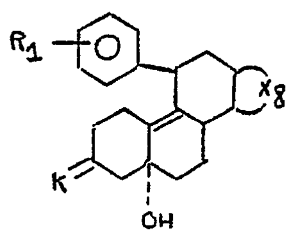

$(II_E)$

dans laquelle:

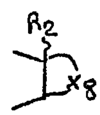

représente:
– soit

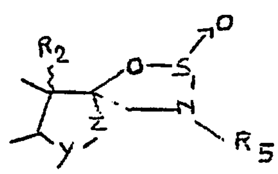

– soit

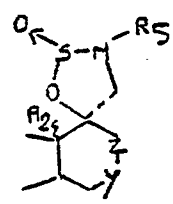

e/. ou bien l'on soumet un produit de formule $V_A$ ou $V_B$ à l'action d'un organomagnésien ou d'un organolithien pour obtenir un produit de formule $II_F$:

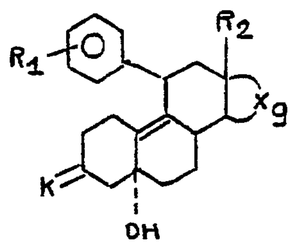

$(II_F)$

dans laquelle:

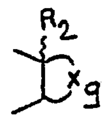

représente:

– soit

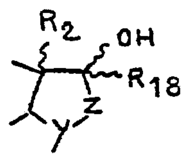

– soit

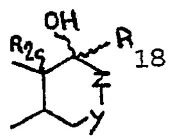

dans lesquels $R_{18}$ représente un radical alkyle, alkényle, alkynyle, ayant au plus 8 atomes de carbone ou un radical aryle, aralkyle, aralkényle ou aralkynyle carbo ou hétérocyclique éventuellement substitués par un ou plusieurs radicaux choisis dans le groupe formé par les radicaux hydroxy, alkoxy ou alkylthio ayant de 1 à 4 atomes de carbone ou halogène,

f/. ou bien l'on soumet un produit de formule $V_A$ ou $V_B$:

– soit à l'action d'un agent de cyanuration, puis à un agent de protection de la fonction hydroxy, puis enfin à l'action d'un magnésien ou d'un lithien,

– soit à l'action d'un lithien de formule

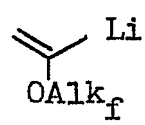

dans laquelle $Alk_f$ représente un radical alkyle ayant de 1 à 4 atomes de carbone, puis à l'action d'une base et d'un acide pour obtenir un produit de formule $II_G$:

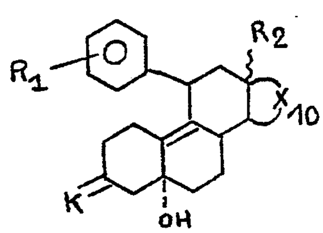

$(II_G)$

dans laquelle

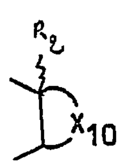

représente:

– soit

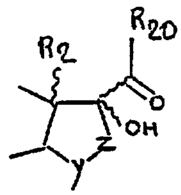

– soit

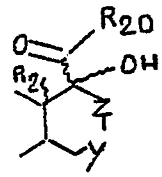

dans laquelle $R_{20}$ représente un radical alkyle éventuellement substitué par hydroxyle produit que l'on soumet éventuellement à l'action d'un réactif d'acylation,

g/. ou bien l'on soumet un produit de formule $V_A$ ou $V_B$ à l'action d'un halogénure de triméthylsulfonium ou du produit de formules $CH_2^{\ominus}S^{\oplus}(CH_3)_2$ en présence d'une base forte pour obtenir un produit de formule $II_H$:

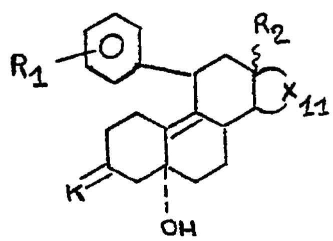

$(II_H)$

dans laquelle

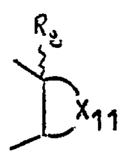

représente:

– soit

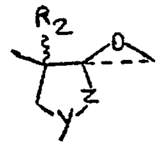

– soit

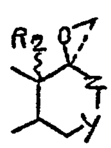

et produit de formule $II_H$ que l'on soumet éventuellement à l'action du produit de formule $CH^{-}$-$s^{+}$-$(CH_3)_2$ pour obtenir un produit de formule $II'_H$:

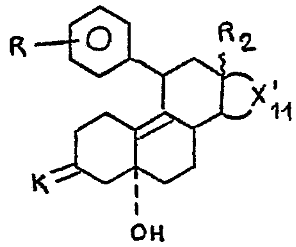

$(II'_H)$

dans laquelle

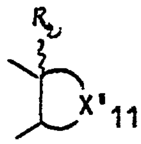

représente:

– soit

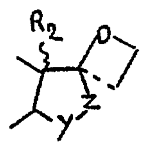

– soit

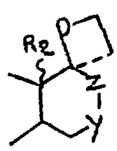

et produit de formule $II_H$ que l'on soumet éventuellement d'abord à une hydrolyse alcaline, puis à l'action d'un produit de formule Hal–$CO_2Alk_g$ dans laquelle Hal représente un atome d'halogène et $Alk_g$ représente un radical alkyle ayant de 1 à 4 atomes de carbone et enfin à l'action d'un carbonate de dialkyle pour obtenir un produit de formule $II''_H$:

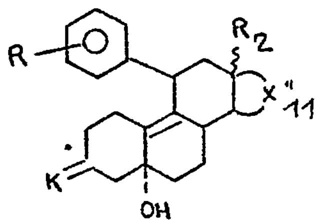

$(II''_H)$

dans laquelle

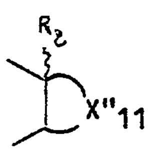

représente:

– soit

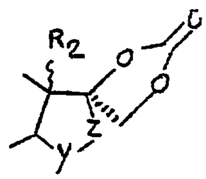

– soit

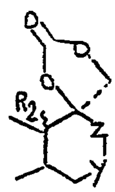

h/. ou bien l'on soumet un produit de formule $V_A$ ou $V_B$ à l'action d'abord d'un lithien de formule

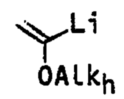

dans lequel $Alk_h$ représente un radical alkyle ayant de 1 à 4 atomes de carbone, puis à l'action d'un réactif d'halogénation, puis enfin à l'action d'une base pour obtenir un produit de formule $II_J$:

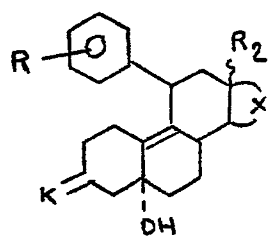

($II_J$)

dans laquelle

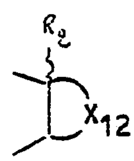

représente:
– soit

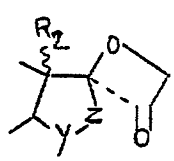

– soit

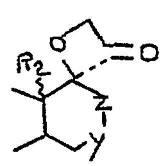

8. A titre de médicaments, les produits de formule générale I telle que définie à la revendication 1 pharmaceutiquement acceptables ainsi que leurs sels d'addition avec les acides et les bases pharmaceutiquement acceptables.

9. A titre de médicaments, les produits de formule générale I' telle que définie à l'une quelcoqnue des revendications 2 ou 3 pharmaceutiquement acceptables ainsi que leurs sels d'addition avec les acides et les bases pharmaceutiquement acceptables.

10. Les compositions pharmaceutiques renfermant, comme principe actif, au moins un médicament défini à l'une des revendications 8 ou 9.

## Claims

1. The products of formula I:

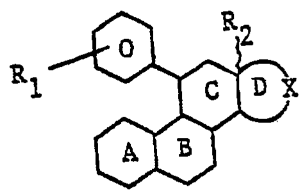

in which $R_1$ represents an alkynyl radical having from 2 to 8 carbon atoms optionally substituted by a radical selected from the radicals, hydroxyl, halogen, trialkylsilyl, alkoxy, alkylthio, dialkylamino and oxo, $R_2$ represents an alkyl radical having from 1 to 3 carbon atoms, the rings A and B have one of the following structures;

a/. either A and B represent the group:

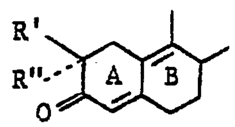

in which R′ and R″ identical or different represent a hydrogen atom or an alkyl radical having from 1 to 4 carbon atoms;

b/. or A and B represent the group:

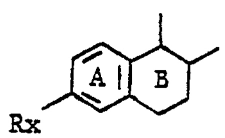

in which Rx represents a hydrogen atom or an ORe group in which Re represents a hydrogen atom, an alkyl radical containing from 1 to 6 carbon atoms optionally substituted by a dialkylamino radical or an acetyl or propionyl radical;

c/. or A and B represent the group:

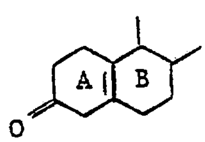

d/. or A and B represent the group:

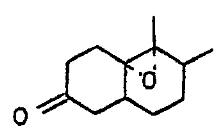

e/. or A and B represent the group:

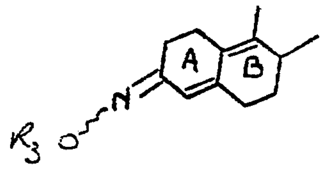

in which $R_3$ represents a hydrogen atom, an alkyl radical having from 1 to 8 carbon atoms or an aralkyl radical having from 7 to 15 carbon atoms;
– the group of formula:

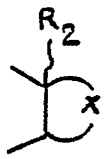

represents:
a/. either:

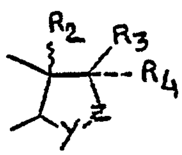

in which $R_3$ and $R_4$, identical or different, represent <u>either</u> a hydrogen atom, a hydroxyl radical, an $Oalk_4$, or

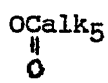

radical, $alk_4$ and $alk_5$ representing an alkyl radical having from 1 to 8 carbon atoms or a benzyl radical; an alkyl, alkenyl or alkynyl radical having at the most 8 carbon carbon atoms or a phenyl, benzyl or phenylethyl or pyridylethynyl, thienyl or furyl radical, each of these radicals being optionally substituted by one or more radicals selected from the group formed by the radicals hydroxy, alkoxy and alkylthio having from 1 to 4 carbon atoms, the halogen atoms and the dialkylamino radical, <u>or</u> $R_3$ and $R_4$ together form the following radicals:

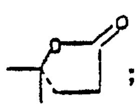 ; 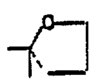 ; 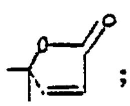 ; 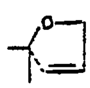 ; 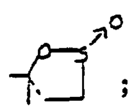 ; 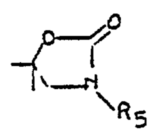 ;

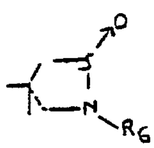 ; 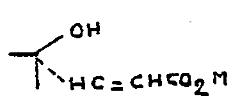 ; 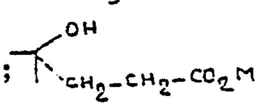 ;

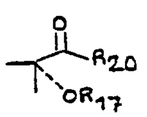 ; 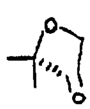 ; 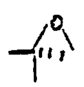 ; 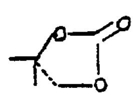 ; 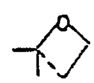

in which $R_5$ and $R_6$ together form a hydrogen atom or an alkyl radical having from 1 to 4 carbon atoms, M represents a hydrogen, sodium or potassium or lithium atom, $R_{20}$ represents an alkyl radical optionally substituted by hydroxyl and $R_{17}$ represents a hydrogen atom or an acetyl radical, Y and Z represent the following groups:

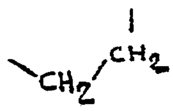 ; 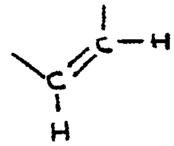 ; 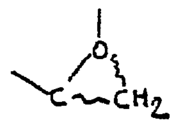 ; 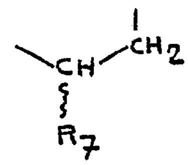 ;

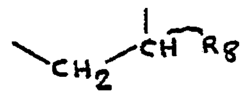

in which $R_7$ and $R_8$ represent an alkyl radical having from 1 to 4 carbon atoms;
b/. or:

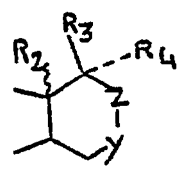

in which $R_3$, $R_4$ and Y–Z have the definitions previously indicated, the wavy lines meaning that the substituents $R_2$, $R_7$ and $R_8$ can be found in one or other of the possible configurations, as well as the addition salts of the products of formula I with acids and bases.

2. The products of formula (I'):

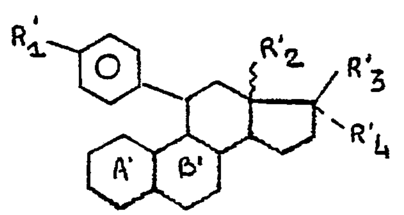

(I')

in which $R'_1$ represents an alkynyl radical having from 2 to 4 carbon atoms optionally substituted by a radical selected from the radicals hydroxy, halogen or trimethylsilyl; $R'_2$ represents a methyl or ethyl radical, $R'_3$ and $R'_4$ represent a hydroxyl radical optionally acylated, or an alkyl, alkenyl or alkynyl radical having at the most 8 carbon atoms and optionally substituted by a radical selected from the hydroxyl or halogen radicals, or $R'_3$ and $R'_4$ together form a radical:

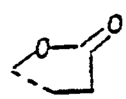 or 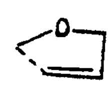

the rings A' and B' have one of the following structures:
a/. either A' and B' represent the group:

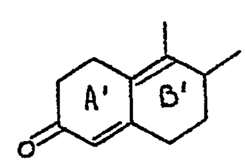

b/. or A' and B' represent the group:

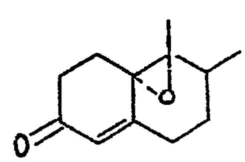

c/. or A' and B' represent the group:

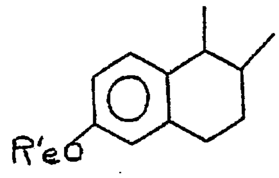

in which R'e represents a hydrogen atom or an alkyl radical having from 1 to 4 carbon atoms.

3. The products of formula I' as defined in claim 2 in which R'1 represents a –C≡C–$R_{11}$ radical in which $R_{11}$ represents a hydrogen atom or a methyl or trimethylsilyl radical, R'3 and R'4 are selected from the radicals hydroxyl, acetoxy, ethynyl and propynyl optionally substituted by a halogen or a hydroxyl, propyl and propenyl optionally substituted by a hydroxy or R'3 and R'4 together form the radical:

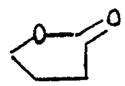 or 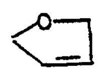

4. The products of the general formula (I) as defined in claim 1 which have the following names:

– 11beta-(4-ethynylphenyl) 17beta-hydroxy 17alpha-(1-propynyl) estra 4,9-dien-3-one,
– 11beta-(4-ethynylphenyl) 17alpha-allyl 17beta-hydroxy estra 4,9-dien-3-one,
– 17alpha(chloroethynyl) 9alpha,10alpha-(ethynylphenyl) 17beta-hydroxy estr-4,9-diene-3-one,
– 17alpha(chloroethynyl) 9alpha,10alpha-epoxy 11beta-(4-ethynylphenyl) 17beta-hydroxy estr-4-ene-3-on.

5. Process for the preparation of the products of the general formula (I) as defined in claim 1, characterized in that a product of formula

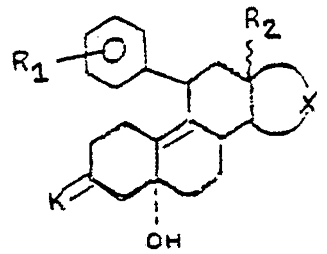

II

in which K represents a blocked ketone function, $R_1$, $R_2$ and X have the meanings already indicated in claim 1, is submitted to the action of a dehydration reagent capable of freeing the ketone function to obtain a product of formula $I_A$:

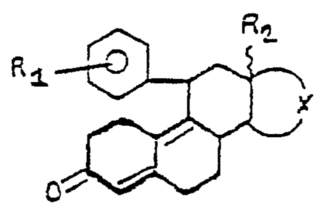

$I_A$

which product of formula $I_A$ is optionally submitted:

– a/. <u>either</u> to the action of a reducing agent, then an acid aromatisation agent to obtain a product of formula $I_{B1}$ in which A and B represent the group:

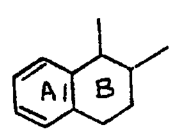

– b/. <u>or</u> to the action of an aromatisation agent, then a saponification agent, then optionally to an alkylation or acylation agent to obtain a product of formula $I_{B2}$ in which A and B represent the group:

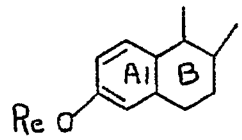

in which Re has the meaning indicated in claim 1.

–c/. <u>or</u> to the action of a reducing agent to obtain a product of formula $I_C$ in which A and B represent the group:

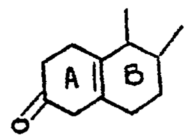

– d/. <u>or</u> to the action of an oxidation agent to obtain the products of formula $I_D$ in which A and B represent the group:

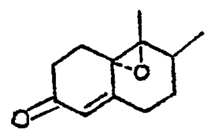

– e/. <u>or</u> to the action of $NH_2OH$ hydroxylamine free or blocked in the form $NH_2OR'_3$ in which $R'_3$ represents an alkyl radical having from 1 to 8 carbon atoms or an aralkyl radical having from 7 to 15 carbon atoms to obtain the products of formula $I_E$ in which A and B represent the group:

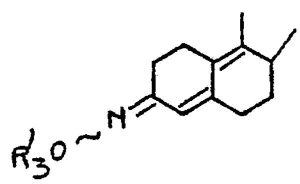

and which products of formulae $I_A$, $I_{B1}$, $I_{B2}$, $I_C$, $I_D$ and $I_E$ are optionally submitted to the action of an acid to obtain a salt.

6. Process according to claim 5 for the preparation of the products of the general formula I comprising an acyloxy radical characterized in that a product of the general formulae $I_A$, $I_{B1}$, $I_{B2}$, $I_C$, $I_D$ or $I_E$ comprising a free hydroxy radical is treated by a derivative of carboxylic acid.

7. Process according to claim 5 or 6, characterized in that the starting product of formula II is prepared:

– <u>either</u> by submitting a product of formula III:

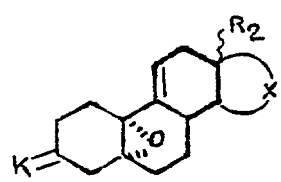

(III)

to the action of a product selected from the group formed by the products of formula

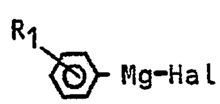

and of formula

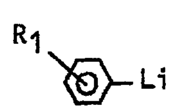

in which $R_1$ has the meaning indicated in claim 1 and Hal represents a halogen atom, if necessary in the presence of a cuprous halogenide;
– <u>or</u> by submitting a product of formula IV:

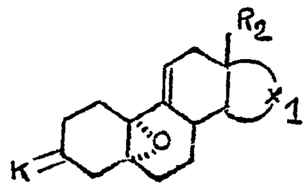

(IV)

in which the group of formula

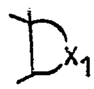

represents:
– or a cycle:

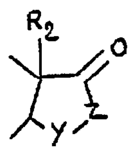

– or a cycle:

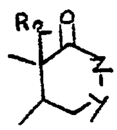

in which $R_2$, Y and Z have the meanings indicated in claim 1, to the action of a product selected from the groups formed by products of formula

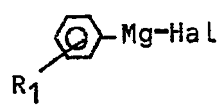

and formula

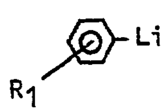

in which $R_1$ and Hal have the meanings indicated previously, if necessary in the presence of a cuprous halogenide to obtain a product of formula $V_A$:

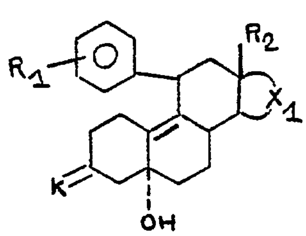

($V_A$)

which product of formula $V_A$ is optionally submitted to the action of ultra-violet radiation to obtain a product of formula $V_B$:

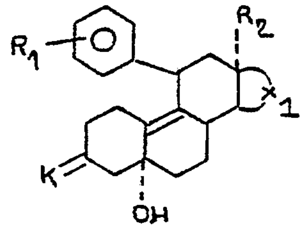

$(V_B)$

which products of formulae $V_A$ and $V_B$ are submitted to one of the following reactions:

a/. <u>either</u> a product of formula $V_A$ or $V_B$ is submitted to the action of a product of formula H–C=C–$CH_2$–O–Gp in which Gp represents a protector group of the hydroxyl radical to obtain a product of formula VI:

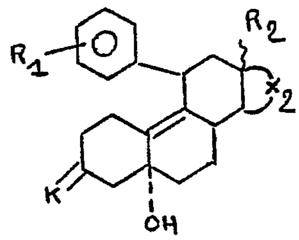

(VI)

in which

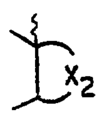

represents:

– either:

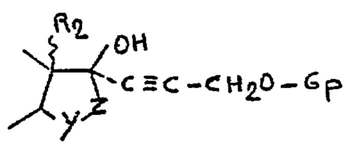

– or:

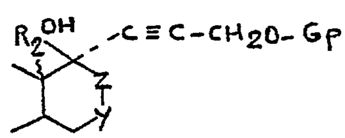

which product of formula VI is submitted to the action of a reduction agent, then to a deprotection agent of the hydroxyl radical to obtain a product of formula VII:

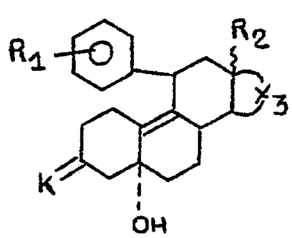

(VII)

in which

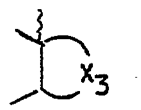

represents:
– either:

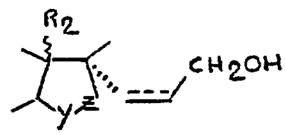

– or:

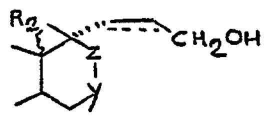

the dotted line indicating the possible presence of a second bond E or Z between the carbons carrying them and which products of formula VII are submitted:
– <u>either</u> to an oxidation reagent to obtain a product of formula $II_A$:

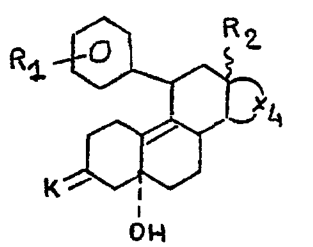

$(II_A)$

in which $X_4$ represents:
– <u>either:</u>

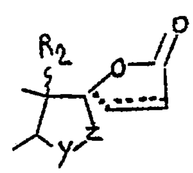

– or

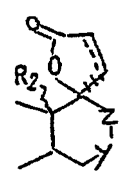

in which the dotted line indicates the possible presence of a second bond between the carbons carrying them;
– <u>or</u> the product of formula VII is submitted to the action of a cyclisation reagent to obtain a product of formula $II_B$:

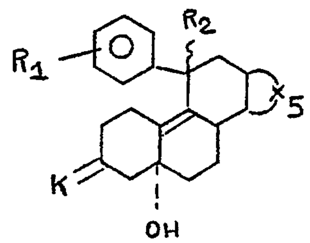

(IIB)

in which

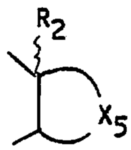

represents:

– either:

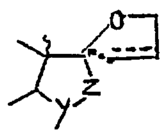

– or:

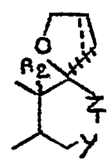

in which the dotted line indicates the possible presence of a second bond between the carbons carrying them.

b/. or a product of formula $V_A$ or $V_B$ is submitted first to the action of an oxiranne formation reagent, then to the action of a product of formula

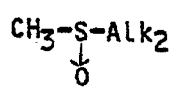

in which $Alk_2$ represents an alkyl radical having from 3 to 5 carbon atoms and finally to the action of a cyclisation reagent to obtain a product of formula $II_C$:

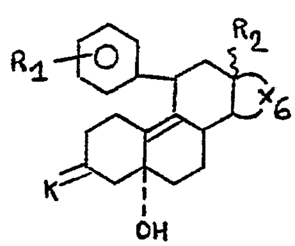

(IIC)

in which:

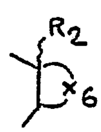

represents:

– either:

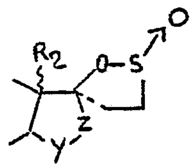

– or:

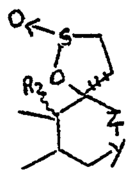

c/. or a product of formula $V_A$ or $V_B$ is submitted first to the action of an oxiranne formation reagent, then to the action of an alkylamine of formula $H_2NR_5$, $R_5$ having the meaning indicated above, then to the action of a carbonic acid derivative to obtain a product of formula $II_D$:

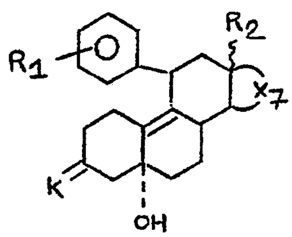

$(II_D)$

in which:

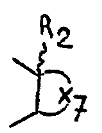

represents:
– either

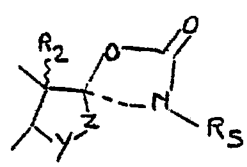

– or

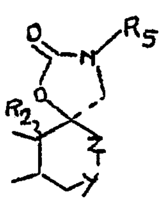

d/. or a product of formula $V_A$ or $V_B$ is first submitted to the action of an oxiranne formation reagent, then to the action of an alkylamine of formula $H_2NR_5$, and finally to the action of thionyl chloride to obtain a product of formula $II_E$:

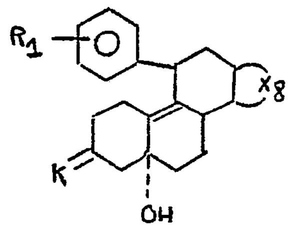

$(II_E)$

in which:

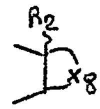

represents:
– either

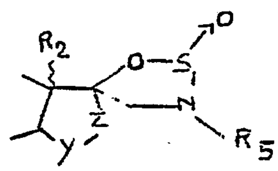

or

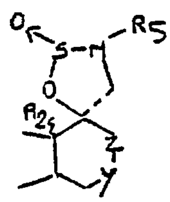

e/. or a product of formula $V_A$ or $V_B$ is submitted to the action of an organomagnesium or an organolithium derivative to obtain a product of formula $II_F$:

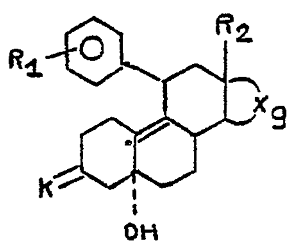

$(II_F)$

in which:

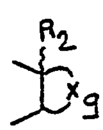

represents:
– either

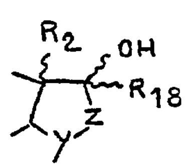

– or

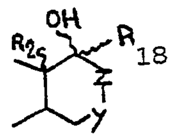

in which $R_{18}$ represents an alkyl, alkenyl, or alkynyl radical having at the most 8 carbon atoms or a carbo- or heterocyclic aryl, aralkyl, aralkenyl or aralkynyl radical optionally substituted by one or more of the radicals selected from the group formed by the radicals hydroxy, alkoxy or alkylthio having from 1 to 4 carbon atoms or halogen.

f/. or a product of formula $V_A$ or $V_B$:

– is either submitted to the action of a cyanuration agent, then to a protection agent of the hydroxyl function, then finally to the action of a magnesium derivative or a lithium derivative,

– or to the action of a lithium derivative of formula

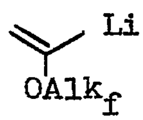

in which $alk_f$ represents an alkyl radical having from 1 to 4 carbon atoms, then to the action of a base and an acid to obtain a product of formula $II_G$:

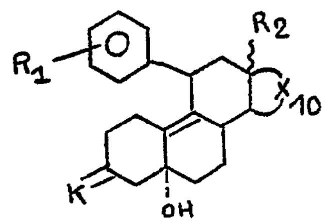

$(II_G)$

in which

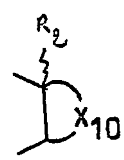

represents:

– either

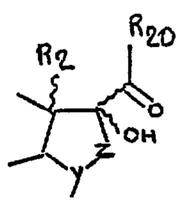

– or

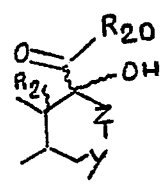

in which $R_{20}$ represents an alkyl radical optionally substituted by hydroxyl which product is optionally submitted to the action of an acylation reagent.

g/. <u>or</u> a product of formula $V_A$ or $V_B$ is submitted to the action of a trimethylsulfonium halogenide or the product of formula $CH_2^{\ominus}S^{\oplus}(CH_3)_2$ in the presence of a strong base to obtain a product of formula $II_H$:

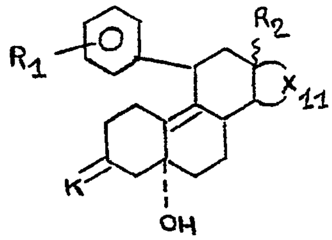

$(II_H)$

in which

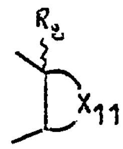

represents:
– either

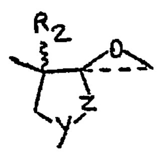

– or

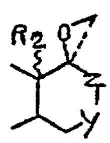

and which product of formula $II_H$ is optionally submitted to the action of the product of formula $CH^{-}$-$s^{+}$-$(CH_3)_2$ to obtain a product of formula $II'_H$:

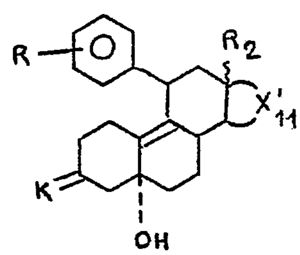

$(II'_H)$

in which

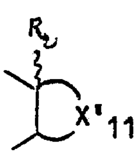

represents:
– either

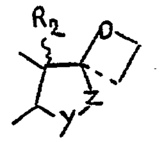

– or

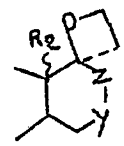

and which product of formula II'$_H$ is optionally submitted first to an alkaline hydrolysis, then to the action of a product of formula Hal–$CO_2Alk_g$ in which Hal represents a halogen atom and $Alk_g$ represents an alkyl radical having from 1 to 4 carbon atoms and finally to the action of a dialkyl carbonate to obtain a product of formula II"$_H$:

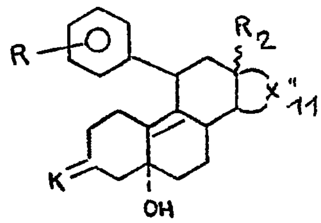

(II"$_H$)

in which

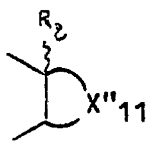

represents:
– either

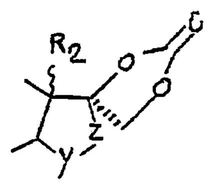

– or

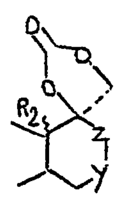

h/. or a product of formula $V_A$ or $V_B$ is submitted to the action first of a lithium derivative of formula

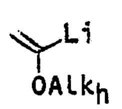

in which $Alk_h$ represents an alkyl radical having from 1 to 4 carbon atoms, then to the action of a halogenation reagent, then finally to the action of a base to obtain a product of formula $II_J$:

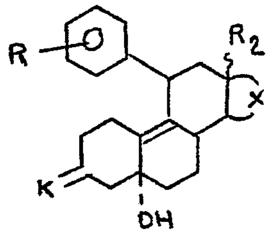

$(II_J)$

in which

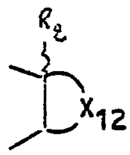

represents:
– either

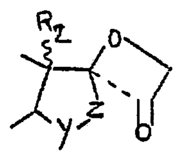

– or

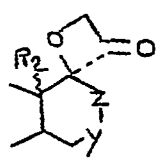

8. As medicaments, the pharmaceutically acceptable products of the general formula I as defined in claim 1 as well as their addition salts with the pharmaceutically acceptable acids and bases.

9. As medicaments, the pharmaceutically acceptable products of the general formula I as defined in either one of claims 2 or 3 as well as their addition salts with the pharmaceutically acceptable acids and bases.

10. The pharmaceutical compositions containing at least one medicament defined in one of the claims 8 or 9 as active principle.

**Patentansprüche**

1. Produkte der Formel I

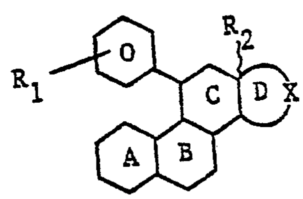

worin $R_1$ für einen Alkinylrest mit 2 bis 8 Kohlenstoffatomen steht, der gegebenenfalls durch einen Rest, ausgewählt unter den Hydroxyl-, Halogen-, Trialkylsilyl-, Alkoxy-, Alkylthio-, Dialkylamino- und Oxoresten, substituiert ist, $R_2$ einen Alkylrest mit 1 bis 3 Kohlenstoffatomen bedeutet, die Ringe A und B eine der folgenden Strukturen besitzen:
(a) entweder A und B für die Gruppe

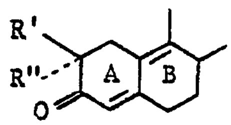

stehen, worin R′ und R″ gleich oder voneinander verschieden sind und ein Wasserstoffatom oder einen Alkylrest mit 1 bis 4 Kohlenstoffatomen wiedergeben;
(b) oder A und B die Gruppe

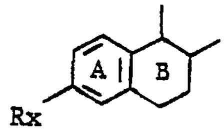

bedeuten, worin Rx für ein Wasserstoffatom oder eine Gruppe ORe steht, in der Re ein Wasserstoffatom, einen Alkylrest mit 1 bis 6 Kohlenstoffatomen, gegebenenfalls substituiert durch einen Dialkylaminorest oder einen Acetyl- oder Propionylrest, bedeutet;
(c) oder A und B die Gruppe

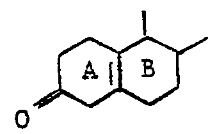

wiedergeben;
(d) oder A und B die Gruppe

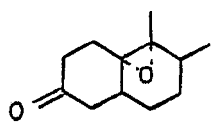

darstellen;
(e) oder A und B die Gruppe

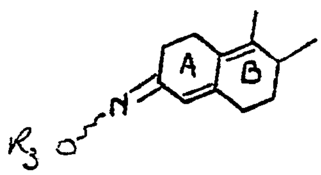

wiedergeben, worin $R_3$ für ein Wasserstoffatom, einen Alkylrest mit 1 bis 8 Kohlenstoffatomen oder einen Aralkylrest mit 7 bis 15 Kohlenstoffatomen steht;
die Gruppe der Formel

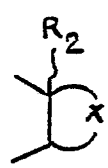

bedeutet:
(a) entweder

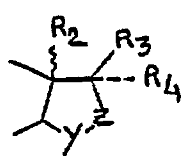

worin $R_3$ und $R_4$, die gleich oder voneinander verschieden sind, entweder ein Wasserstoffatom, einen Hydroxylrest, einen Rest

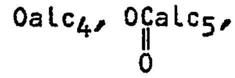

worin $alc_4$ und $alc_5$ für einen Alkylrest mit 1 bis 8 Kohlenstoffatomen oder einen Benzylrest stehen; einen Alkyl-, Alkenyl- oder Alkinylrest mit höchstens 8 Kohlenstoffatomen oder einen Phenyl-, Benzyl-, Phenylethyl- oder Pyridylethinyl-, Thienyl- oder Furylrest wiedergeben, wobei ein jeder dieser Reste gegebenenfalls durch einen oder mehrere Reste, ausgewählt unter den Hydroxy-, Alkoxy- und Alkylthioresten mit 1 bis 4 Kohlenstoffatomen, den Halogenatomen und dem Dialkylaminorest, substituiert ist;
oder $R_3$ und $R_4$ gemeinsam einen der folgenden Reste bilden:

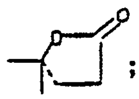 ; 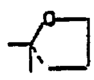 ; 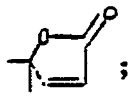 ; 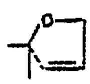 ; 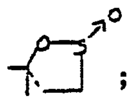 ; 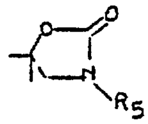 ;

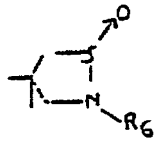 ; 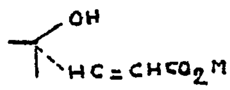 ; 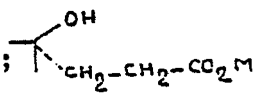 ;

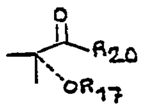 ; 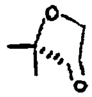 ; 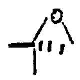 ; 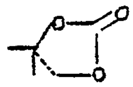 ; 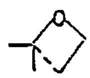

worin $R_5$ und $R_6$ ein Wasserstoffatom oder einen Alkylrest mit 1 bis 4 Kohlenstoffatomen wiedergeben, M für ein Wasserstoff-, Natrium-, Kalium- oder Lithiumatom steht, $R_{20}$ einen Alkylrest, der gegebenenfalls durch Hydroxyl substituiert ist, bedeutet und $R_{17}$ für ein Wasserstoffatom oder einen Acetylrest steht, Y und Z die folgenden Gruppen

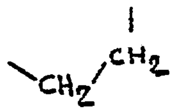 ; 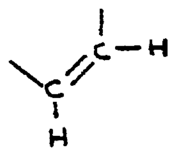 ; 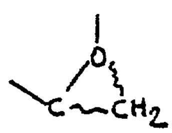 ; 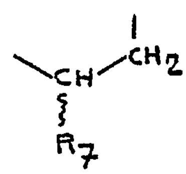 ;

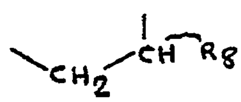

wiedergeben, worin $R_7$ und $R_8$ einen Alkylrest mit 1 bis 4 Kohlenstoffatomen darstellen;
(b) oder

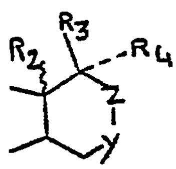

worin $R_3$, $R_4$ und Y–Z die vorstehend angegebenen Bedeutungen besitzen, wobei die gewellten Linien anzeigen, daß die Substituenten $R_2$, $R_7$ und $R_8$ sich in der einen oder anderen der möglichen Konfigurationen befinden können, sowie die Additionssalze der Produkte der Formel I mit Säuren und Basen.

2. Produkte der Formel (I')

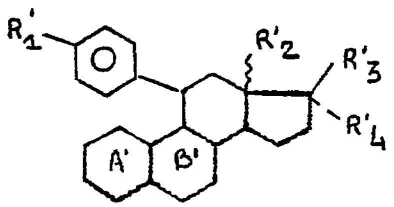

(I')

worin $R'_1$ einen Alkinylrest mit 2 bis 4 Kohlenstoffatomen, der gegebenenfalls durch einen Rest, ausgewählt unter den Hydroxy-, Halogen- oder Trimethylsilylresten, substituiert ist, wiedergibt; $R'_2$ für einen Methyl- oder Ethylrest steht; $R'_3$ und $R'_4$ einen gegebenenfalls acylierten Hydroxylrest oder einen Alkyl-, Alkenyl- oder Alkinylrest mit höchstens 8 Kohlenstoffatomen und gegebenenfalls substituiert durch einen Rest, ausgewählt unter den Hydroxyl- oder Halogenresten, bedeuten oder $R'_3$und $R'_4$ gemeinsam einen Rest:

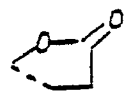 oder 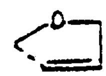

bilden; die Ringe A' und B' eine der folgenden Strukturen aufweisen:
(a) entweder A' und B' die Gruppe:

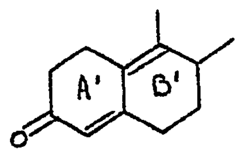

bedeuten,
(b) oder A' und B' die Gruppe:

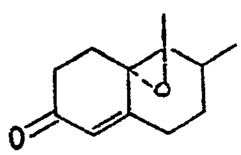

wiedergeben,
(c) oder A' und B' die Gruppe:

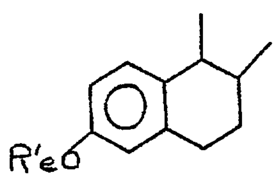

darstellen,
worin R'e ein Wasserstoffatom oder einen Alkylrest mit 1 bis 4 Kohlenstoffatomen bedeutet.

3. Produkte der Formel I' gemäß Anspruch 2, worin $R'_1$ für einen Rest $-C{\equiv}C-R_{11}$ steht, worin $R_{11}$ ein Wasserstoffatom oder einen Methyl- oder Trimethylsilylrest wiedergibt, $R'_3$ und $R'_4$ unter den Hydroxyl-, Acetoxy-, Ethinyl- und Propinylresten, gegebenenfalls substituiert durch ein Halogenatom oder durch eine Hydroxylgruppe, Propyl und Propenyl, gegebenenfalls substituiert durch eine Hydroxygruppe, ausgewählt sind oder $R'_3$ und $R'_4$ gemeinsam den Rest

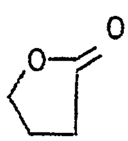 oder 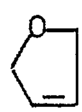

bilden.

4. Produkte der allgemeinen Formel (I) gemäß Anspruch 1 mit den folgenden Bezeichnungen:
11β-(4-Ethinylphenyl)-17β-hydroxy-17α-(1-propinyl)-östra-4,9-dien-3-on,

11β-(4-Ethinylphenyl)-17α-allyl-17β-hydroxy-östra-4,9-dien-3-on,
17α-(Chlorethinyl)-11β-(4-ethinylphenyl)-17β-hydroxy-östra-4,9-dien-3-on,
17α-(Chlorethinyl)-9α,10α-epoxy-11β-(4-ethinylphenyl)-17β-hydroxy-östr-4-en-3-on.

5. Verfahren zur Herstellung der Produkte der allgemeinen Formel (I) gemäß Anspruch 1, dadurch gekennzeichnet, daß man ein Produkt der Formel II

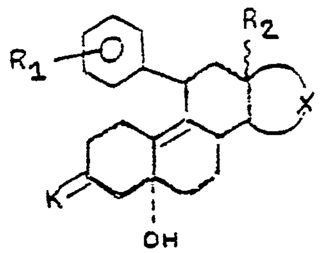

II

worin K eine blockierte Ketonfunktion wiedergibt, $R_1$ und $R_2$ und X die in Anspruch 1 angegebene Bedeutung besitzen, der Einwirkung eines Dehydratationsreagens, das zur Freisetzung der Ketonfunktion befähigt ist, unterzieht, um zu einem Produkt der Formel $I_A$

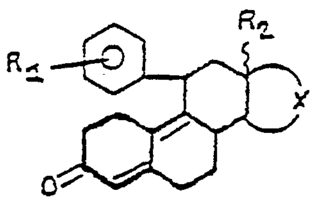

$I_A$

zu gelangen, das Produkt der Formel $I_A$ man gegebenenfalls

(a) <u>entweder</u> der Einwirkung eines Reduktionsmittels, dann eines sauren Aromatisierungsmittels unterzieht, um zu einem Produkt der Formel $I_{B1}$ zu gelangen, worin A und B die Gruppe

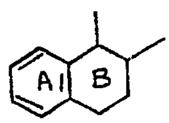

wiedergeben;

(b) <u>oder</u> der Einwirkung eines Aromatisierungsmittels, dann derjenigen eines Verseifungsmittels, hiernach gegebenenfalls eines Alkylierungs- oder Acylierungsmittels unterzieht, um zu einem Produkt der Formel $I_{B2}$ zu gelangen, worin A und B die Gruppe

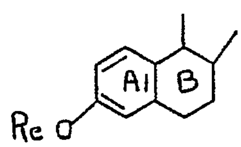

wiedergeben, worin Re die in Anspruch 1 angegebene Bedeutung besitzt;

(c) <u>oder</u> der Einwirkung eines Reduktionsmittels unterzieht, um zu einem Produkt der Formel $I_C$ zu gelangen, worin A und B die Gruppe

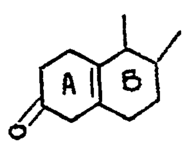

wiedergeben;

(d) <u>oder</u> der Einwirkung eines Oxidationsmittels unterzieht, um zu den Produkten der Formel $I_D$ zu gelangen, worin A und B die Gruppe

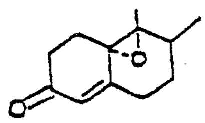

darstellen;

(e) oder der Einwirkung von Hydroxylamin $NH_2OH$ in freier oder in Form von $NH_2OR'_3$ blockierter Form, worin $R'_3$ für einen Alkylrest mit 1 bis 8 Kohlenstoffatomen oder einen Aralkylrest mit 7 bis 15 Kohlenstoffatomen steht, unterzieht, um zu den Produkten der Formel $I_E$ zu gelangen, worin A und B die Gruppe

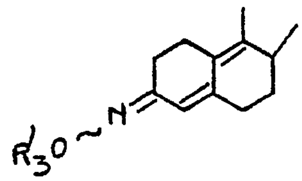

wiedergeben, und die Produkte der Formel $I_A$, $I_{B1}$, $I_{B2}$, $I_C$, $I_D$ und $I_E$ gegebenenfalls der Einwirkung einer Säure unterzieht, um ein Salz zu erhalten.

6. Verfahren gemäß Anspruch 5 zur Herstellung von Produkten der allgemeinen Formel I mit einer Acyloxygruppe, dadurch gekennzeichnet, daß man ein Produkt der allgemeinen Formel $I_A$, $I_{B1}$, $I_{B2}$, $I_C$, $I_D$ oder $I_E$, das eine freie Hydroxylgruppe enthält, mit einem Carbonsäurederivat behandelt.

7. Verfahren gemäß Anspruch 5 oder 6, dadurch gekennzeichnet, daß das Ausgangsprodukt der Formel II hergestellt wird:

entweder indem man ein Produkt der Formel III

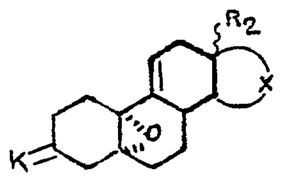

(III)

der Einwirkung eines Produkts, ausgewählt unter den Produkten der Formel

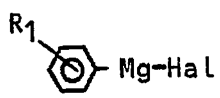

und der Formel

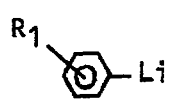

worin $R_1$ die in Anspruch 1 angegebene Bedeutung besitzt und Hal für ein Halogenatom steht, gegebenenfalls in Gegenwart eines Kupfer(I)-halogenids unterzieht,

oder indem man ein Produkt der Formel IV

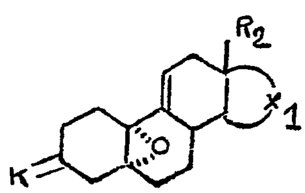

(IV)

worin die Gruppe der Formel

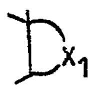

bedeutet:
– entweder einen Ring

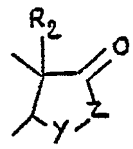

– oder einen Ring

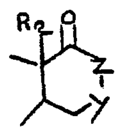

worin $R_2$, Y und Z die in Anspruch 1 angegebene Bedeutung besitzen, der Einwirkung eines Produkts, ausgewählt unter den Produkten der Formel

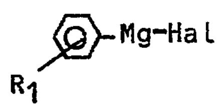

und der Formel

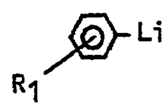

worin $R_1$ und Hal die vorstehend angegebenen Bedeutungen besitzen, gegebenenfalls in Gegenwart eines Kupfer(I)-halogenids unterzieht, um zu einem Produkt der Formel $V_A$

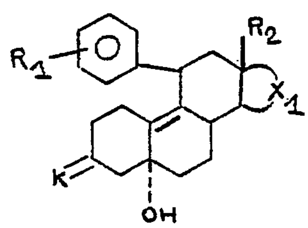

($V_A$)

zu gelangen, das Produkt der Formel $V_A$ gegebenenfalls der Einwirkung von Ultraviolettstrahlen unterzieht, um zu einem Produkt der Formel $V_B$

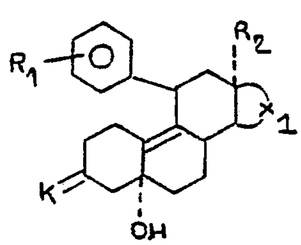

($V_B$)

zu gelangen, die Produkte der Formeln $V_A$ oder $V_B$ einer der folgenden Reaktionen unterzieht:
(a) entweder unterzieht man ein Produkt der Formel $V_A$ oder $V_B$ der Einwirkung eines Produkts der Formel H–C≡C–$CH_2$–O–Gp, worin Gp für eine Schutzgruppe des Hydroxylrestes steht, um zu einem Produkt der Formel VI

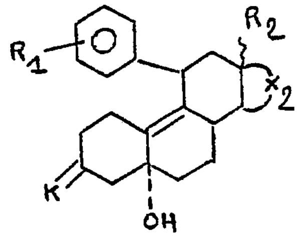

(VI)

zu gelangen,
worin

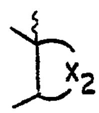

bedeutet:
– entweder

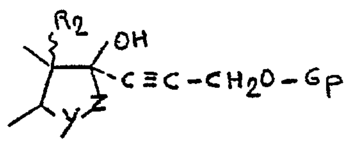

– oder

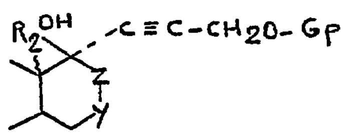

unterzieht man das Produkt der Formel VI der Einwirkung eines Reduktionsmittels, dann derjenigen eines Mittels zur Abspaltung der Schutzgruppe von der Hydroxylgruppe, um zu einem Produkt der Formel VII

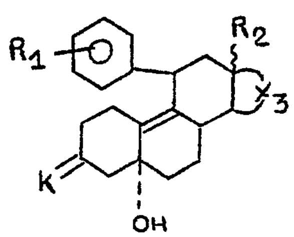

(VII)

zu gelangen,
worin

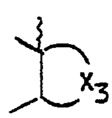

bedeutet:
– entweder

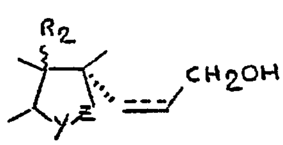

– oder

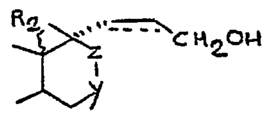

wobei die gestrichelte Linie die etwaige Anwesenheit einer zweiten Bindung E oder Z zwischen den Kohlenstoffatomen, die sie enthalten, anzeigt, und unterzieht die Produkte der Formel VII:
- <u>entweder</u> der Einwirkung eines Oxidationsreagens, um zu einem Produkt der Formel $II_A$

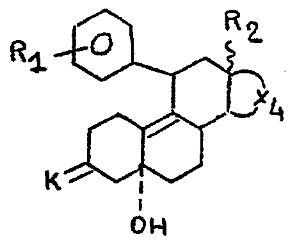

$(II_A)$

zu gelangen,
worin $X_4$ bedeutet:
- <u>entweder</u>

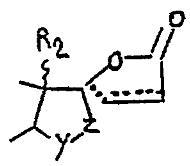

- <u>oder</u>

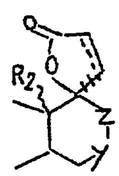

worin die gestrichelte Linie die etwaige Anwesenheit einer zweiten Bindung zwischen den Kohlenstoffatomen, die sie enthalten, anzeigt;
- <u>oder</u> man unterzieht das Produkt der Formel VII der Einwirkung eines Cyclisierungsmittels, um zu einem Produkt der Formel

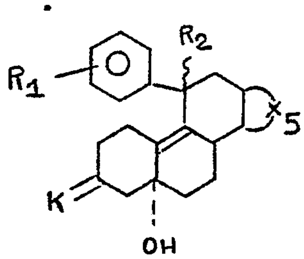

$(II_B)$

zu gelangen,
worin

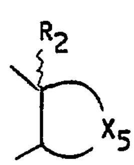

bedeutet:

– <u>entweder</u>

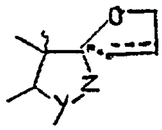

– <u>oder</u>

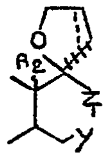

worin die gestrichelte Linie die etwaige Anwesenheit einer zweiten Bindung zwischen den Kohlenstoffatomen, die sie enthalten, anzeigt;
(b) <u>oder</u> man unterzieht ein Produkt der Formel $V_A$ oder $V_B$ zunächst der Einwirkung eines Reagens zur Bildung des Oxirans, danach der Einwirkung eines Produkts der Formel

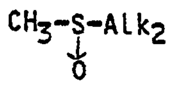

worin $Alk_2$ für einen Alkylrest mit 3 bis 5 Kohlenstoffatomen steht, und schließlich der Einwirkung eines Cyclisierungsreagens, um zu einem Produkt der Formel $II_C$

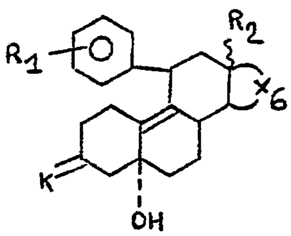

$(II_C)$

zu gelangen,
worin

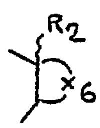

bedeutet:
– entweder

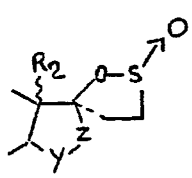

– oder

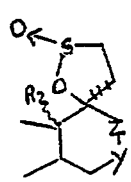

(c) oder man unterzieht ein Produkt der Formel $V_A$ oder $V_B$ zunächst der Einwirkung eines Reagens zur Bildung des Oxirans, dann der Einwirkung eines Alkylamins der Formel $H_2NR_5$, worin $R_5$ die vorstehend angegebene Bedeutung besitzt, schließlich der Einwirkung eines Carbonsäurederivats, um zu einem Produkt der Formel $II_D$

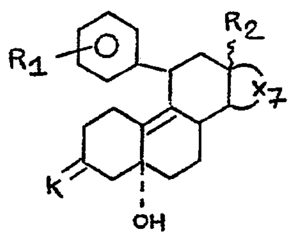

$(II_D)$

zu gelangen,
worin:

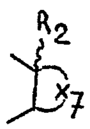

bedeutet:
– entweder

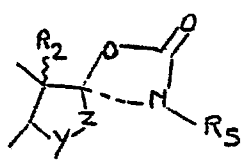

oder

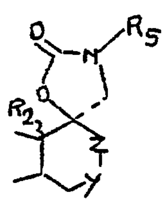

(d) oder man unterzieht ein Produkt der Formel $V_A$ oder $V_B$ zunächst der Einwirkung eines Reagens zur Bildung des Oxirans, dann der Einwirkung eines Alkylamins der Formel $H_2NR_5$, schließlich der Einwirkung von Thionylchlorid, um zu einem Produkt der Formel $II_E$

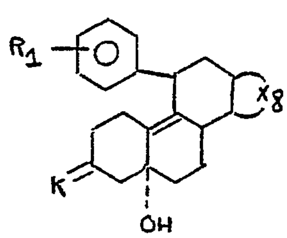

$(II_E)$

zu gelangen,
worin:

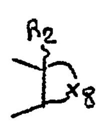

bedeutet:

– entweder

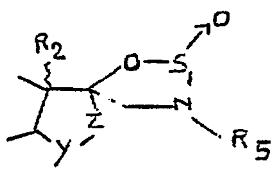

– oder

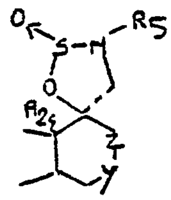

(e) oder man unterzieht ein Produkt der Formel $V_A$ oder $V_B$ der Einwirkung einer Organomagnesiumverbindung oder einer Organolithiumverbindung, um zu einem Produkt der Formel $II_F$

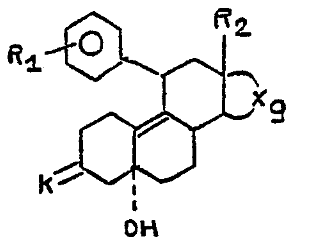

zu gelangen,
worin

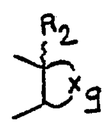

bedeutet:
– entweder

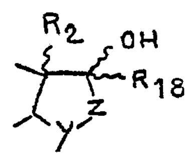

– oder

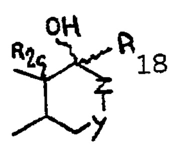

worin $R_{18}$ für einen Alkyl-, Alkenyl-, Alkinylrest mit höchstens 8 Kohlenstoffatomen oder einen carbo- oder heterocyclischen Aryl-, Aralkyl-, Aralkenyl- oder Aralkinylrest, gegebenenfalls substituiert durch einen oder mehrere Reste, ausgewählt unter den Hydroxy-, Alkoxy- oder Alkylthioresten mit 1 bis 4 Kohlenstoffatomen oder Halogen, steht;
(f) oder man unterzieht ein Produkt der Formel $V_A$ oder $V_B$
entweder der Einwirkung eines Cyanurierungsmittels, dann derjenigen eines Mittels zum Schutz der Hydroxyfunktion und hiernach schließlich der Einwirkung einer Magnesium- oder Lithiumverbindung
oder der Einwirkung einer Lithiumverbindung der Formel

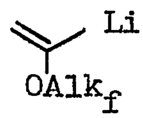

worin $Alk_f$ für einen Alkylrest mit 1 bis 4 Kohlenstoffatomen steht, dann der Einwirkung einer Base und einer Säure, um zu einem Produkt der Formel $II_G$

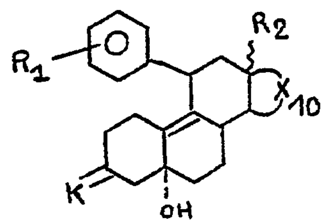

$(II_G)$

zu gelangen,
worin

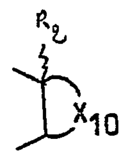

bedeutet:
– entweder

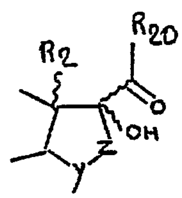

– oder

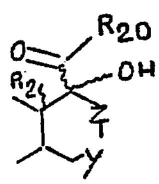

worin $R_{20}$ für einen gegebenenfalls durch Hydroxyl substituierten Alkylrest steht, welches Produkt man gegebenenfalls der Einwirkung eines Acylierungsmittels unterzieht;
(g) oder man unterzieht ein Produkt der Formel $V_A$ oder $V_B$ der Einwirkung eines Trimethylsulfoniumhalogenids oder eines Produkts der Formel $CH^{-}$-$s^{+}$-$(CH_3)_2$ in Gegenwart einer starken Base, um zu einem Produkt der Formel $II_H$:

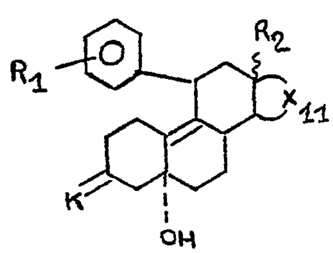

$(II_H)$

zu gelangen,
worin

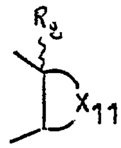

bedeutet:
– entweder

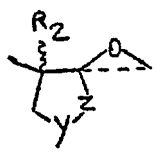

– oder

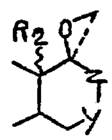

und unterzieht das Produkt der Formel $II_H$ gegebenenfalls der Einwirkung des Produkts der Formel $CH^{-}$-$s^{+}$-$(CH_3)_2$, zu einem Produkt der Formel $II'_H$:

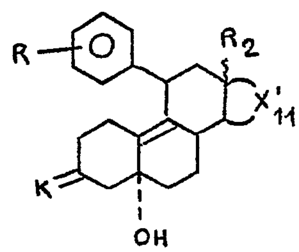

(II'H)

zu gelangen, worin

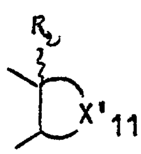

bedeutet:
– entweder

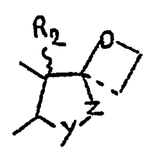

– oder

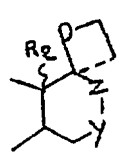

und unterzieht das Produkt der Formel $II_H$ gegebenenfalls zunächst einer alkalischen Hydrolyse, dann der Einwirkung eines Produkts der Formel Hal–$CO_2Alk_g$, worin Hal für ein Halogenatom steht und $Alk_g$

für einen Alkylrest mit 1 bis 4 Kohlenstoffatomen steht, und schließlich der Einwirkung eines Dialkylcarbonats, um zu einem Produkt der Formel II"H

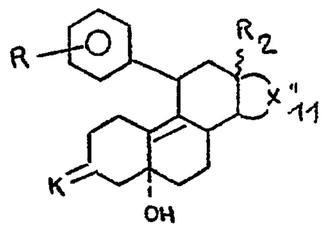

(II"H)

zu gelangen,
worin

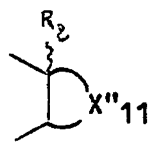

bedeutet:
– entweder

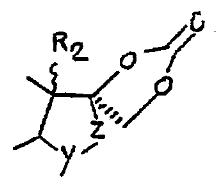

– oder

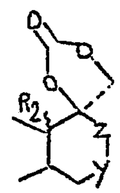

(h) oder man unterzieht ein Produkt der Formel $V_A$ oder $V_B$ zunächst der Einwirkung einer Lithiumverbindung der Formel

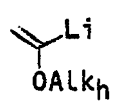

worin $Alk_h$ für einen Alkylrest mit 1 bis 4 Kohlenstoffatomen steht, dann der Einwirkung eines Halogenierungsmittels und hiernach schließlich der Einwirkung einer Base, um zu einem Produkt der Formel $II_J$

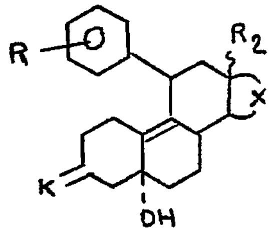

($II_J$)

zu gelangen,
worin

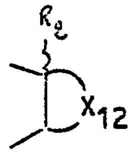

bedeutet
– entweder

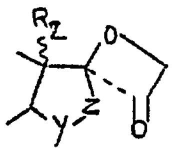

– oder

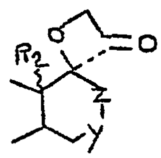

8. Als Arzneimittel die pharmazeutisch verträglichen Produkte der allgemeinen Formel I gemäß Anspruch 1 sowie deren Additionssalze mit pharmazeutisch verträglichen Säuren und Basen.

9. Als Arzneimittel die pharmazeutisch verträglichen Produkte der allgemeinen Formel I' gemäß einem der Ansprüche 2 oder 3 sowie deren pharmazeutisch verträgliche Additionssalze mit Säuren und Basen.

10. Pharmazeutische Zusammensetzungen, enthaltend als Wirkstoff zumindest ein Arzneimittel gemäß einem der Ansprüche 8 oder 9.